# EUROPEAN PATENT APPLICATION

(11) **EP 4 134 370 A1**
(43) Date of publication of application: **15.02.2023**
(21) Application number: 21784269.9
(22) Date of filing: 02.04.2021
(51) Int. Cl.: C07D 493/04, H01L 51/00, H01L 51/50

(54) **HETEROCYCLIC COMPOUND AND ORGANIC LIGHT-EMITTING DEVICE COMPRISING SAME**

(30) Priority: 07.04.2020 KR 20200042138
(71) Applicant: LT Materials Co., Ltd., Yongin-si, Gyeonggi-do 17118 (KR)
(72) Inventor: JEONG, Won-Jang, Yongin-si Gyeonggi-do 17118 (KR); LEE, Nam-Jin, Yongin-si Gyeonggi-do 17118 (KR); LA, Hyun-Ju, Yongin-si Gyeonggi-do 17118 (KR); KIM, Dong-Jun, Yongin-si Gyeonggi-do 17118 (KR)
(74) Representative: Goddar, Heinz J.
(86) International application number: PCT/KR2021/004120
(87) International publication number: WO 2021/206375

(57) **Abstract**

The present specification provides a heterocyclic compound represented by Chemical Formula 1, and an organic light emitting device including the same.

## Description

### [Technical Field]

The present specification relates to a heterocyclic compound, and an organic light emitting device including the same.

The present specification claims priority to and the benefits of Korean Patent Application No. 10-2020-0042138, filed with the Korean Intellectual Property Office on April 7, 2020, the entire contents of which are incorporated herein by reference.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a hostdopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

Studies on an organic light emitting device including a compound capable of satisfying conditions required for materials usable in an organic light emitting device, for example, satisfying proper energy level, electrochemical stability, thermal stability and the like, and having a chemical structure capable of performing various roles required in an organic light emitting device depending on substituents have been required.

### [Disclosure]

### [Technical Problem]

The present application relates to a heterocyclic compound, and an organic light emitting device including the same.

### [Technical Solution]

One embodiment of the present application provides a heterocyclic compound represented by the following Chemical Formula 1. 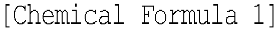 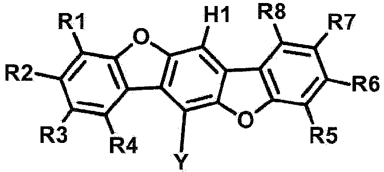 In Chemical Formula 1,
R1 to R8 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted phosphine oxide group; and a substituted or unsubstituted amine group, or two or more groups adjacent to each other bond to each other to form a substituted or unsubstituted C6 to C60 aliphatic or aromatic hydrocarbon ring; or a substituted or unsubstituted C2 to C60 heteroring,
H1 is hydrogen; or deuterium, and
Y is represented by any one of the following Chemical Formula 1-1 to Chemical Formula 1-4, 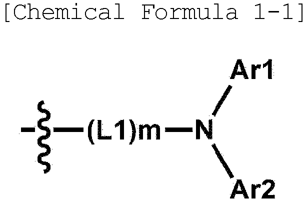 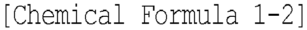 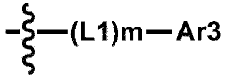 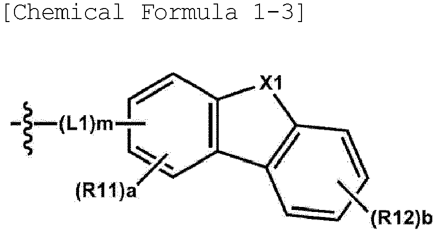 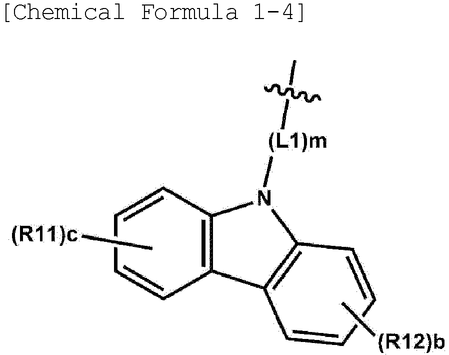 in Chemical Formulae 1-1 to 1-4,
X1 is O or S,
L1 is a direct bond; a substituted or unsubstituted C6 to C60 arylene group; or a substituted or unsubstituted C2 to C60 heteroarylene group,
R11 and R12 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other bond to each other to form a substituted or unsubstituted C6 to C60 aliphatic or aromatic hydrocarbon ring; or a substituted or unsubstituted C2 to C60 heteroring,
Ar1 and Ar2 are each independently a substituted or unsubstituted C1 to C60 alkyl group; a substituted or unsubstituted C6 to C60 aryl group; or a substituted or unsubstituted C2 to C60 heteroaryl group,
Ar3 is a substituted or unsubstituted C6 to C60 aryl group,
m is an integer of 0 to 5, and
a is an integer of 0 to 3, and b and c are each an integer of 0 to 4.

In addition, one embodiment of the present application provides an organic light emitting device including a first electrode; a second electrode provided opposite to the first electrode; and one or more organic material layers provided between the first electrode and the second electrode, wherein one or more layers of the organic material layers include the heterocyclic compound represented by Chemical Formula 1.

### [Advantageous Effects]

A compound described in the present specification can be used as a material of an organic material layer of an organic light emitting device. The compound is capable of performing a role of a hole injection material, a hole transfer material, a light emitting material, an electron transfer material, an electron injection material or the like in the organic light emitting device. Particularly, the compound can be used as a hole transfer material, an electron blocking material or a light emitting material of the organic light emitting device. For example, the compound can be used alone as a light emitting material, can be used with another compound so that two compounds are used as a light emitting material, and can be used as a host material of a light emitting layer.

The heterocyclic compound of Chemical Formula 1 according to the present application has a structure in which benzofuran is fused to a dibenzofuran structure, and by particularly the substituent Y satisfying any one of Chemical Formulae 1-1 to 1-4, a more stable structure is obtained by substituting a specific substituent having a hole transfer ability, and as a result, properties of lifetime and driving stability, and improving efficiency are obtained in an organic light emitting device.

By using the heterocyclic compounds of Chemical Formula 1, an organic light emitting device having improved lifetime, driving stability and efficiency can be manufactured.

### [Description of Drawings]

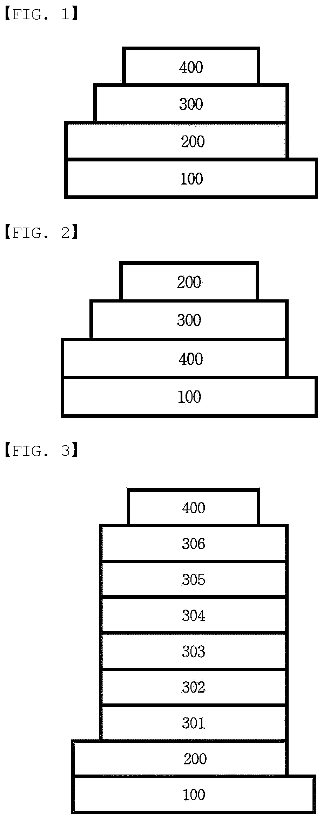

FIG. 1 to FIG. 3 are diagrams each schematically illustrating a lamination structure of an organic light emitting device according to one embodiment of the present application.

### [Reference Numeral]

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

### [Mode for Disclosure]

Hereinafter, the present application will be described in detail.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tertbutoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The number of carbon atoms of the aryl group may be from 6 to 60, specifically from 6 to 40 and more specifically from 6 to 25. Specific examples of the aryl group may include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, an indenyl group, an acenaphthylenyl group, a 2,3-dihydro-1H-indenyl group, a fused ring group thereof, and the like, but are not limited thereto.

In the present specification, a fluorenyl group may be substituted, and adjacent substituents may bond to each other to form a ring.
When the fluorenyl group is substituted, 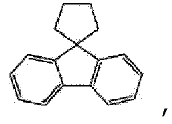 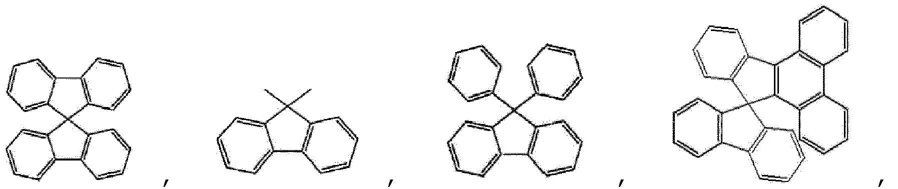 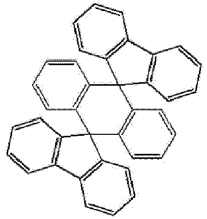 and the like may be included, however, the structure is not limited thereto.

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, the phosphine oxide group is represented by -P(=O)(R101)(R102), and R101 and R102 are the same as or different from each other and may be each independently a substituent formed with at least one of hydrogen; deuterium; halogen; an alkyl group; an alkenyl group; an alkoxy group; a cycloalkyl group; an aryl group; and a heterocyclic group. Specific examples of the phosphine oxide may include a diphenylphosphine oxide group, a dinaphthylphosphine oxide group and the like, but are not limited thereto.

In the present specification, the silyl group is a substituent including Si, having the Si atom directly linked as a radical, and is represented by -Si(R104) (R105) (R106) . R104 to R106 are the same as or different from each other, and may be each independently a substituent formed with at least one of hydrogen; deuterium; halogen; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic hydrocarbon ring, the aliphatic heteroring, the aromatic hydrocarbon ring or the aromatic heteroring that adjacent groups may form, the structures illustrated as the cycloalkyl group, the heterocycloalkyl group, the aryl group and the heteroaryl group described above may be used except for those that are not a monovalent group.

In the present specification, the heteroring is not limited as long as it includes a heteroatom in the ring structure. For example, the heteroring may include a heterocycloalkyl group or a heteroaryl group, and an additional ring may be fused to the heterocycloalkyl group or the heteroaryl group.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of deuterium; halogen; a cyano group; C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; -P(=O)RR'; C1 to C20 alkylamine; C6 to C60 monocyclic or polycyclic arylamine; and C2 to C60 monocyclic or polycyclic heteroarylamine, or being substituted with a substituent linking two or more substituents selected from among the substituents illustrated above, or being unsubstituted, and

R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 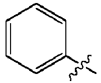 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

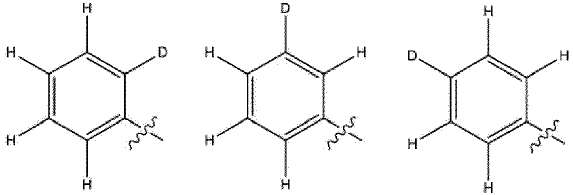

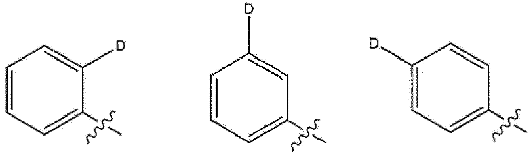

In addition, in one embodiment of the present application, "a phenyl group having a deuterium content of 0%" may mean a phenyl group that does not include a deuterium atom, that is, a phenyl group that has 5 hydrogen atoms.

In one embodiment of the present application, Chemical Formula 1 may be represented by the following Chemical Formula 11. 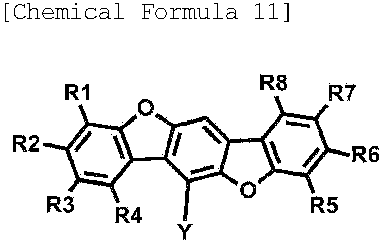 In Chemical Formula 11,
R1 to R8 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted phosphine oxide group; and a substituted or unsubstituted amine group, or two or more groups adjacent to each other bond to each other to form a substituted or unsubstituted C6 to C60 aliphatic or aromatic hydrocarbon ring; or a substituted or unsubstituted C2 to C60 heteroring,
Y is represented by any one of the following Chemical Formula 1-1 to Chemical Formula 1-4, 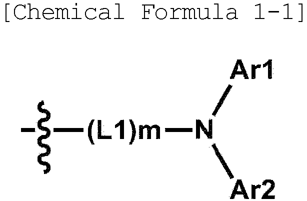 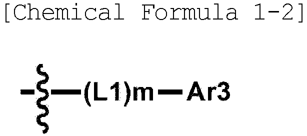 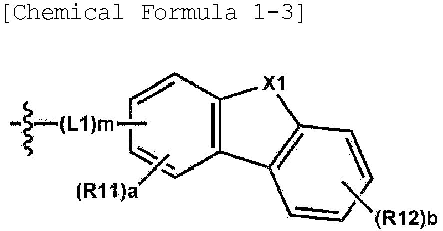 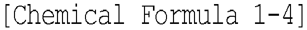 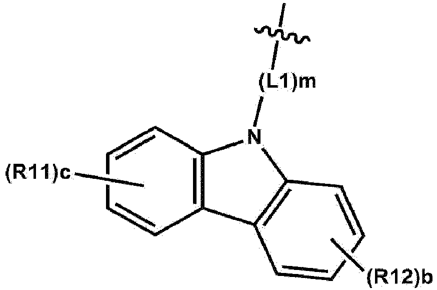 in Chemical Formulae 1-1 to 1-4,
X1 is O or S,
L1 is a direct bond; a substituted or unsubstituted C6 to C60 arylene group; or a substituted or unsubstituted C2 to C60 heteroarylene group,
R11 and R12 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other bond to each other to form a substituted or unsubstituted C6 to C60 aliphatic or aromatic hydrocarbon ring; or a substituted or unsubstituted C2 to C60 heteroring,
Ar1 and Ar2 are each independently a substituted or unsubstituted C1 to C60 alkyl group; a substituted or unsubstituted C6 to C60 aryl group; or a substituted or unsubstituted C2 to C60 heteroaryl group,
Ar3 is a substituted or unsubstituted C6 to C60 aryl group,
m is an integer of 0 to 5, and
a is an integer of 0 to 3, b and c are each an integer of 0 to 4.

In one embodiment of the present application, Chemical Formula 1 may have a deuterium content of 0% to 100%.

In one embodiment of the present application, Chemical Formula 1 may have a deuterium content of 0%.

The deuterium content of 0% may mean that the heterocyclic compound of Chemical Formula 1 does not include deuterium.

In one embodiment of the present application, Chemical Formula 1 may have a deuterium content of greater than 0% and less than or equal to 100%.

In one embodiment of the present application, Chemical Formula 1 may have a deuterium content of 30% to 100%.

In one embodiment of the present application, Chemical Formula 1 may have a deuterium content of 50% to 100%.

In one embodiment of the present application, Chemical Formula 1 may have a deuterium content of 100%. This is effective in improving a lifetime compared to the same structure that does not contain deuterium.

In one embodiment of the present application, the heterocyclic compound of Chemical Formula 1 may be represented by the following Chemical Formula 2 or Chemical Formula 3. 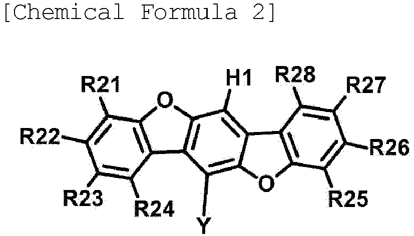 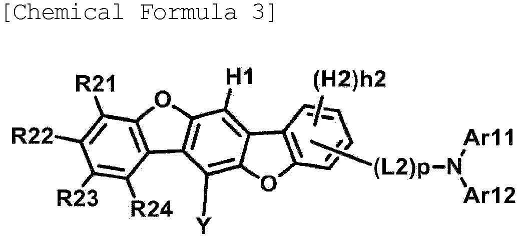 In Chemical Formula 2 and Chemical Formula 3,
Y and H1 have the same definitions as in Chemical Formula 1,
R21 to R28 are the same as or different from each other, and each independently hydrogen; deuterium; a substituted or unsubstituted C1 to C60 alkyl group; or a substituted or unsubstituted C6 to C60 aryl group,
L2 is a direct bond; a substituted or unsubstituted C6 to C60 arylene group; or a substituted or unsubstituted C2 to C60 heteroarylene group,
p is an integer of 0 to 5, and when 2 or greater, L2s are the same as or different from each other,
Ar11 and Ar12 are each independently a substituted or unsubstituted C1 to C60 alkyl group; a substituted or unsubstituted C6 to C60 aryl group; or a substituted or unsubstituted C2 to C60 heteroaryl group,
H2 is hydrogen; or deuterium, and
h2 is an integer of 0 to 3, and when 2 or greater, H2s are the same as or different from each other.

In one embodiment of the present application, Chemical Formula 3 may be represented by any one of the following Chemical Formulae 3-1 to 3-4. 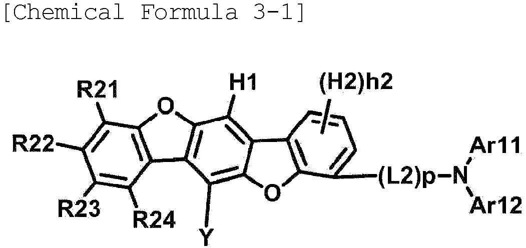 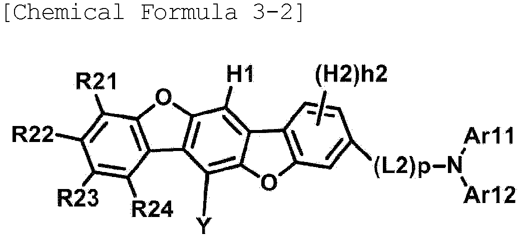 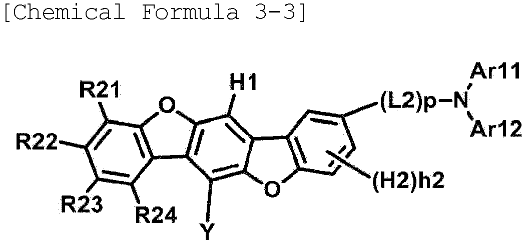 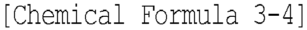 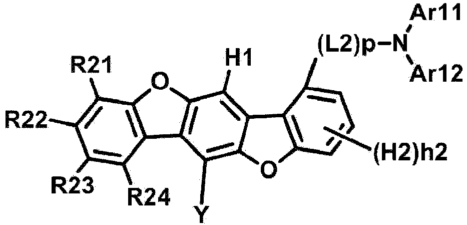 In Chemical Formulae 3-1 to 3-4,
each substituent has the same definition as in Chemical Formula 3.

In one embodiment of the present application, 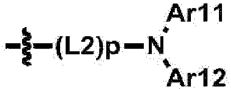 of Chemical Formula 3 may be represented by the following Chemical Formula 4 or Chemical Formula 5. 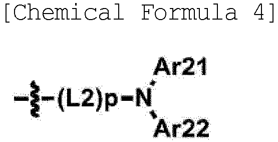 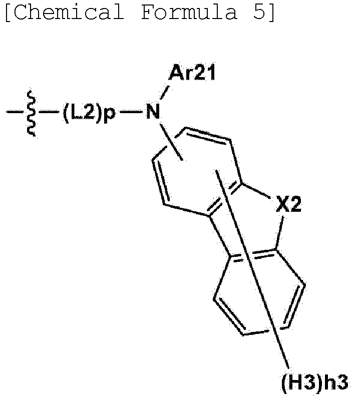 In Chemical Formula 4 and Chemical Formula 5,
L2 and p have the same definitions as in Chemical Formula 3,
X2 is O; or S,
Ar21 and Ar22 are each independently a substituted or unsubstituted C1 to C60 alkyl group; or a substituted or unsubstituted C6 to C60 aryl group,
H3 is hydrogen; or deuterium, and
h3 is an integer of 0 to 7, and when 2 or greater, H3s are the same as or different from each other.

In one embodiment of the present application, L2 may be a direct bond; a substituted or unsubstituted C6 to C60 arylene group; or a substituted or unsubstituted C2 to C60 heteroarylene group.

In another embodiment, L2 may be a direct bond; or a substituted or unsubstituted C6 to C60 arylene group.

In another embodiment, L2 may be a direct bond; or a substituted or unsubstituted C6 to C40 arylene group.

In another embodiment, L2 may be a direct bond; or a substituted or unsubstituted C6 to C20 arylene group.

In another embodiment, L2 may be a direct bond; or a substituted or unsubstituted monocyclic or polycyclic C6 to C20 arylene group.

In another embodiment, L2 may be a direct bond; or a monocyclic C6 to C20 arylene group unsubstituted or substituted with deuterium.

In another embodiment, L2 may be a direct bond; or a phenylene group unsubstituted or substituted with deuterium.

In another embodiment, L2 may be a direct bond; or a monocyclic C6 to C20 arylene group.

In another embodiment, L2 may be a direct bond; or a phenylene group.

In one embodiment of the present application, Ar11 and Ar12 may be each independently a substituted or unsubstituted C1 to C60 alkyl group; a substituted or unsubstituted C6 to C60 aryl group; or a substituted or unsubstituted C2 to C60 heteroaryl group.

In another embodiment, Ar11 and Ar12 may be each independently a substituted or unsubstituted C6 to C60 aryl group; or a substituted or unsubstituted C2 to C60 heteroaryl group.

In another embodiment, Ar11 and Ar12 may be each independently a substituted or unsubstituted C6 to C40 aryl group; or a substituted or unsubstituted C2 to C40 heteroaryl group.

In another embodiment, Ar11 and Ar12 may be each independently a C6 to C40 aryl group unsubstituted or substituted with one or more substituents selected from the group consisting of a C1 to C10 alkyl group, a C6 to C40 aryl group and a C2 to C40 heteroaryl group; or a C2 to C40 heteroaryl group.

In another embodiment, Ar11 and Ar12 may be each independently a phenyl group unsubstituted or substituted with a dibenzofuran group or a dibenzothiophene group; a biphenyl group; a naphthyl group; a dimethylfluorenyl group; a diphenylfluorenyl group; a spirobifluorenyl group; a dibenzofuran group; or a dibenzothiophene group.

In one embodiment of the present application, Ar21 and Ar22 may be each independently a substituted or unsubstituted C1 to C60 alkyl group; or a substituted or unsubstituted C6 to C60 aryl group.

In another embodiment, Ar21 and Ar22 may be each independently a substituted or unsubstituted C6 to C60 aryl group.

In another embodiment, Ar21 and Ar22 may be each independently a substituted or unsubstituted monocyclic or polycyclic C6 to C60 aryl group.

In another embodiment, Ar21 and Ar22 may be each independently a monocyclic or polycyclic C6 to C60 aryl group unsubstituted or substituted with one or more substituents selected from the group consisting of a C1 to C10 alkyl group, a C6 to C40 aryl group and a C2 to C40 heteroaryl group.

In another embodiment, Ar21 and Ar22 may be each independently a phenyl group unsubstituted or substituted with a dibenzothiophene group or a dibenzofuran group; a biphenyl group; a naphthyl group; a dimethylfluorenyl group; a diphenylfluorenyl group; or a spirobifluorenyl group.

In another embodiment, Ar11 and Ar12 may be each independently a C6 to C40 aryl group unsubstituted or substituted with one or more substituents selected from the group consisting of deuterium, a C1 to C10 alkyl group, a C6 to C40 aryl group and a C2 to C40 heteroaryl group; or a C2 to C40 heteroaryl group unsubstituted or substituted with deuterium.

In another embodiment, Ar11 and Ar12 may be each independently a phenyl group unsubstituted or substituted with deuterium, a dibenzofuran group or a dibenzothiophene group; a biphenyl group unsubstituted or substituted with deuterium; a naphthyl group unsubstituted or substituted with deuterium; a dimethylfluorenyl group unsubstituted or substituted with deuterium; a diphenylfluorenyl group; a spirobifluorenyl group; a dibenzofuran group unsubstituted or substituted with deuterium; or a dibenzothiophene group unsubstituted or substituted with deuterium.

In another embodiment, Ar21 and Ar22 may be each independently a monocyclic or polycyclic C6 to C60 aryl group unsubstituted or substituted with one or more substituents selected from the group consisting of deuterium, a C1 to C10 alkyl group, a C6 to C40 aryl group and a C2 to C40 heteroaryl group.

In another embodiment, Ar21 and Ar22 may be each independently a phenyl group unsubstituted or substituted with deuterium, a dibenzothiophene group or a dibenzofuran group; a biphenyl group; a naphthyl group; a dimethylfluorenyl group; a diphenylfluorenyl group; or a spirobifluorenyl group.

In one embodiment of the present application, R1 to R8 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted phosphine oxide group; and a substituted or unsubstituted amine group, or two or more groups adjacent to each other may bond to each other to form a substituted or unsubstituted C6 to C60 aliphatic or aromatic hydrocarbon ring; or a substituted or unsubstituted C2 to C60 heteroring.

In another embodiment, R1 to R8 are the same as or different from each other, and each independently selected from the group consisting of hydrogen; deuterium; a substituted or unsubstituted C6 to C60 aryl group; and a substituted or unsubstituted amine group, or two or more groups adjacent to each other may bond to each other to form a substituted or unsubstituted C6 to C60 aliphatic or aromatic hydrocarbon ring; or a substituted or unsubstituted C2 to C60 heteroring.

In another embodiment, R1 to R8 are the same as or different from each other, and may be each independently hydrogen; deuterium; a substituted or unsubstituted C6 to C60 aryl group; or a substituted or unsubstituted amine group.

In another embodiment, R1 to R8 are the same as or different from each other, and may be each independently hydrogen; deuterium; a C6 to C60 aryl group unsubstituted or substituted with a substituted or unsubstituted amine group; or a substituted or unsubstituted amine group.

In another embodiment, R1 to R8 are the same as or different from each other, and may be each independently hydrogen; deuterium; a C6 to C60 aryl group unsubstituted or substituted with an amine group unsubstituted or substituted with one or more substituents selected from the group consisting of a C1 to C10 alkyl group unsubstituted or substituted with deuterium, a C6 to C60 aryl group unsubstituted or substituted with deuterium and a C2 to C60 heteroaryl group unsubstituted or substituted with deuterium; or an amine group unsubstituted or substituted with one or more substituents selected from the group consisting of a C1 to C10 alkyl group unsubstituted or substituted with deuterium, a C6 to C60 aryl group unsubstituted or substituted with deuterium and a C2 to C60 heteroaryl group unsubstituted or substituted with deuterium.

In another embodiment, R1 to R8 are the same as or different from each other, and may be each independently hydrogen; deuterium; or

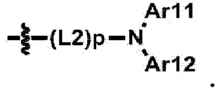

In another embodiment, R1 to R8 are the same as or different from each other, and may be each independently hydrogen; or a substituted or unsubstituted amine group.

In another embodiment, R1 to R8 are the same as or different from each other, and may be each independently hydrogen; or an amine group unsubstituted or substituted with one or more substituents selected from the group consisting of a C1 to C10 alkyl group, a C6 to C60 aryl group and a C2 to C60 heteroaryl group.

In another embodiment, R1 to R8 may be each independently hydrogen; or deuterium.

In another embodiment, R1 to R8 may be hydrogen.

In another embodiment, any one of R1 to R8 is an amine group unsubstituted or substituted with one or more substituents selected from the group consisting of a C1 to C10 alkyl group unsubstituted or substituted with deuterium, a C6 to C60 aryl group unsubstituted or substituted with deuterium and a C2 to C60 heteroaryl group unsubstituted or substituted with deuterium, and the rest may be each independently hydrogen; or deuterium.

In another embodiment, any one of R1 to R8 is an amine group unsubstituted or substituted with one or more substituents selected from the group consisting of a C1 to C10 alkyl group, a C6 to C60 aryl group and a C2 to C60 heteroaryl group, and the rest may be hydrogen.

In one embodiment of the present application, R1 to R4 may be each independently hydrogen; or deuterium.

In one embodiment of the present application, Y of Chemical Formula 1 may be represented by any one of the following Chemical Formula 1-1 to Chemical Formula 1-4. 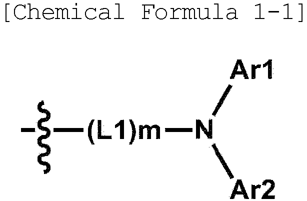 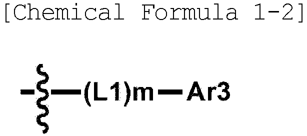 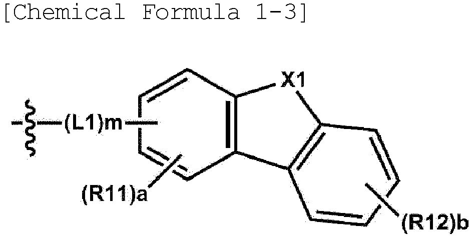 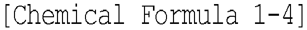 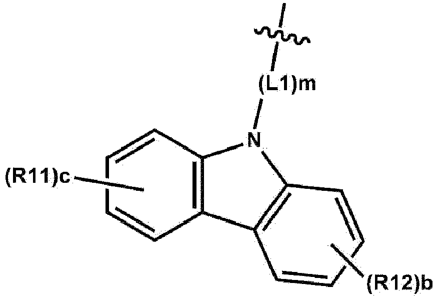 In Chemical Formulae 1-1 to 1-4,
X1 is O or S,
L1 is a direct bond; a substituted or unsubstituted C6 to C60 arylene group; or a substituted or unsubstituted C2 to C60 heteroarylene group,
R11 and R12 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other bond to each other to form a substituted or unsubstituted C6 to C60 aliphatic or aromatic hydrocarbon ring; or a substituted or unsubstituted C2 to C60 heteroring,
Ar1 and Ar2 are each independently a substituted or unsubstituted C1 to C60 alkyl group; a substituted or unsubstituted C6 to C60 aryl group; or a substituted or unsubstituted C2 to C60 heteroaryl group,
Ar3 is a substituted or unsubstituted C6 to C60 aryl group,
m is an integer of 0 to 5, and
a is an integer of 0 to 3, and b and c are each an integer of 0 to 4.

In one embodiment of the present application, L1 may be a direct bond; a substituted or unsubstituted C6 to C60 arylene group; or a substituted or unsubstituted C2 to C60 heteroarylene group.

In another embodiment, L1 may be a direct bond; or a substituted or unsubstituted C6 to C60 arylene group.

In another embodiment, L1 may be a direct bond; or a substituted or unsubstituted C6 to C40 arylene group.

In another embodiment, L1 may be a direct bond; or a substituted or unsubstituted C6 to C20 arylene group.

In another embodiment, L1 may be a direct bond; or a substituted or unsubstituted monocyclic or polycyclic C6 to C20 arylene group.

In another embodiment, L1 may be a direct bond; or a monocyclic C6 to C20 arylene group.

In another embodiment, L1 may be a direct bond; or a phenylene group.

In another embodiment, L1 may be a direct bond; or a monocyclic C6 to C20 arylene group unsubstituted or substituted with deuterium.

In another embodiment, L1 may be a direct bond; or a phenylene group unsubstituted or substituted with deuterium.

In one embodiment of the present application, m is an integer of 0 to 5, and when 2 or greater, L1s are the same as or different from each other.

In one embodiment of the present application, R11 and R12 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other may bond to each other to form a substituted or unsubstituted C6 to C60 aliphatic or aromatic hydrocarbon ring; or a substituted or unsubstituted C2 to C60 heteroring.

In another embodiment, R11 and R12 are the same as or different from each other, and may be each independently selected from the group consisting of hydrogen; deuterium; a substituted or unsubstituted C1 to C60 alkyl group; a substituted or unsubstituted C6 to C60 aryl group; a substituted or unsubstituted C2 to C60 heteroaryl group; a substituted or unsubstituted phosphine oxide group; and a substituted or unsubstituted amine group.

In another embodiment, R11 and R12 may be each independently hydrogen; or deuterium.

In another embodiment, R11 and R12 may be hydrogen.

In one embodiment of the present application, a is an integer of 0 to 3, and when 2 or greater, R11s of Chemical Formula 1-3 are the same as or different from each other.

In one embodiment of the present application, b is an integer of 0 to 4, and when 2 or greater, R12s of Chemical Formulae 1-3 and 1-4 are the same as or different from each other.

In one embodiment of the present application, c is an integer of 0 to 4, and when 2 or greater, R11s of Chemical Formula 1-4 are the same as or different from each other.

In one embodiment of the present application, Ar1 and Ar2 may be each independently a substituted or unsubstituted C1 to C60 alkyl group; a substituted or unsubstituted C6 to C60 aryl group; or a substituted or unsubstituted C2 to C60 heteroaryl group.

In another embodiment, Ar1 and Ar2 may be each independently a substituted or unsubstituted C6 to C60 aryl group; or a substituted or unsubstituted C2 to C60 heteroaryl group.

In another embodiment, Ar1 and Ar2 may be each independently a substituted or unsubstituted C6 to C40 aryl group; or a substituted or unsubstituted C2 to C40 heteroaryl group.

In another embodiment, Ar1 and Ar2 may be each independently a C6 to C40 aryl group unsubstituted or substituted with one or more substituents selected from the group consisting of a C1 to C10 alkyl group, a C6 to C40 aryl group and a C2 to C40 heteroaryl group; or a C2 to C40 heteroaryl group.

In another embodiment, Ar1 and Ar2 may be each independently a phenyl group unsubstituted or substituted with a dibenzofuran group or a dibenzothiophene group; a biphenyl group; a naphthyl group; a dimethylfluorenyl group; a diphenylfluorenyl group; a spirobifluorenyl group; a dibenzofuran group; or a dibenzothiophene group.

In another embodiment, Ar1 and Ar2 may be each independently a C6 to C40 aryl group unsubstituted or substituted with one or more substituents selected from the group consisting of deuterium, a C1 to C10 alkyl group, a C6 to C40 aryl group and a C2 to C40 heteroaryl group; or a C2 to C40 heteroaryl group unsubstituted or substituted with deuterium.

In another embodiment, Ar1 and Ar2 may be each independently a phenyl group unsubstituted or substituted with a dibenzofuran group or a dibenzothiophene group; a biphenyl group unsubstituted or substituted with deuterium; a naphthyl group unsubstituted or substituted with deuterium; a dimethylfluorenyl group unsubstituted or substituted with deuterium; a diphenylfluorenyl group; a spirobifluorenyl group; a dibenzofuran group unsubstituted or substituted with deuterium; or a dibenzothiophene group unsubstituted or substituted with deuterium.

In one embodiment of the present application, Ar3 may be a substituted or unsubstituted C6 to C60 aryl group.

In another embodiment, Ar3 may be a substituted or unsubstituted C6 to C40 aryl group.

In another embodiment, Ar3 may be a C6 to C40 aryl group unsubstituted or substituted with one or more substituents selected from the group consisting of a C1 to C10 alkyl group, a C6 to C40 aryl group and a C2 to C40 heteroaryl group.

In another embodiment, Ar3 may be a phenyl group; a biphenyl group; a naphthyl group; a dimethylfluorenyl group; a diphenylfluorenyl group; or a spirobifluorenyl group.

In another embodiment, Ar3 may be a C6 to C40 aryl group unsubstituted or substituted with one or more substituents selected from the group consisting of deuterium, a C1 to C10 alkyl group, a C6 to C40 aryl group and a C2 to C40 heteroaryl group.

In another embodiment, Ar3 may be a phenyl group unsubstituted or substituted with deuterium; a biphenyl group unsubstituted or substituted with deuterium; a naphthyl group unsubstituted or substituted with deuterium; a dimethylfluorenyl group; a diphenylfluorenyl group; or a spirobifluorenyl group.

According to one embodiment of the present application, Chemical Formula 1 may be represented by any one of the following compounds, but is not limited thereto.

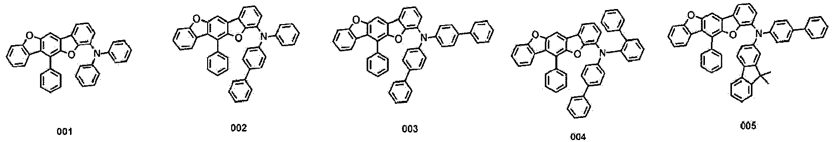

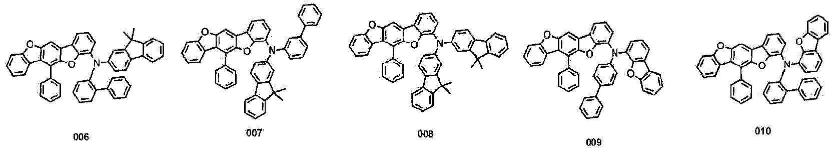

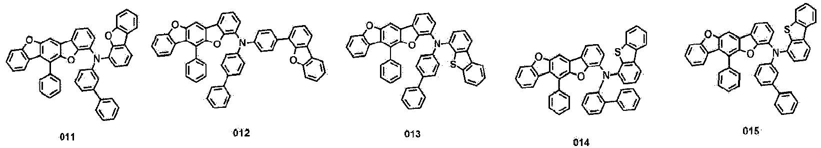

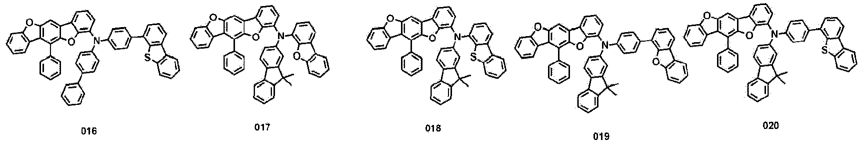

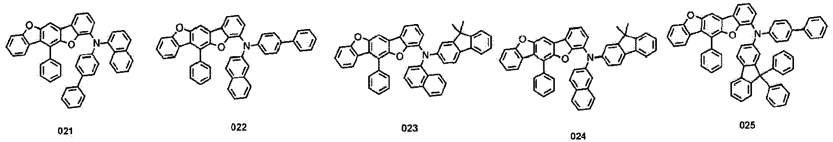

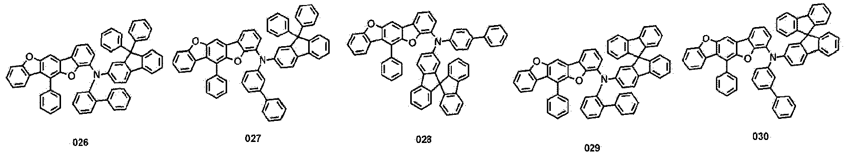

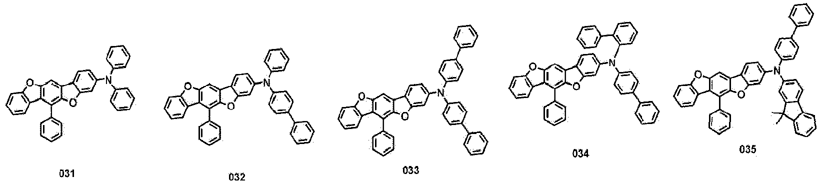

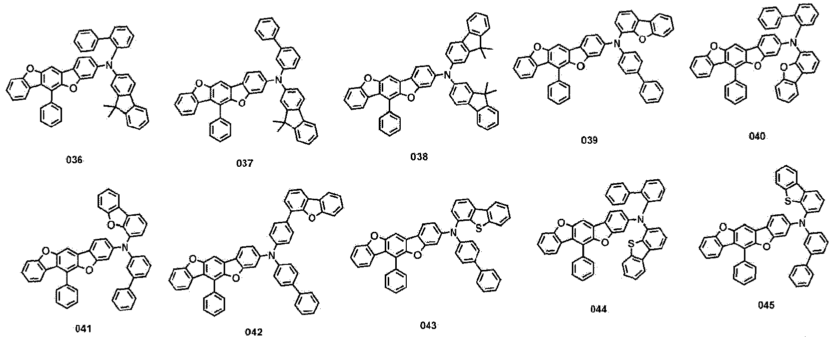

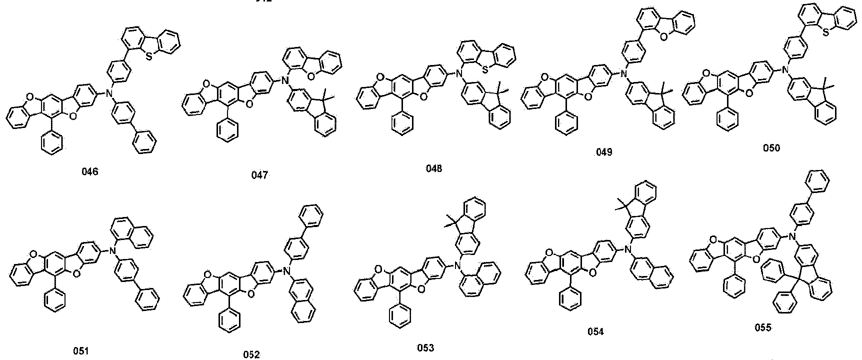

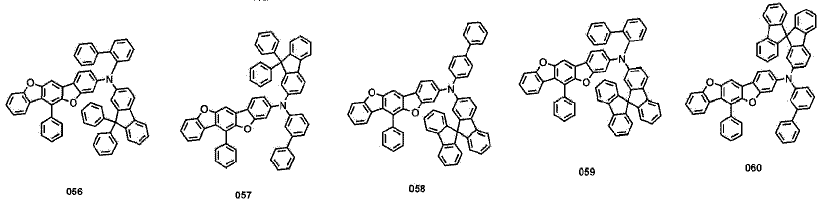

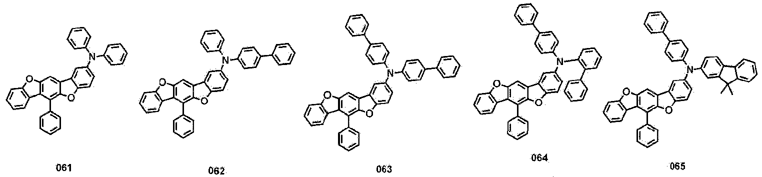

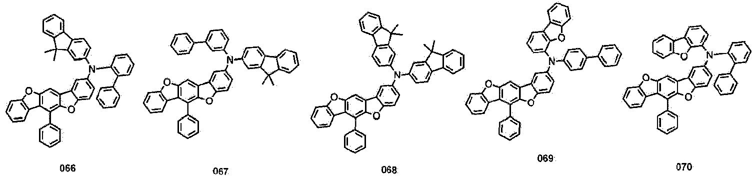

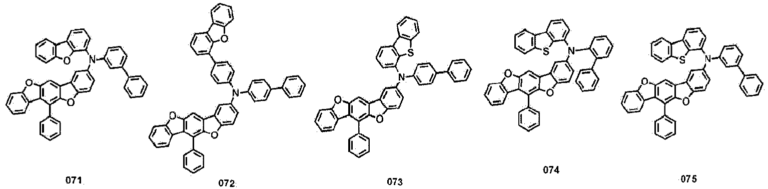

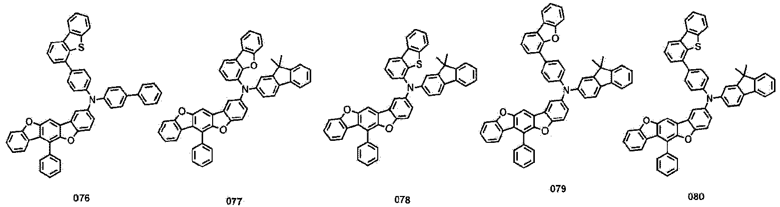

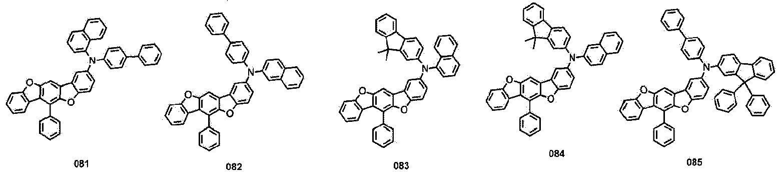

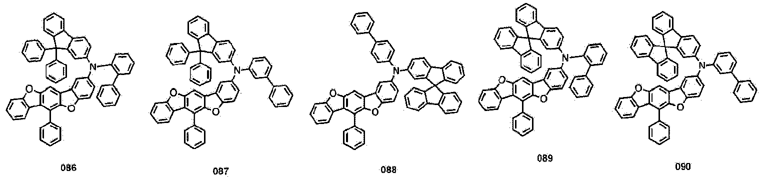

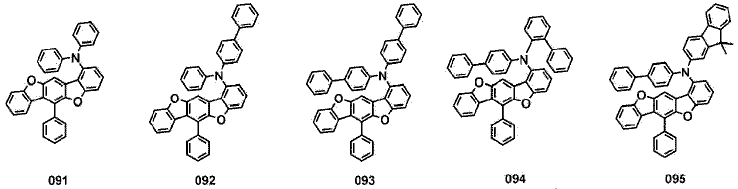

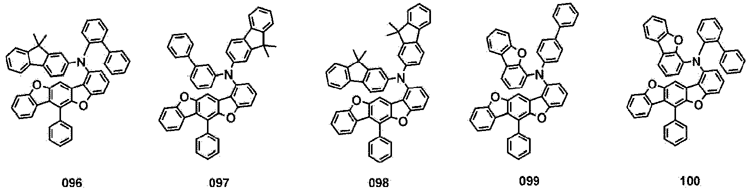

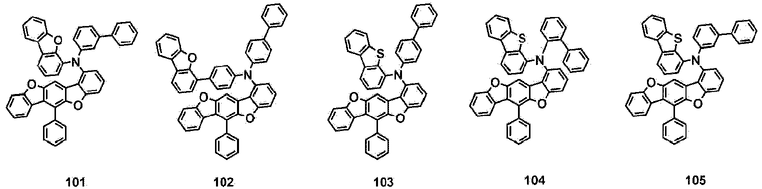

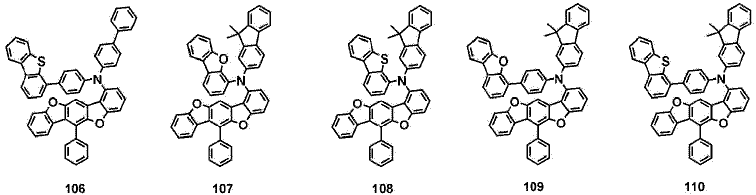

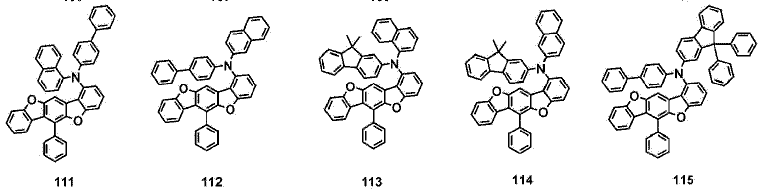

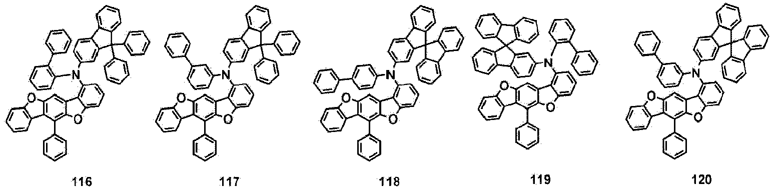

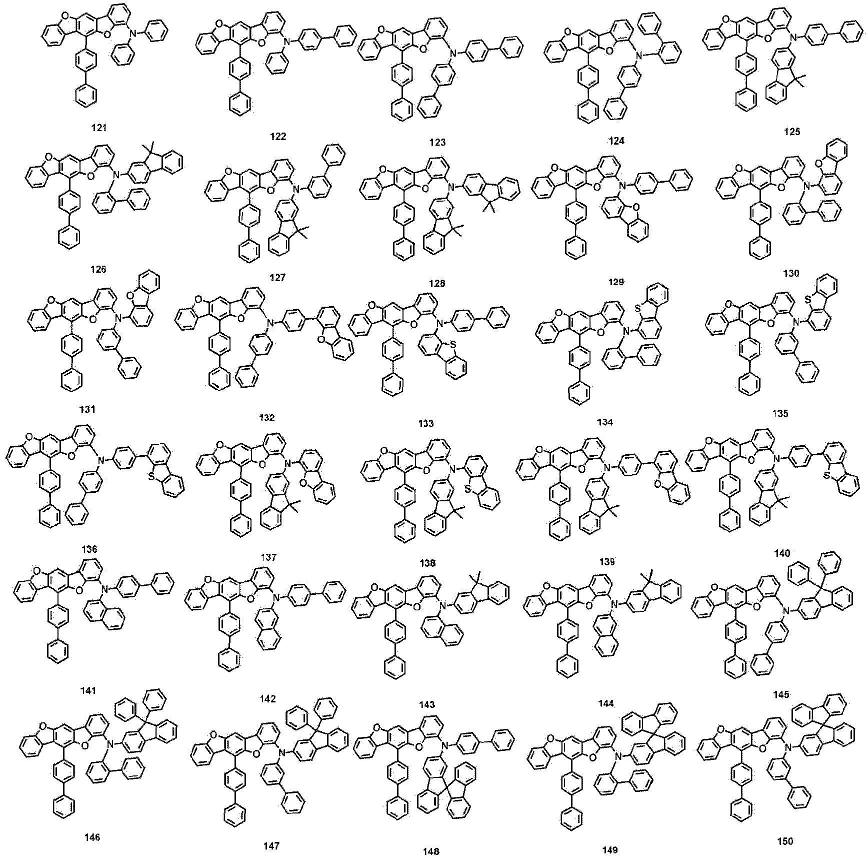

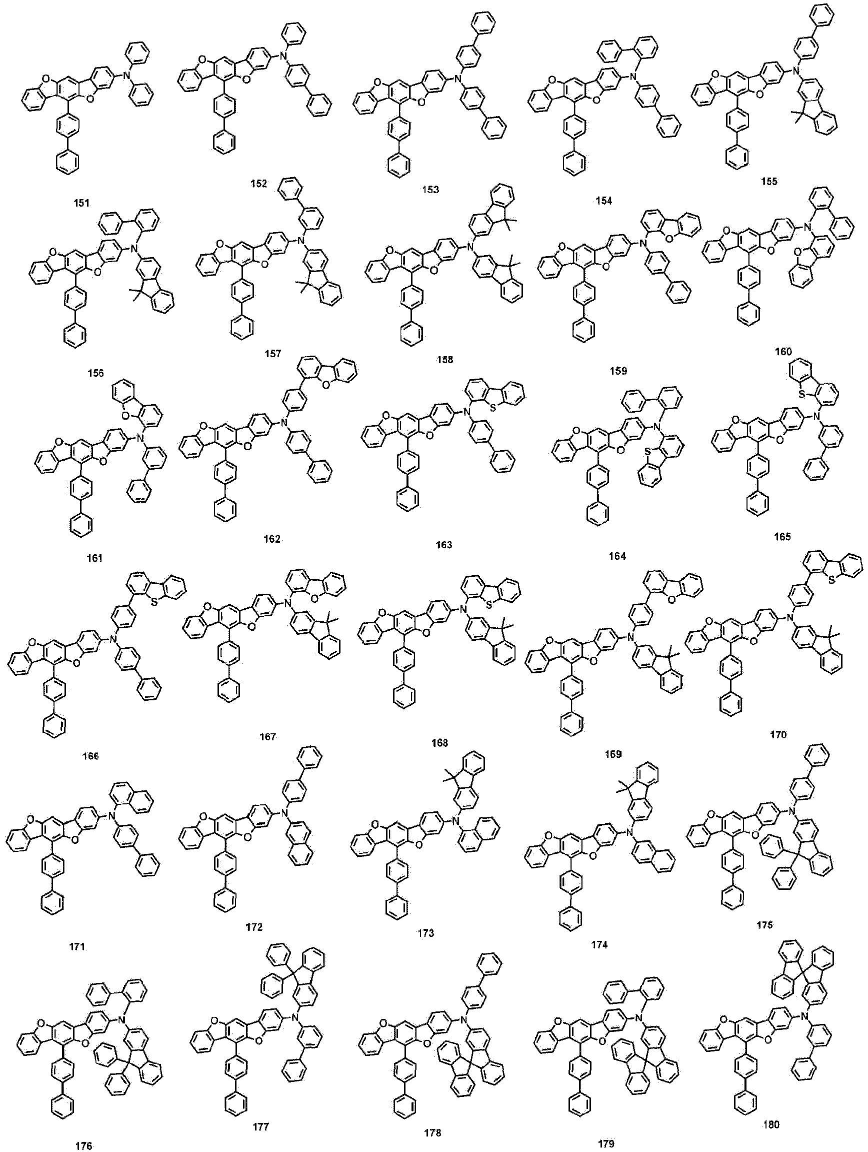

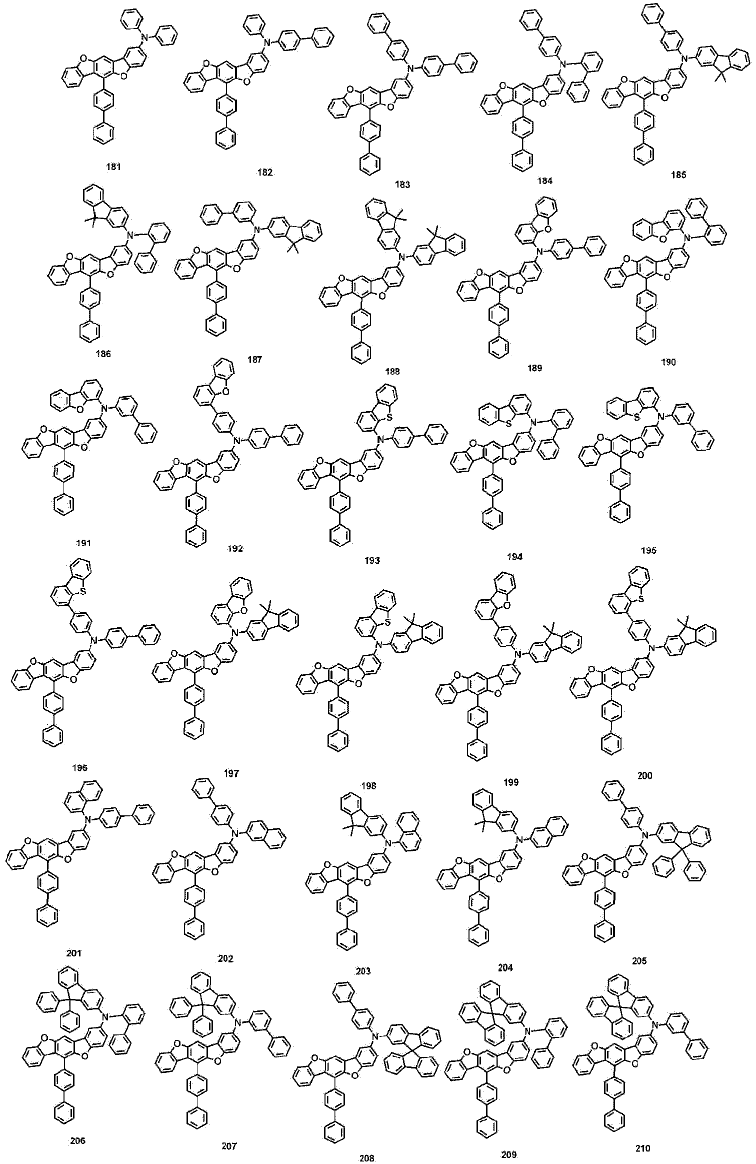

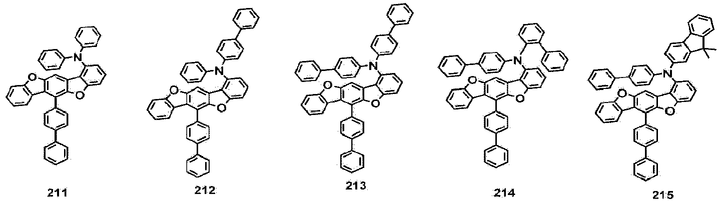

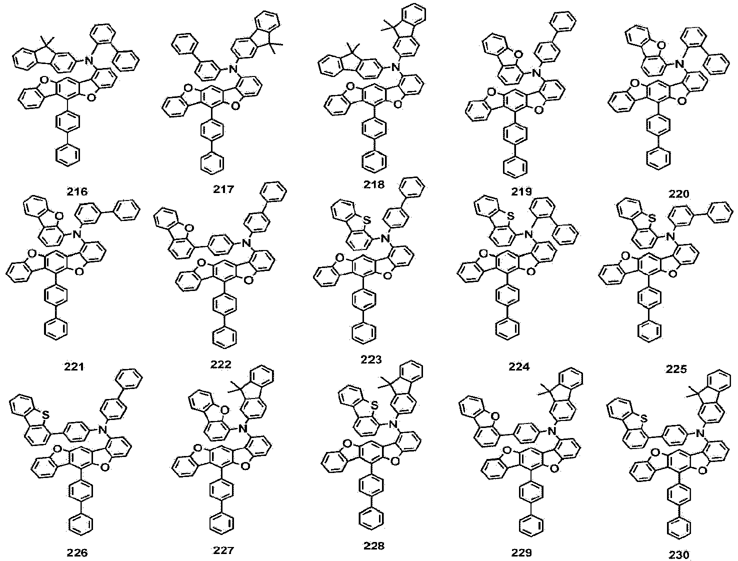

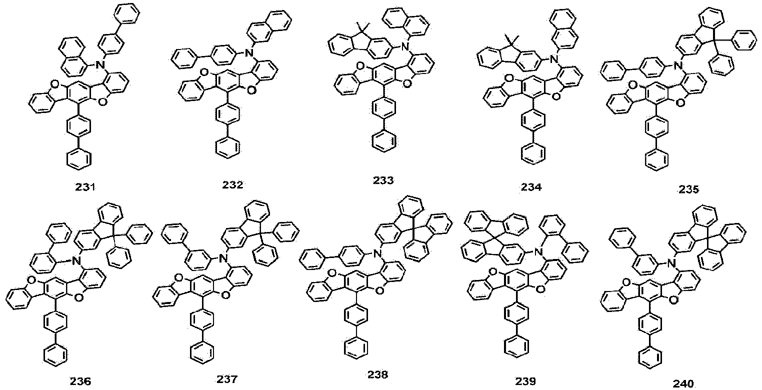

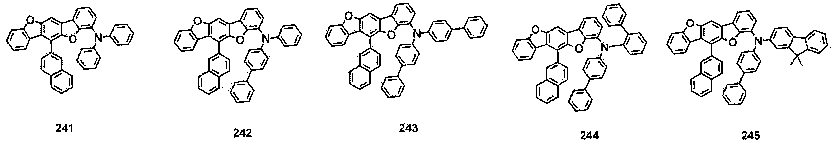

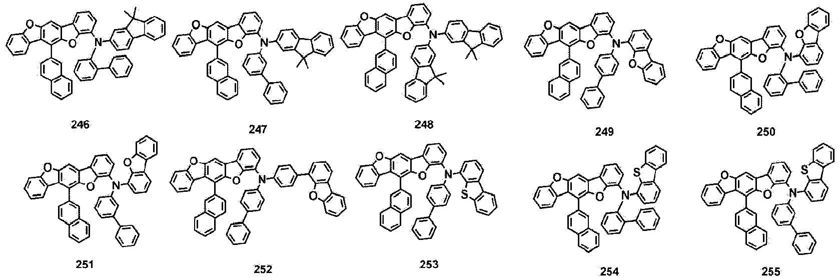

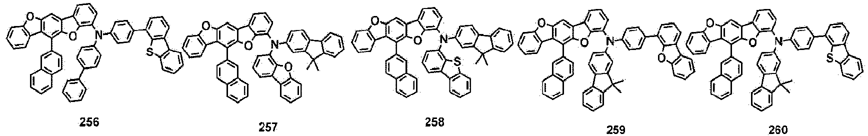

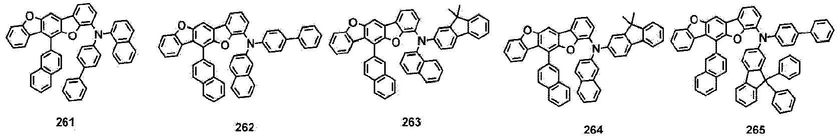

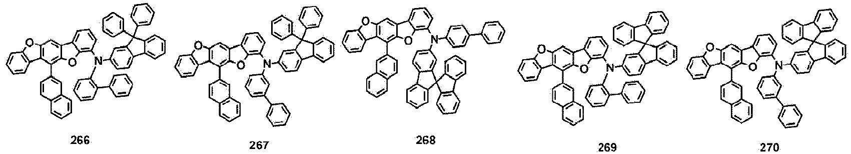

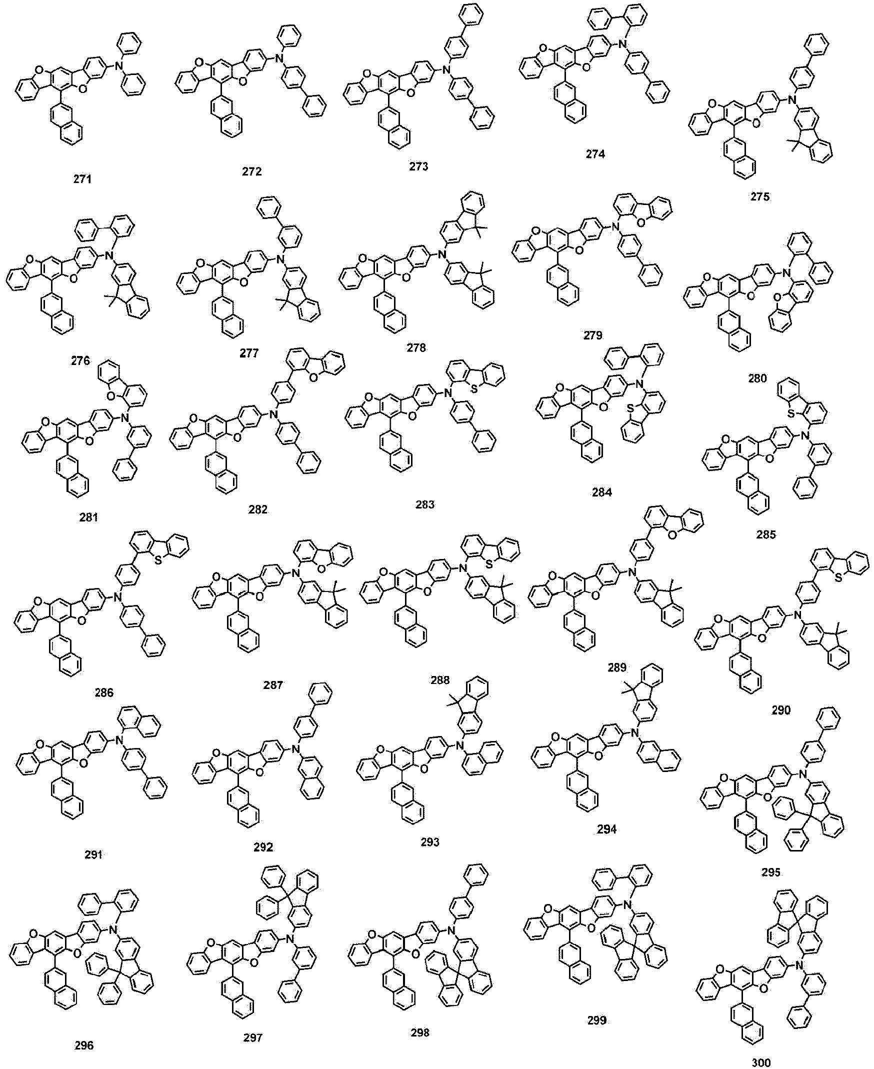

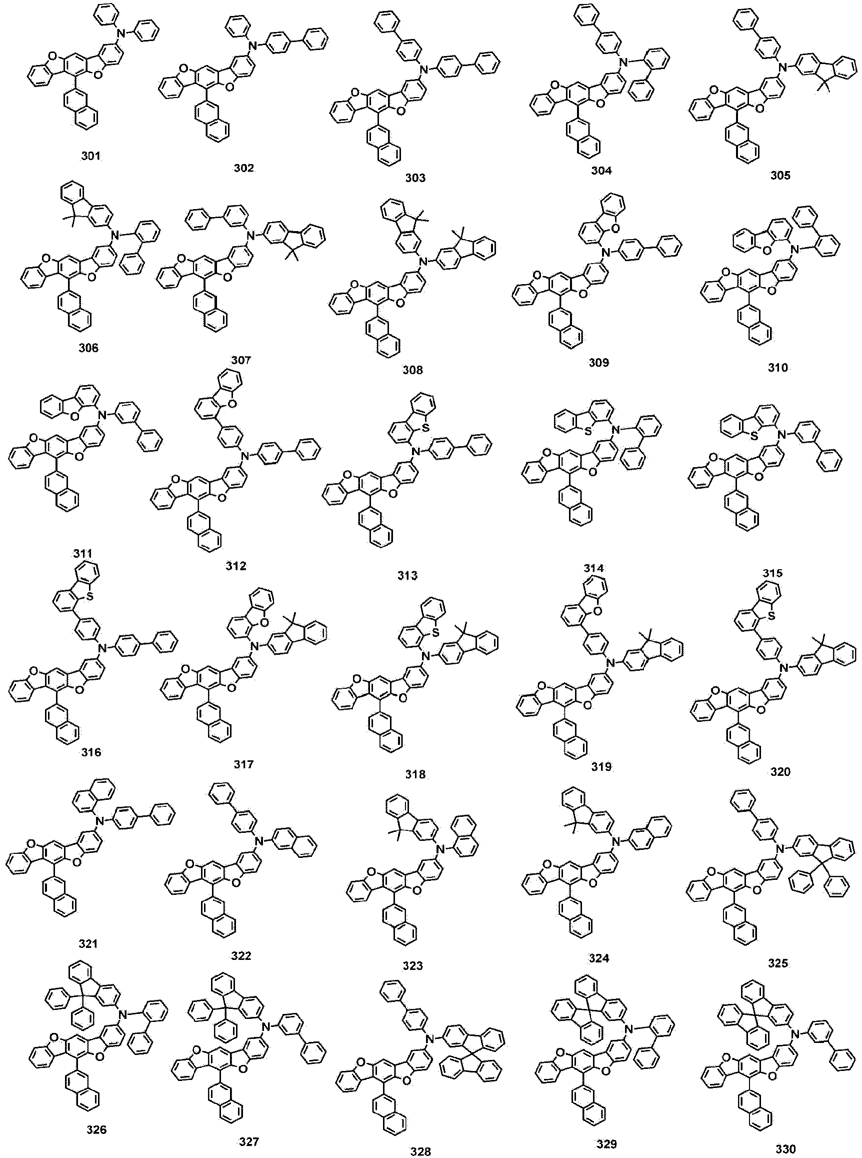

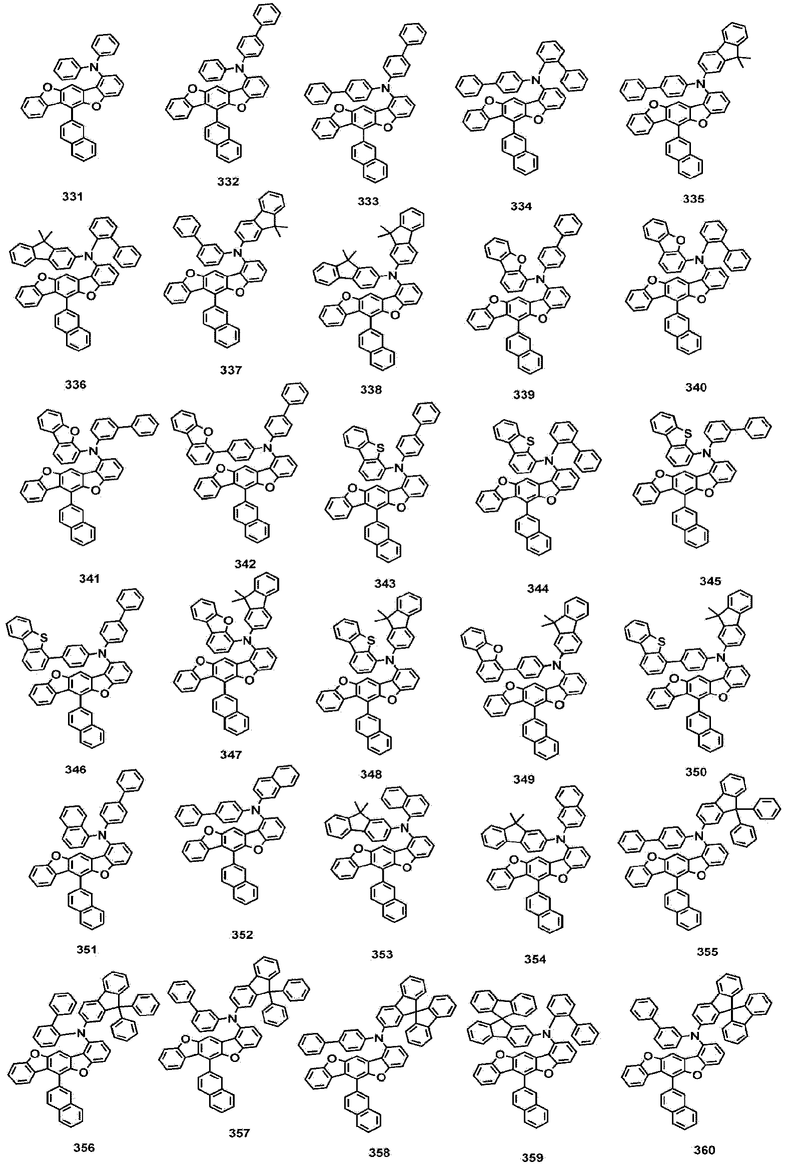

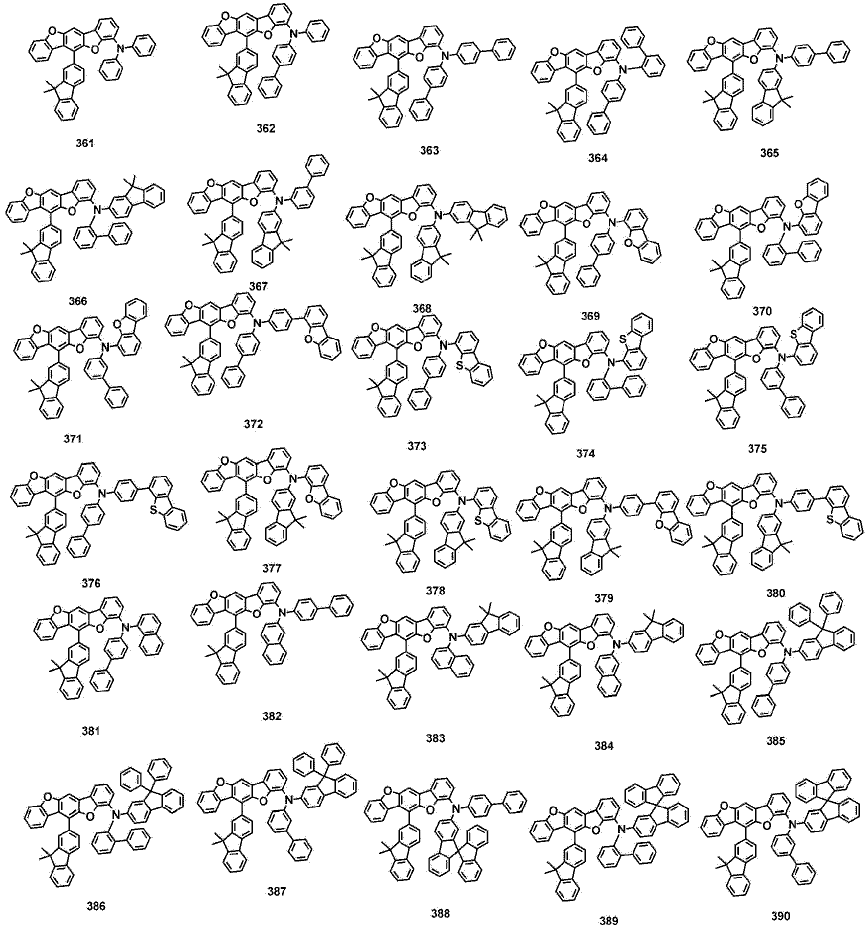

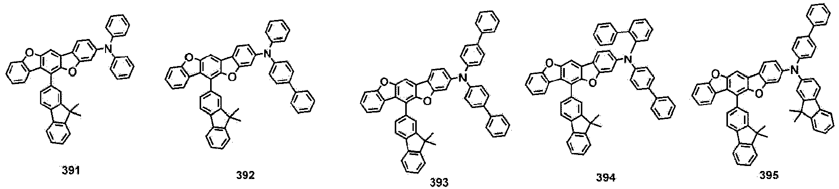

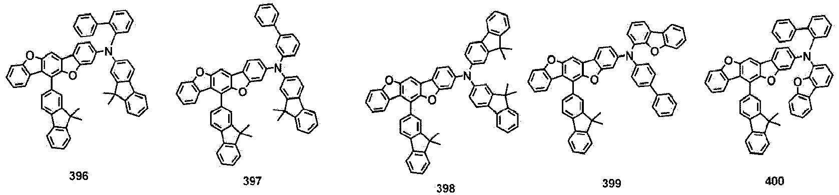

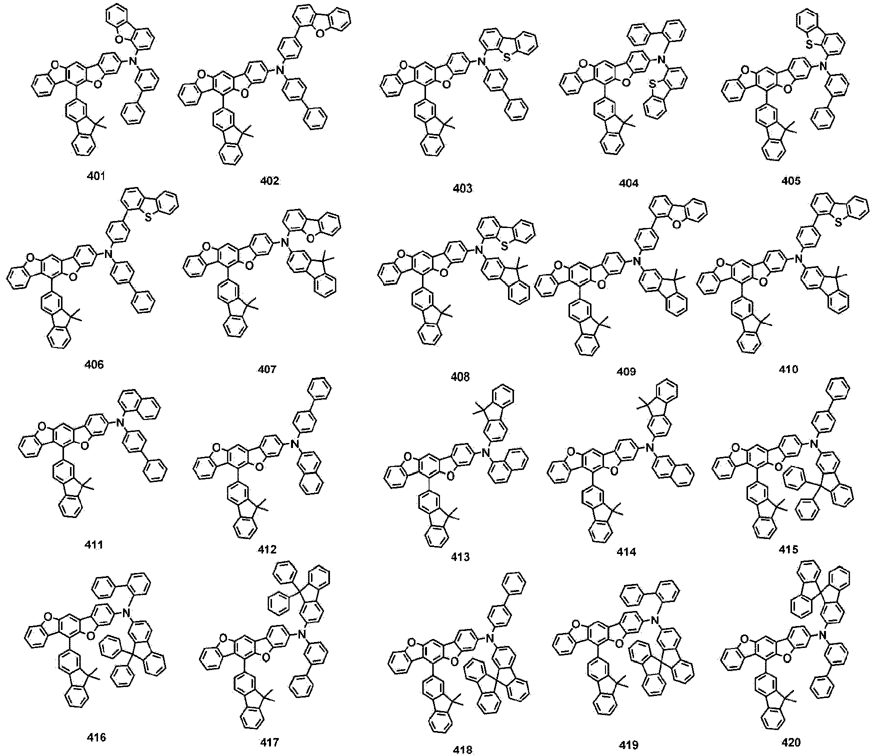

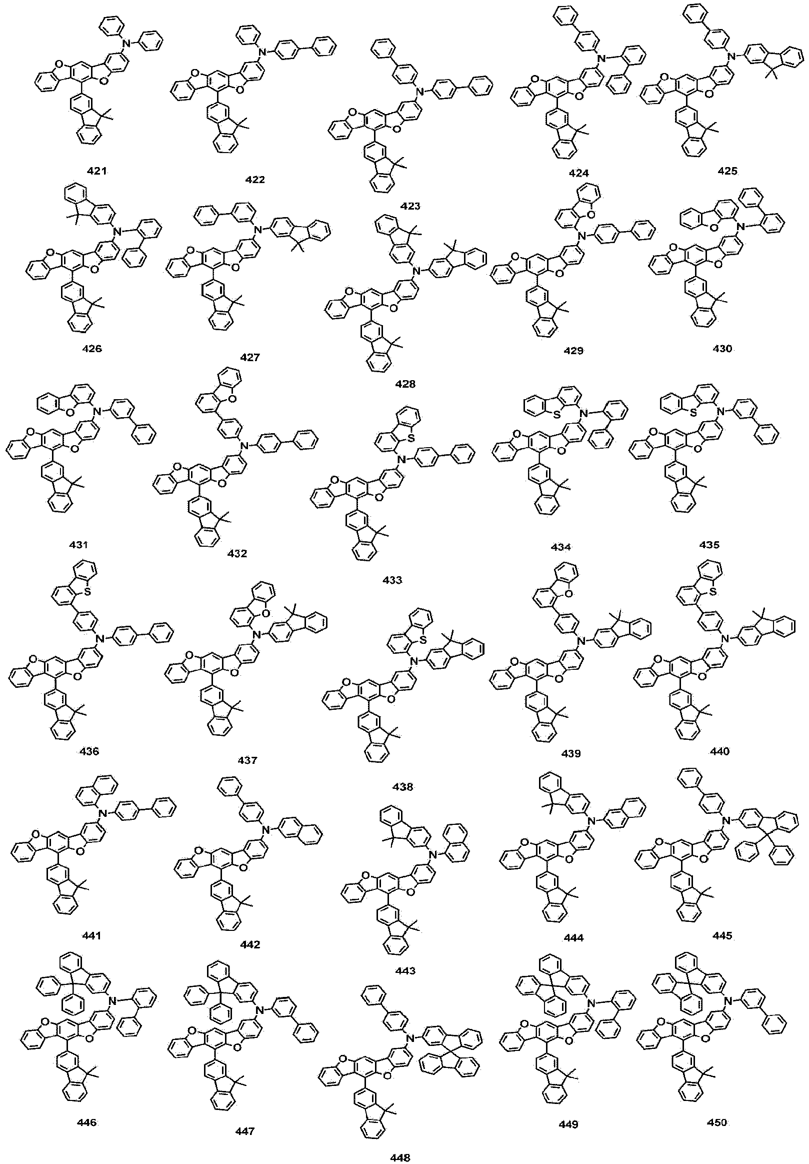

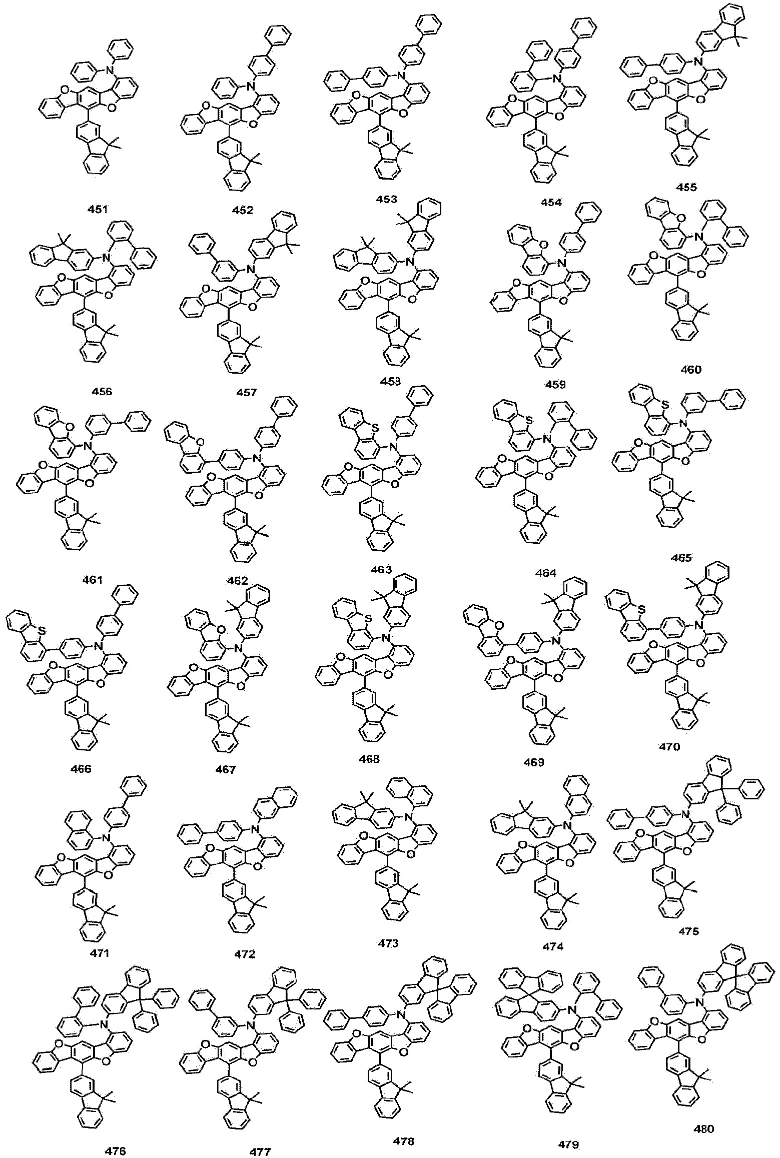

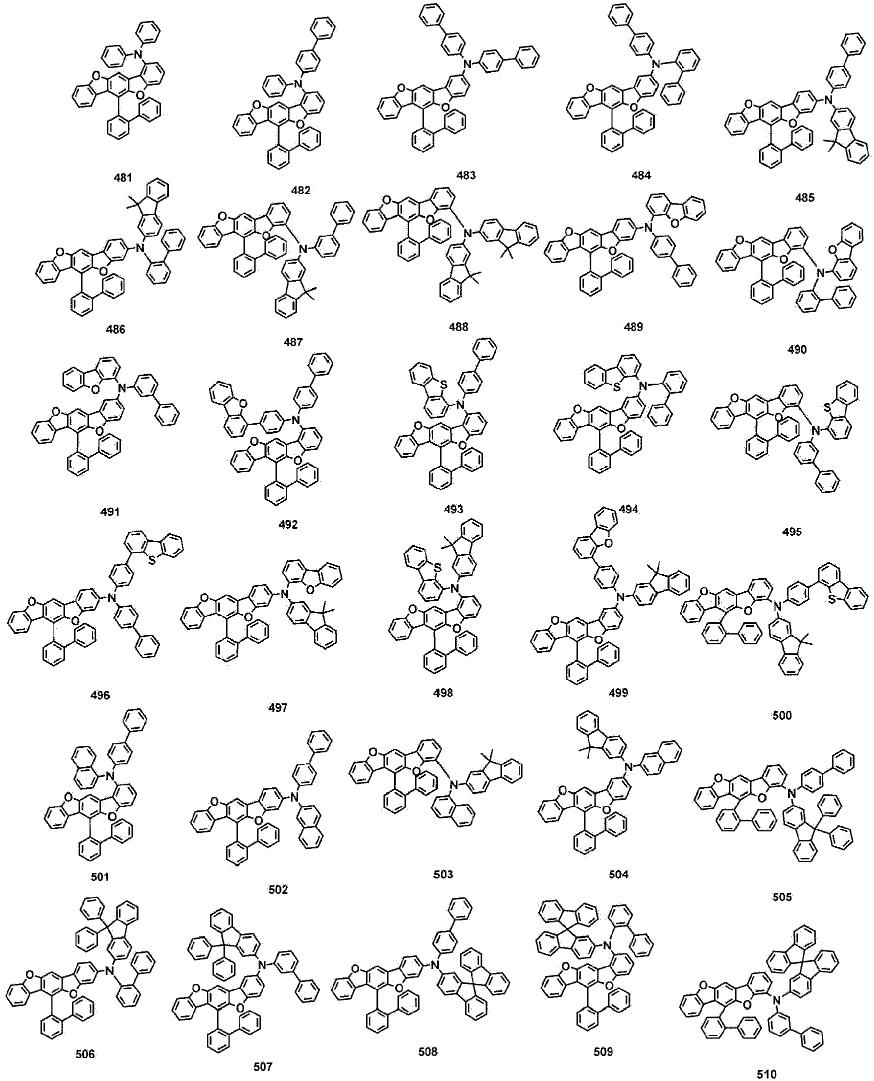

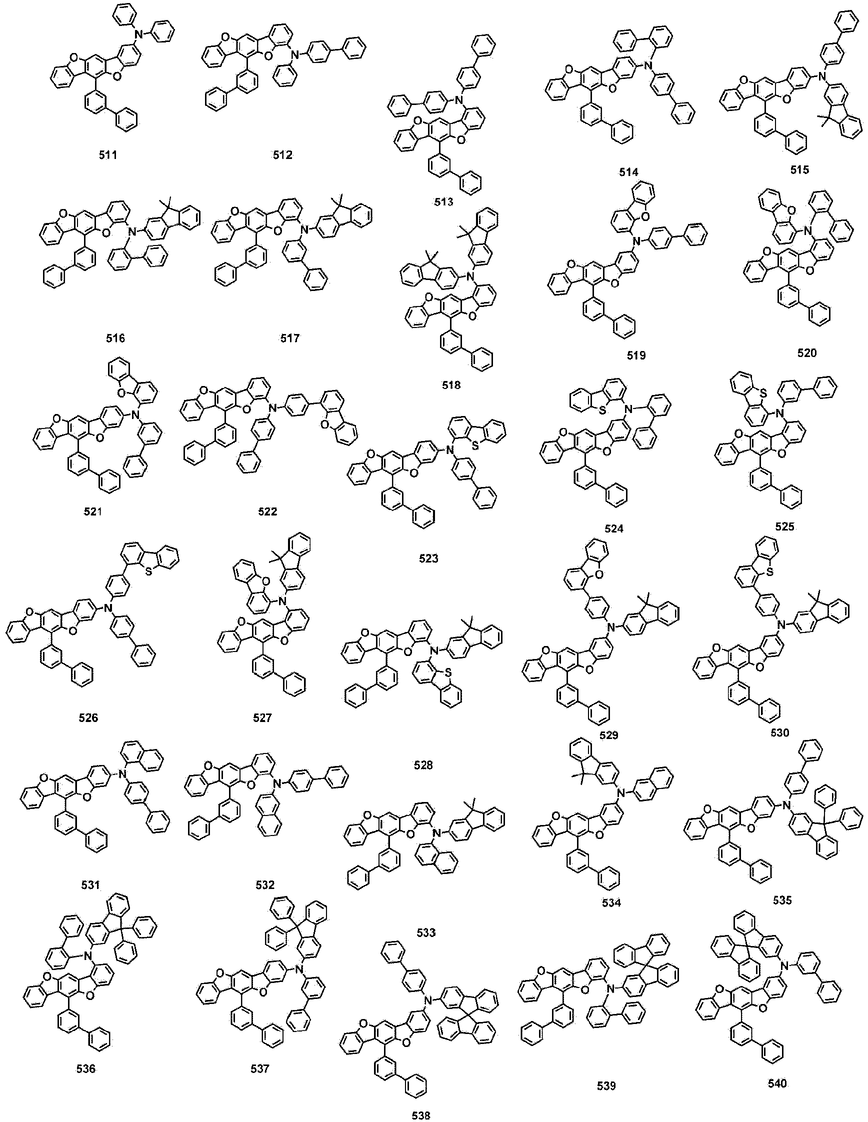

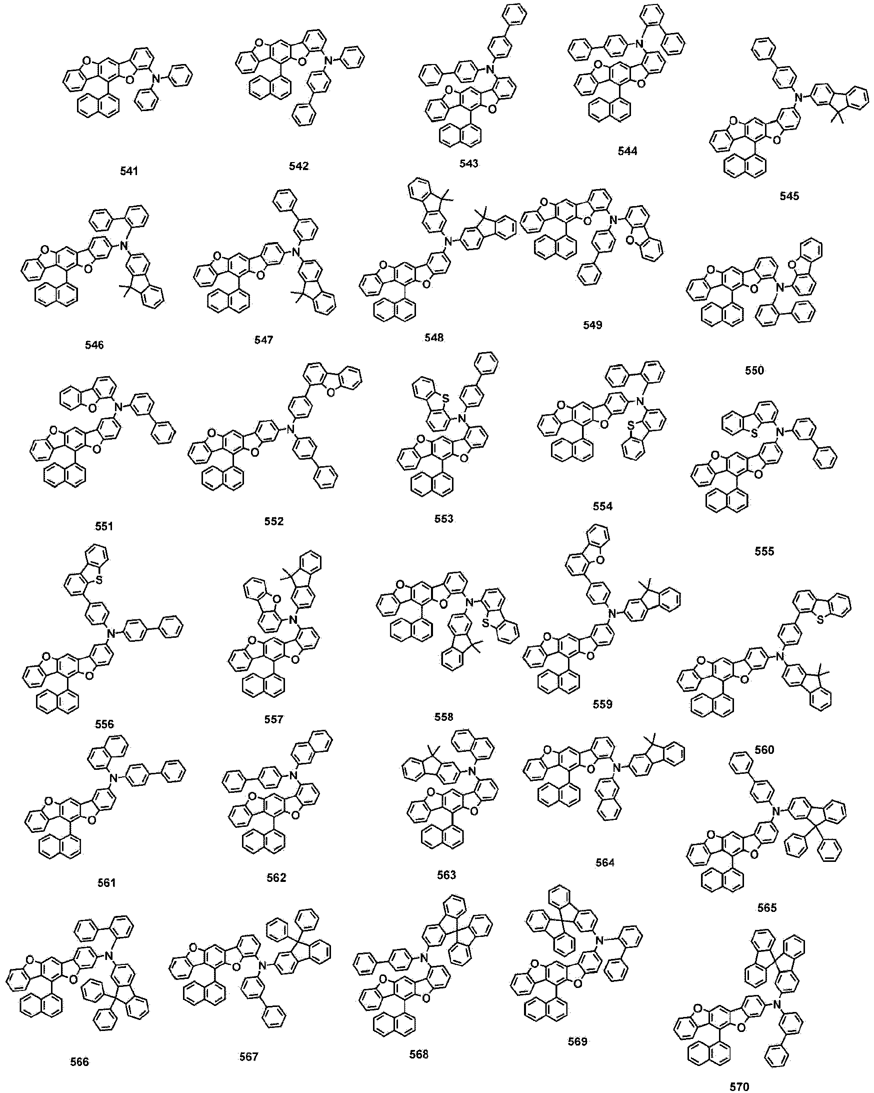

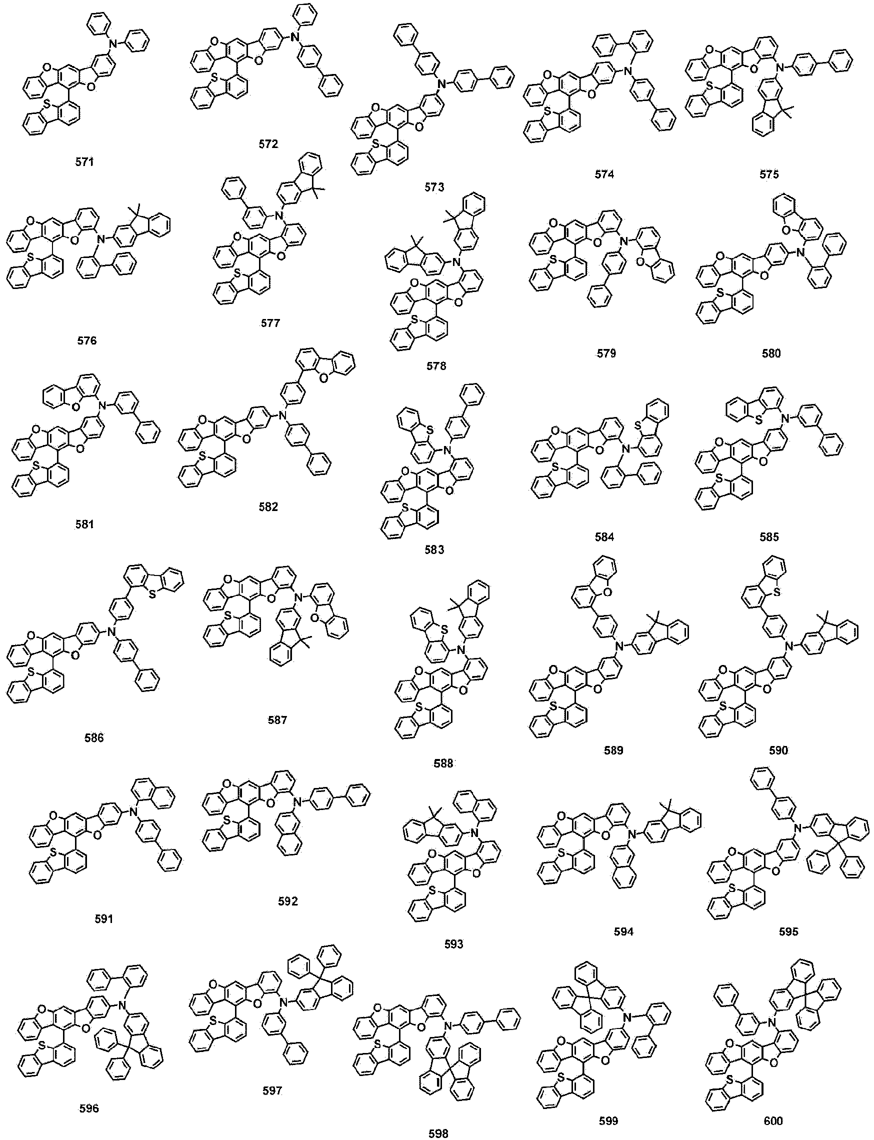

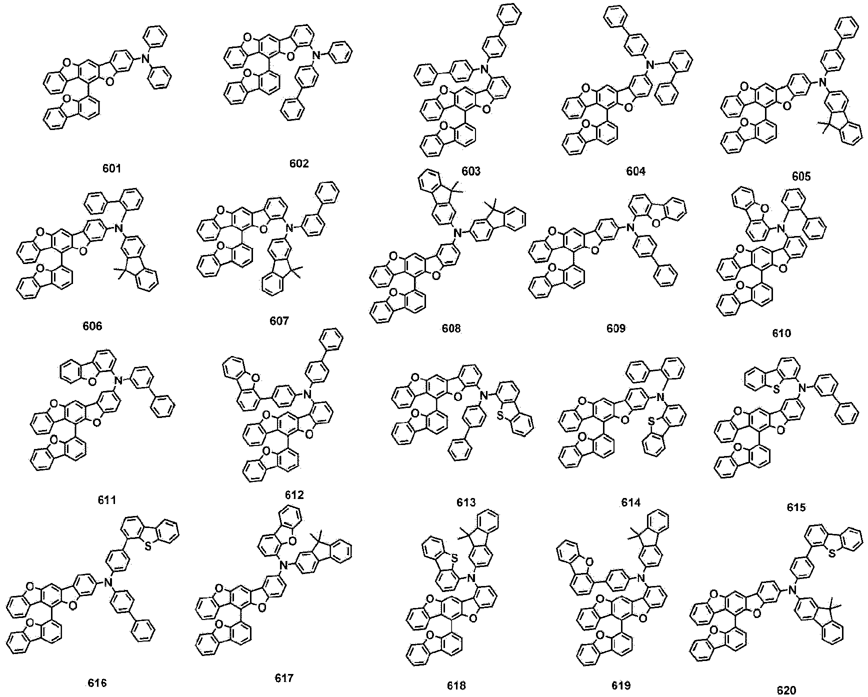

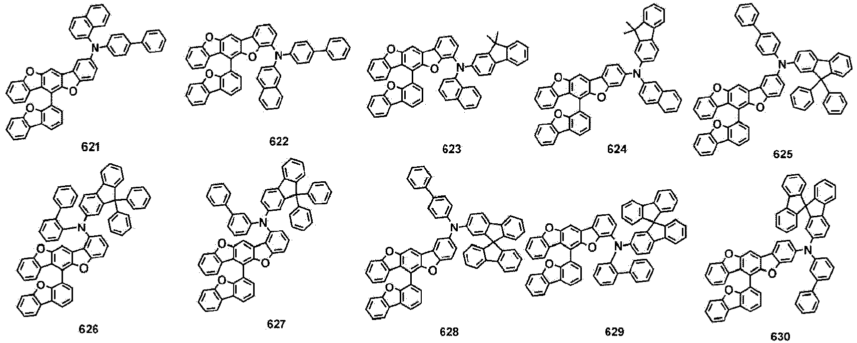

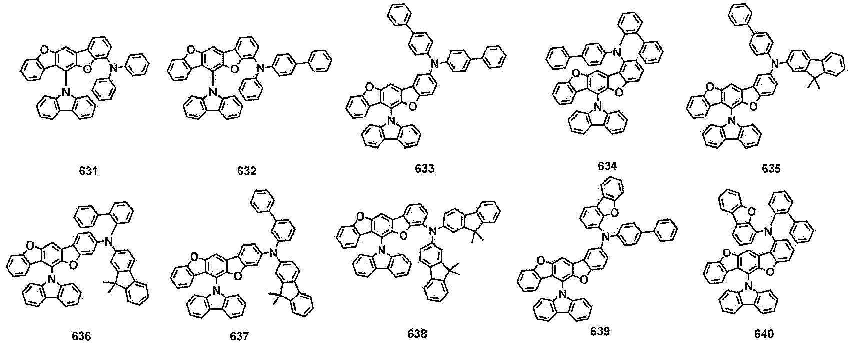

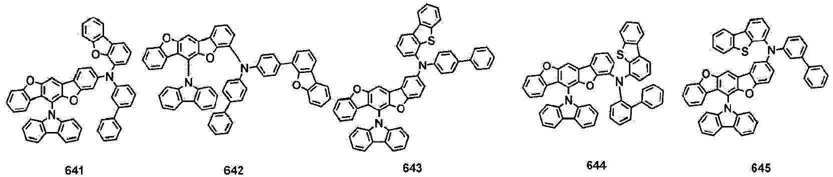

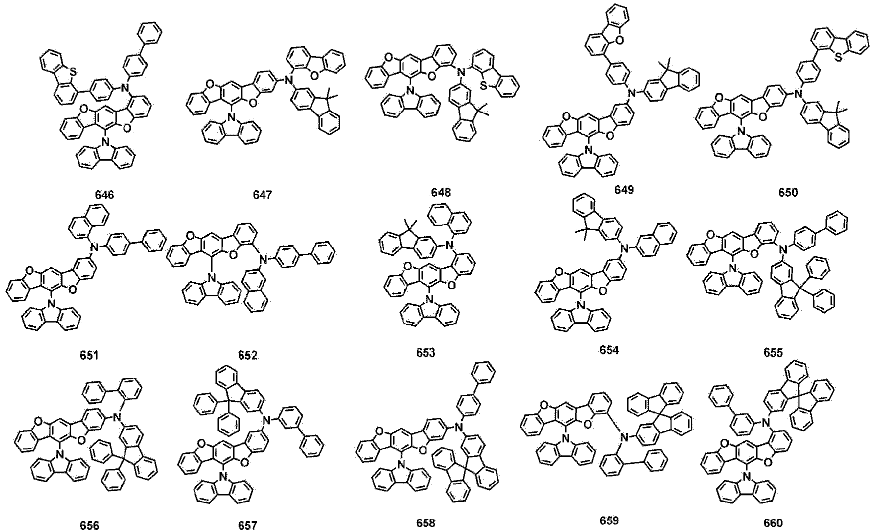

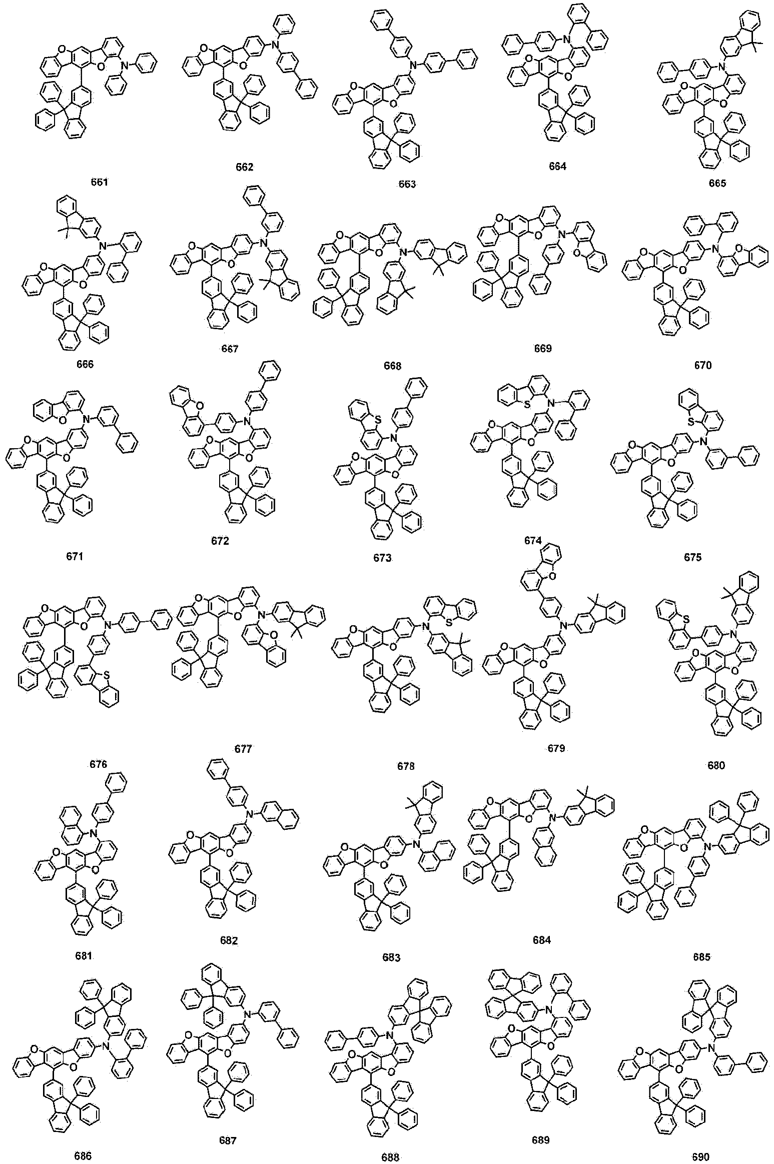

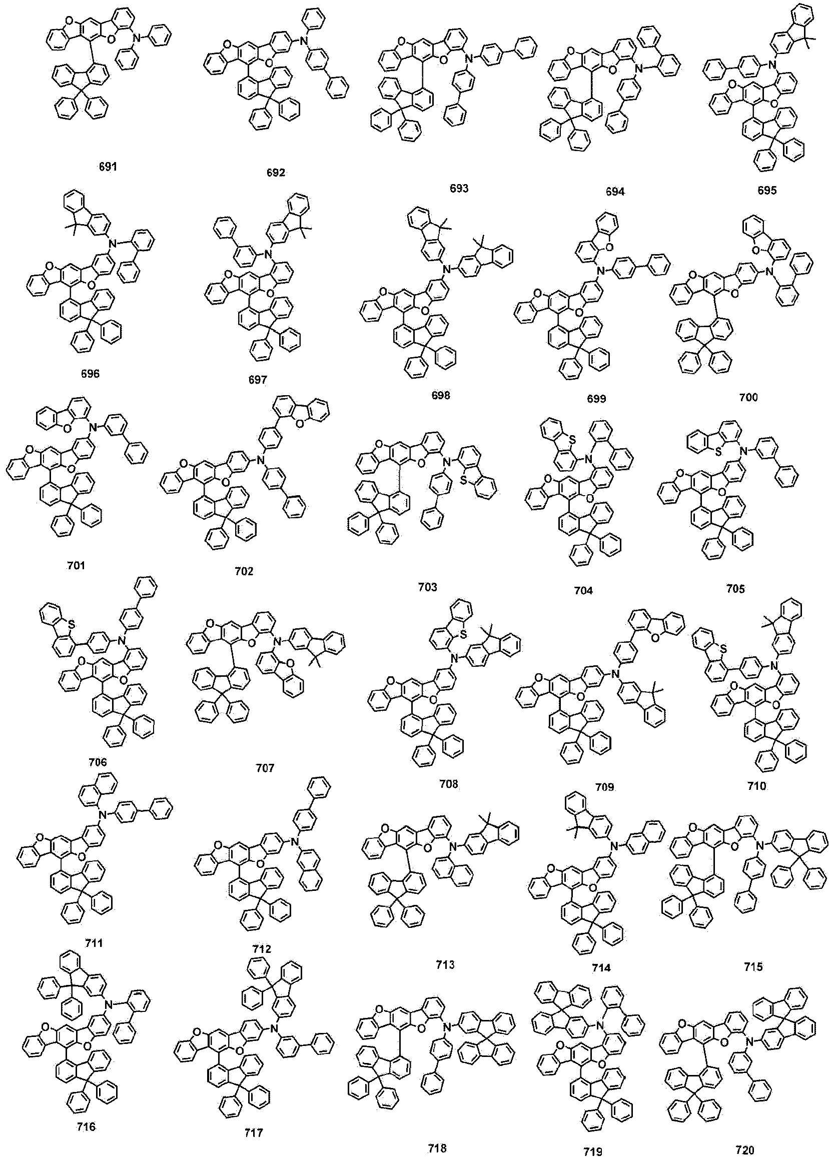

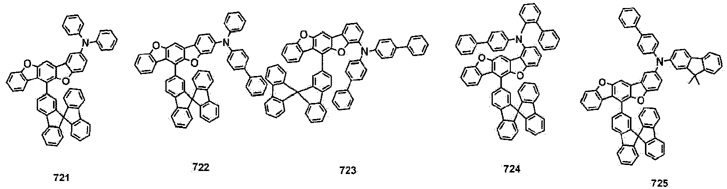

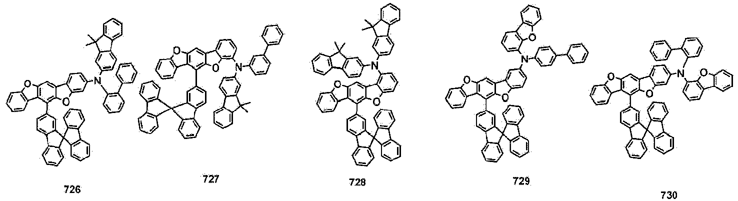

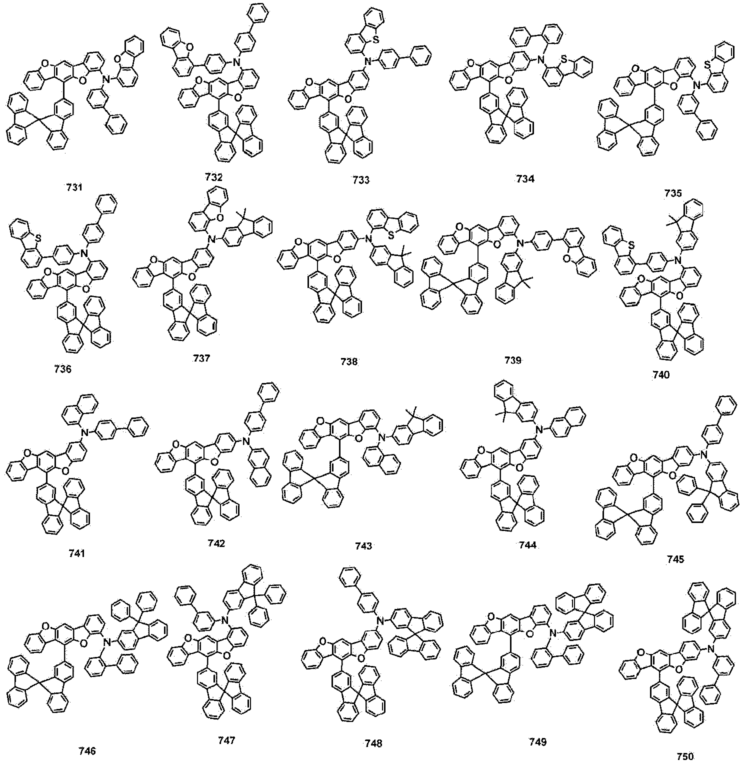

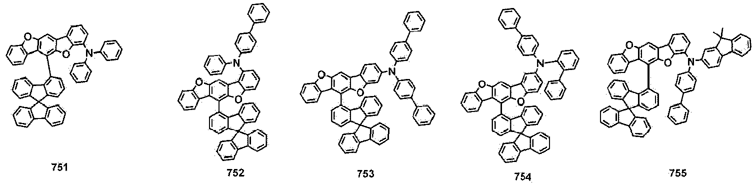

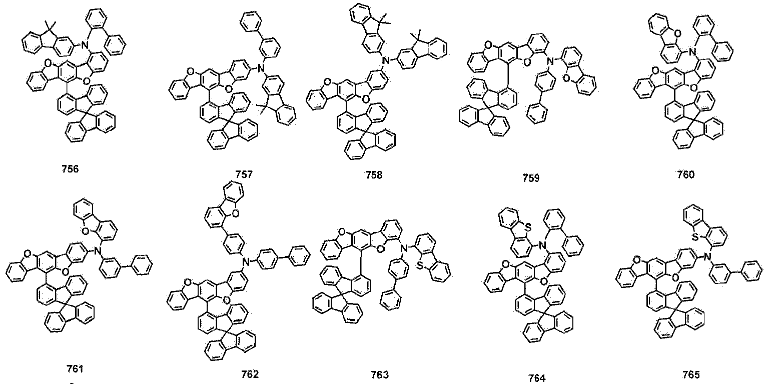

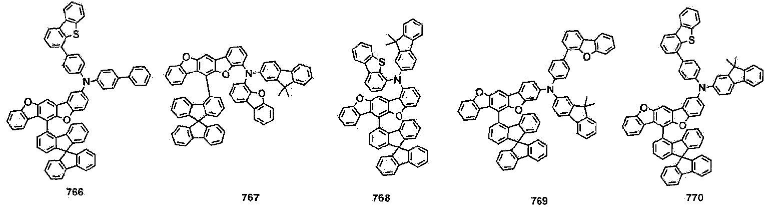

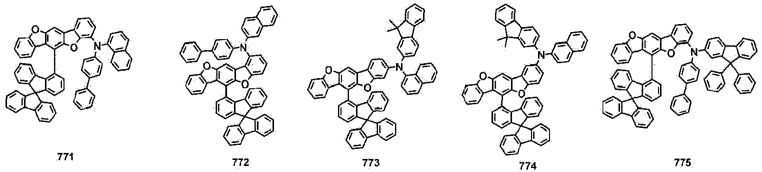

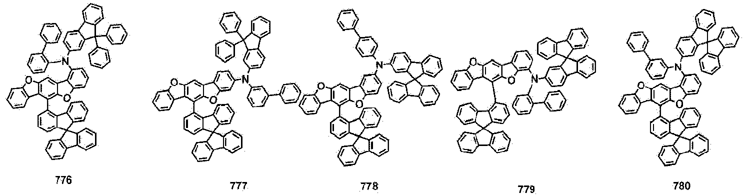

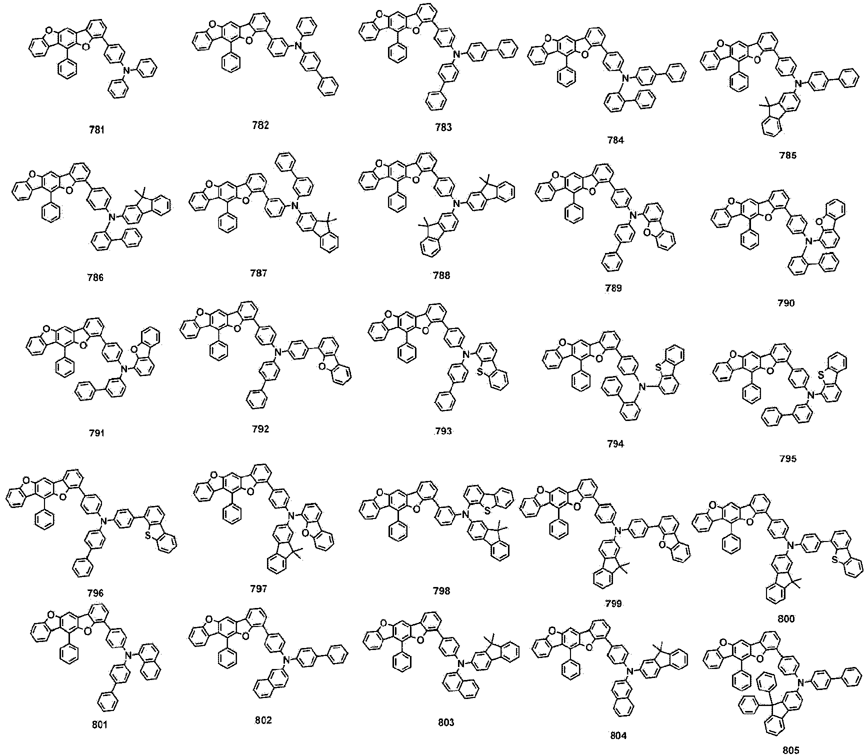

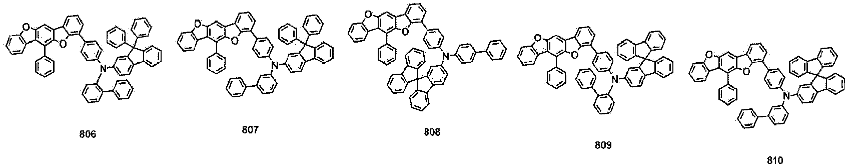

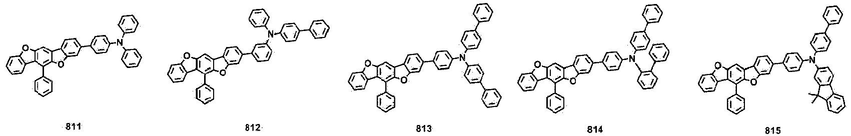

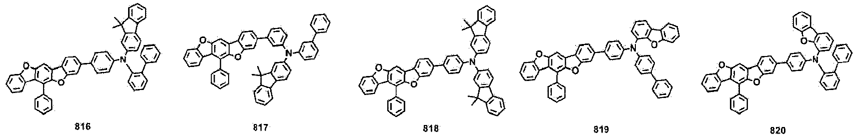

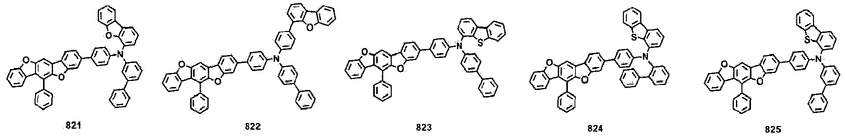

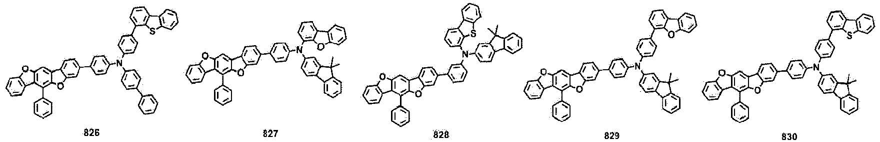

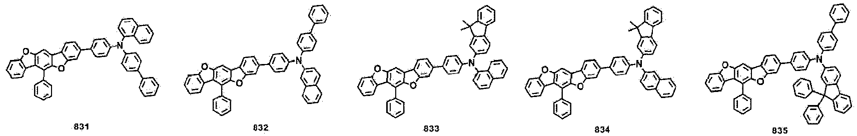

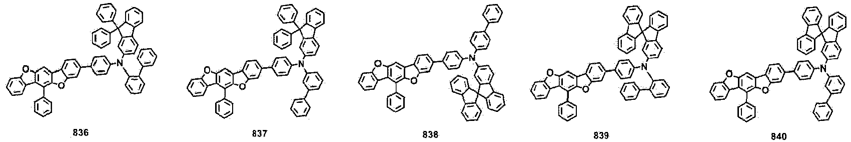

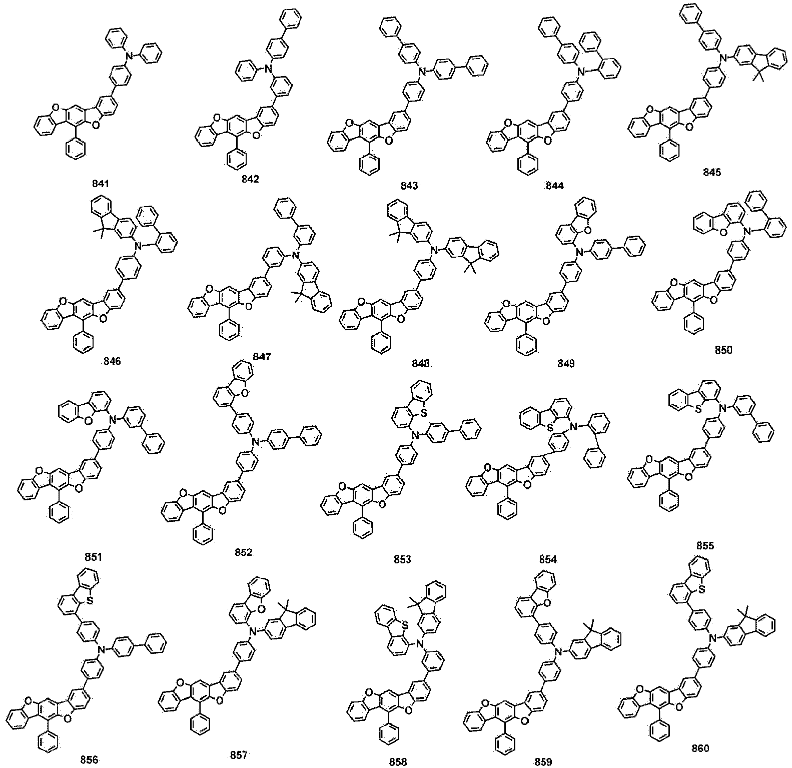

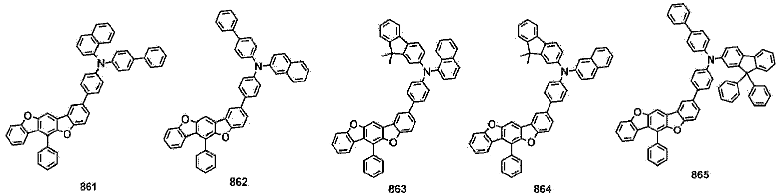

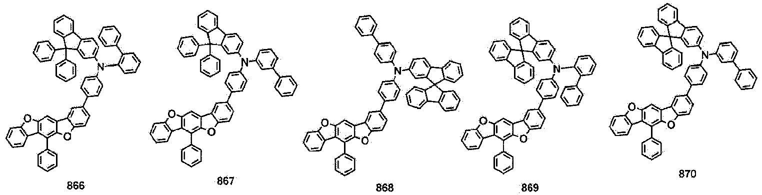

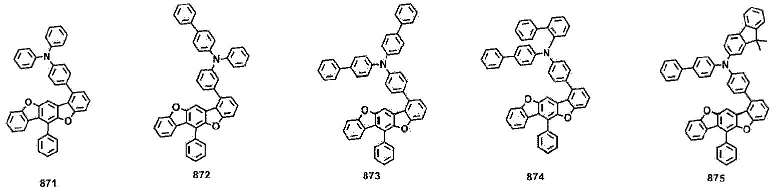

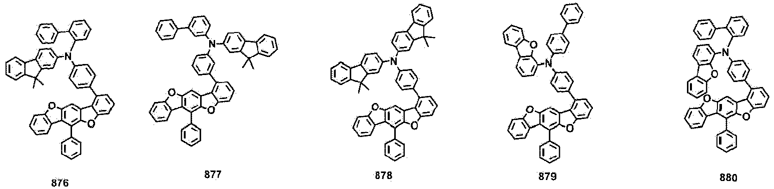

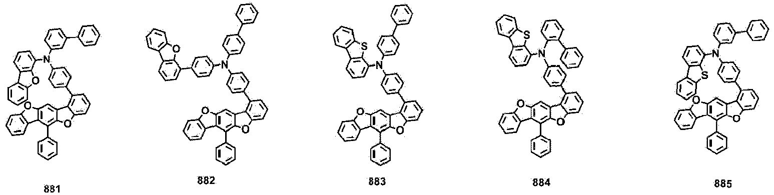

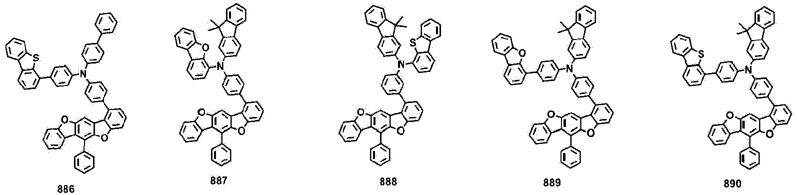

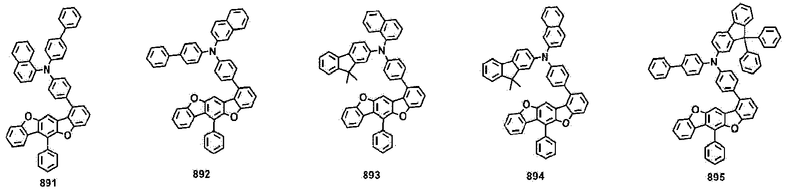

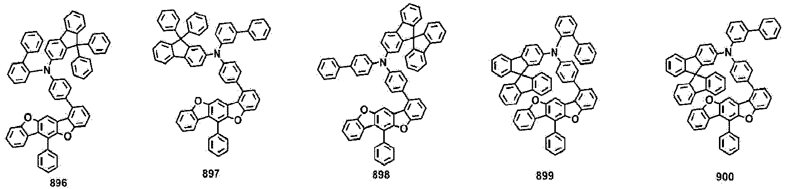

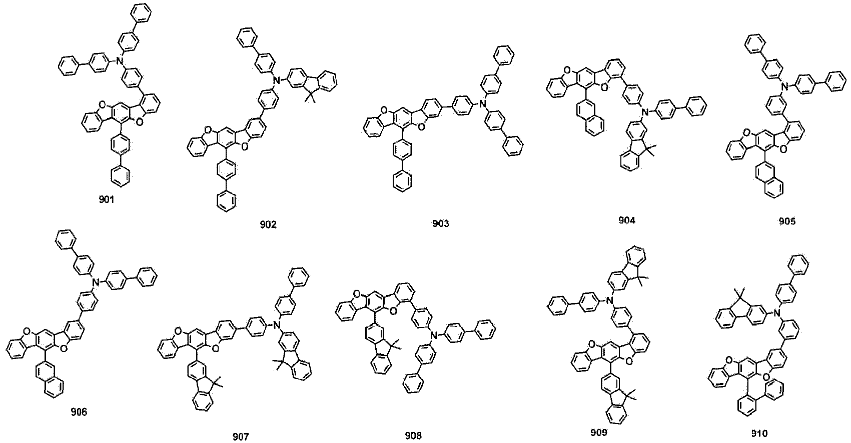

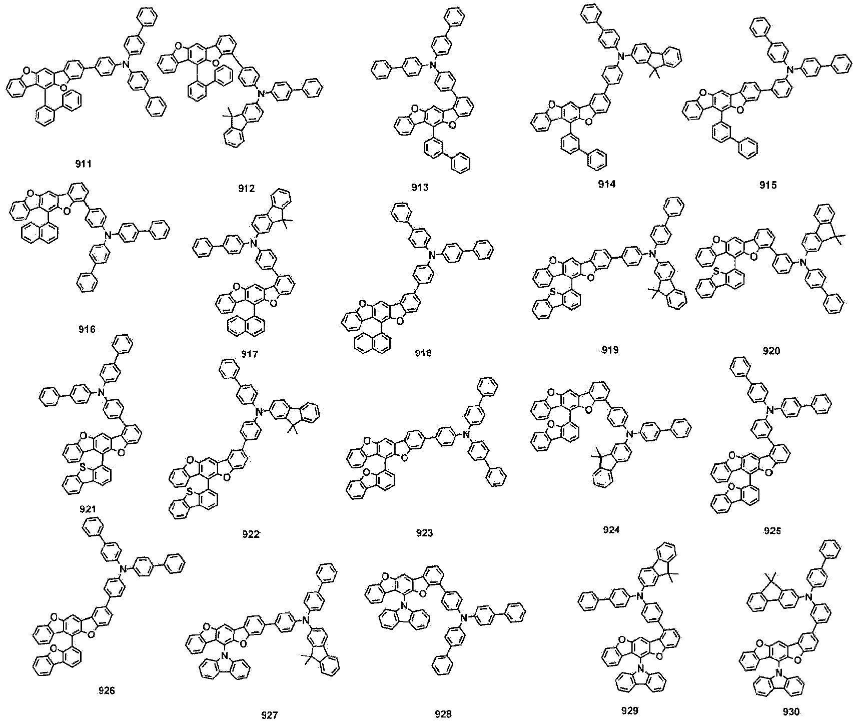

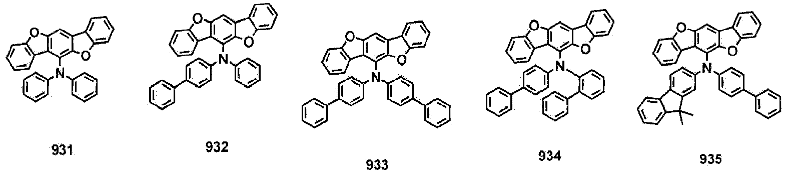

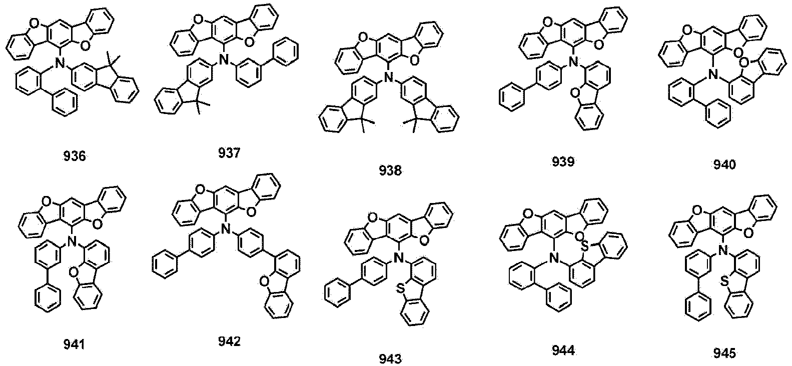

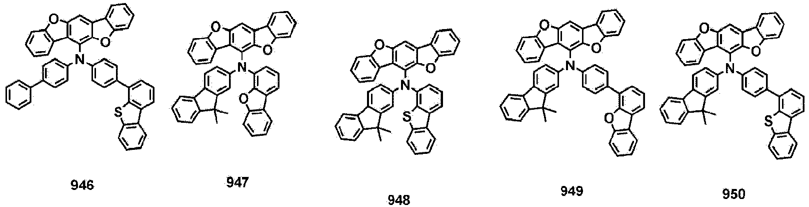

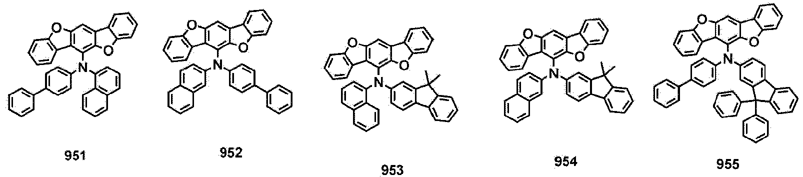

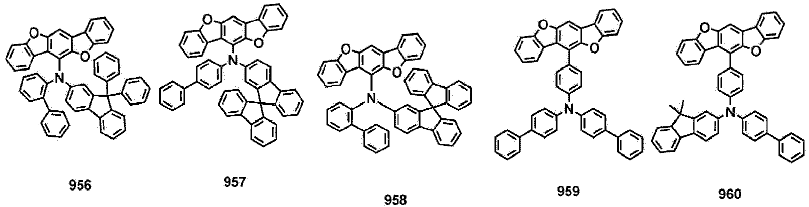

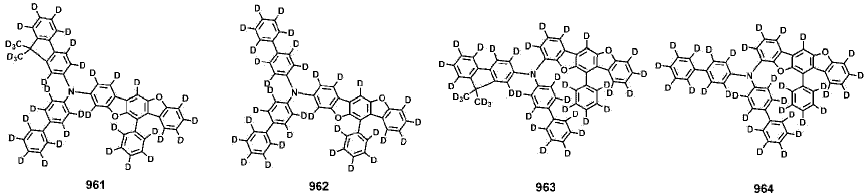

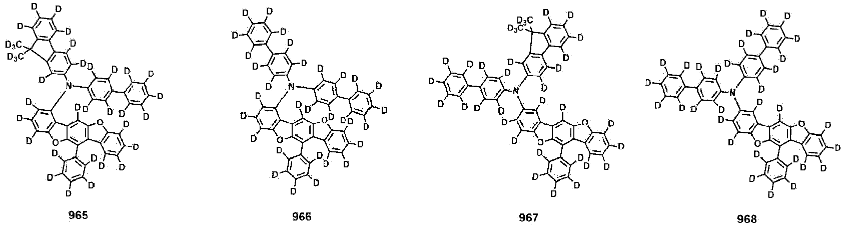

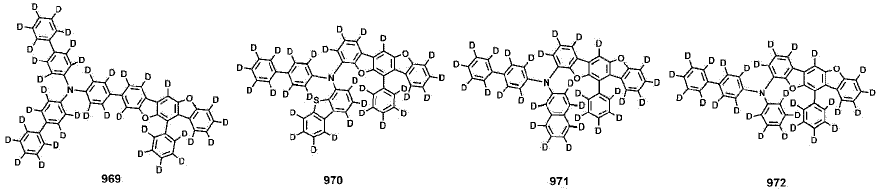

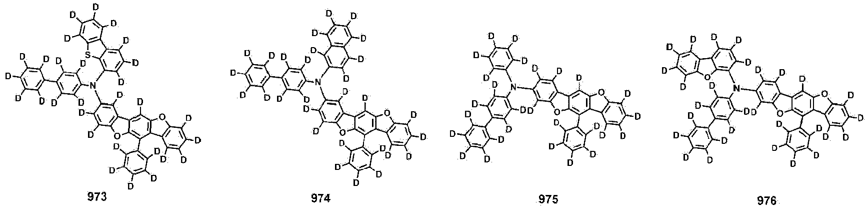

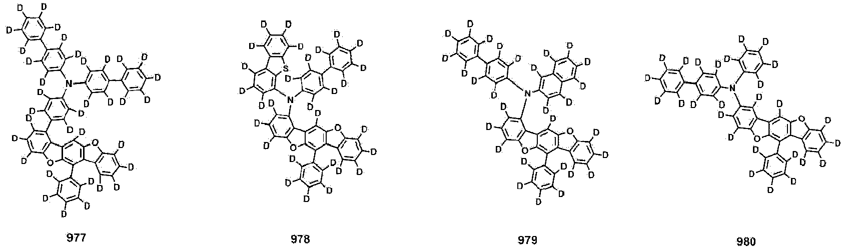

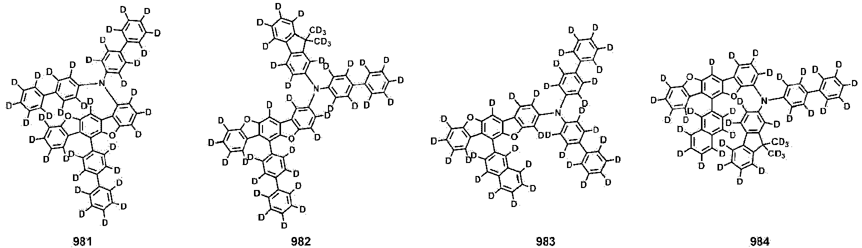

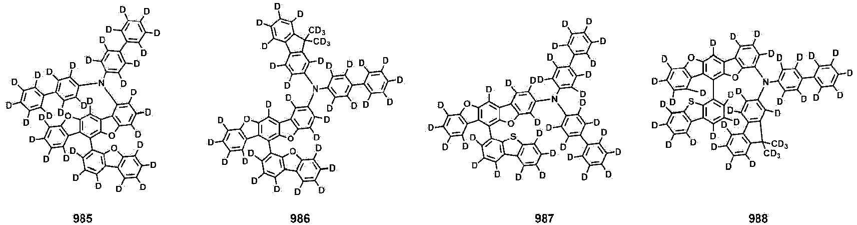

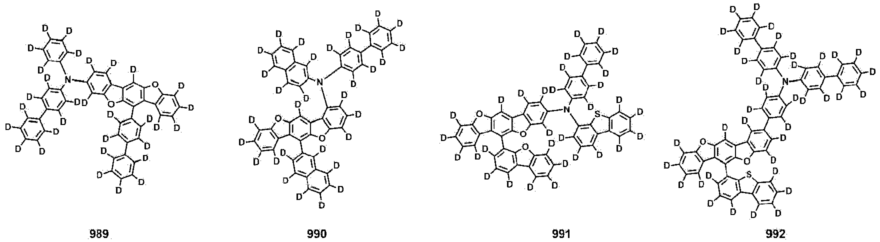

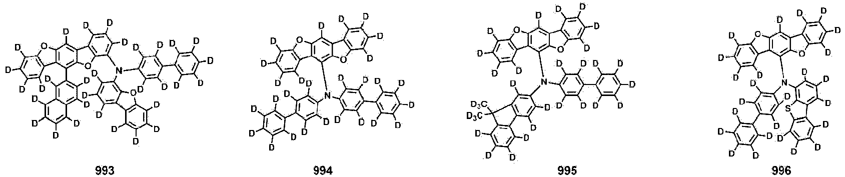

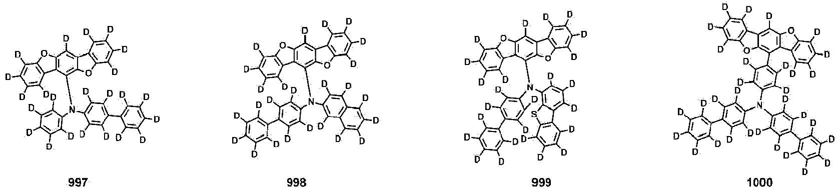

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

In addition, one embodiment of the present application provides an organic light emitting device including a first electrode; a second electrode provided opposite to the first electrode; and one or more organic material layers provided between the first electrode and the second electrode, wherein one or more layers of the organic material layers include the heterocyclic compound represented by Chemical Formula 1.

Another embodiment provides an organic light emitting device including a first electrode; a second electrode provided opposite to the first electrode; and one or more organic material layers provided between the first electrode and the second electrode, wherein one or more layers of the organic material layers include one type of the heterocyclic compound represented by Chemical Formula 1.

Specific details on the heterocyclic compound represented by Chemical Formula 1 are the same as the descriptions provided above.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound represented by Chemical Formula 1 may be used as a material of the blue organic light emitting device. For example, the heterocyclic compound represented by Chemical Formula 1 may be included in a host material of a blue light emitting layer of a blue organic light emitting device.

In one embodiment of the present application, the organic light emitting device may be a green organic light emitting device, and the heterocyclic compound represented by Chemical Formula 1 may be used as a material of the green organic light emitting device. For example, the heterocyclic compound represented by Chemical Formula 1 may be included in a host material of a green light emitting layer of a green organic light emitting device.

In one embodiment of the present application, the organic light emitting device may be a red organic light emitting device, and the heterocyclic compound represented by Chemical Formula 1 may be used as a material of the red organic light emitting device. For example, the heterocyclic compound represented by Chemical Formula 1 may be included in a host material of a red light emitting layer of a red organic light emitting device.

The organic light emitting device of the present disclosure may be manufactured using common organic light emitting device manufacturing methods and materials except that one or more organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, or may also be formed in a multilayer structure in which two or more organic material layers are laminated. For example, the organic light emitting device according to one embodiment of the present disclosure may have a structure including a hole injection layer, a hole transfer layer, a light emitting layer, an electron transfer layer, an electron injection layer and the like as the organic material layer. However, the structure of the organic light emitting device is not limited thereto, and may include a smaller number of organic material layers.

In the organic light emitting device of the present disclosure, the organic material layer may include a light emitting layer, and the light emitting layer may include the heterocyclic compound.

In another organic light emitting device, the organic material layer includes a light emitting layer, the light emitting layer includes a host material, and the host material may include the heterocyclic compound.

In another embodiment, the organic material layer including the heterocyclic compound includes the heterocyclic compound represented by Chemical Formula 1 as a host, and an iridium-based dopant may be used therewith.

In the organic light emitting device of the present disclosure, the organic material layer includes an electron injection layer or an electron transfer layer, and the electron injection layer or the electron transfer layer may include the heterocyclic compound.

In the organic light emitting device of the present disclosure, the organic material layer includes a hole transfer layer, and the hole transfer layer may include the heterocyclic compound.

In the organic light emitting device of the present disclosure, the organic material layer includes an electron blocking layer, and the electron blocking layer may include the heterocyclic compound.

In another organic light emitting device, the organic material layer includes an electron blocking layer or a hole blocking layer, and the electron blocking layer or the hole blocking layer may include the heterocyclic compound.

The organic light emitting device of the present disclosure may further include one, two or more layers selected from the group consisting of a light emitting layer, a hole injection layer, a hole transfer layer, an electron injection layer, an electron transfer layer, an electron blocking layer and a hole blocking layer.

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer including the heterocyclic compound of Chemical Formula 1 may further include other materials as necessary.

In one embodiment of the present application, the organic material layer including the heterocyclic compound of Chemical Formula 1 may also include a triazine-based compound, a 1,2,4-triazole-based compound or a biscarbazole-based compound.

When the organic material layer includes a triazine-based compound, a 1,2,4-triazole-based compound or a biscarbazole-based compound together with the heterocyclic compound of Chemical Formula 1 in the organic material layer of the organic light emitting device, effects of more superior efficiency and lifetime are obtained. Such results may lead to a forecast that an exciplex phenomenon occurs when including the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

In one embodiment of the present application, the triazine-based compound may be represented by the following Chemical Formula X. 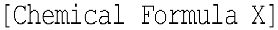 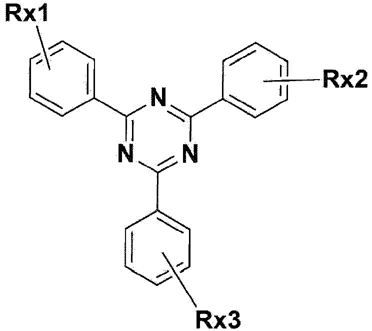 In Chemical Formula X,
Rx1 to Rx3 are the same as or different from each other, and may be each independently hydrogen; a substituted or unsubstituted C6 to C60 aryl group; or a substituted or unsubstituted C2 to C60 heteroaryl group.

In one embodiment of the present application, the 1,2,4-triazole-based compound may be represented by the following Chemical Formula Y. 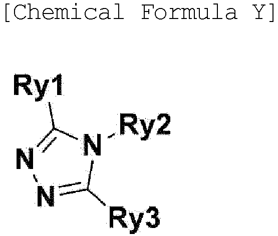 In Chemical Formula Y,
Ry1 to Ry3 are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In the organic light emitting device according to one embodiment of the present application, materials other than the heterocyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Compound 033

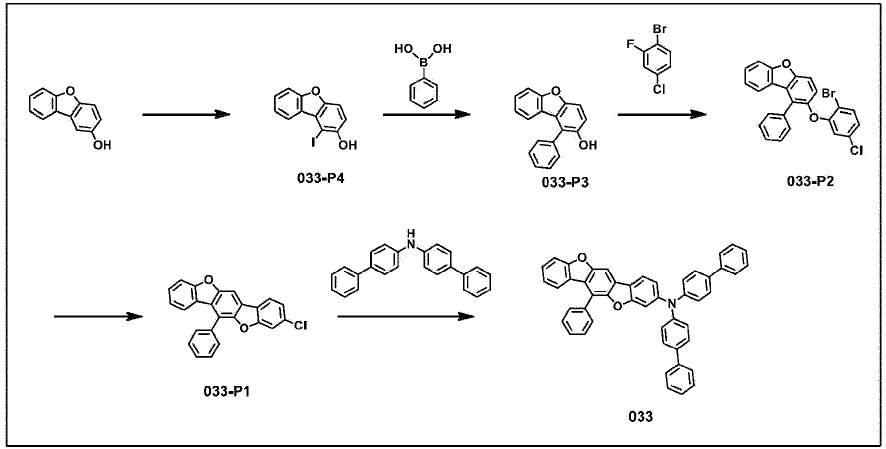

### 1) Preparation of Compound 033-P4

Dibenzo[b,d]furan-2-ol (150 g, 814.38 mmol) was introduced to acetic acid (600 ml), and the solution was stirred. Iodine monochloride (132.22 g, 814.38 mmol), HCl (195 ml) and acetic acid (345 ml) were mixed and then introduced to the solution prepared above, and the result was stirred for 12 hours at room temperature. After the reaction was completed, distilled water (3 L) was introduced thereto, and produced solids were filtered and then purified by column chromatography using dichloromethane and hexane as a developing solvent to obtain Compound 033-P4 (155 g, 61%).

### 2) Preparation of Compound 033-P3

After dissolving Compound 033-P4 (118 g, 380.53 mmol) and phenylboronic acid (51.04 g, 418.58 mmol) in tetrahydrofuran (THF) (1000 ml) and distilled water (200 ml), Pd(PPh₃)₄ (13.19 g, 11.42 mmol) and K₂CO₃ (131.48 g, 951.33 mmol) were introduced thereto, and the result was stirred for 12 hours under reflux. After the reaction was completed, ethyl acetate was introduced to the reaction solution for dissolution, and the result was extracted with distilled water. The organic layer was dried with anhydrous MgSO₄, and after removing the solvent using a rotary evaporator, the result was purified by column chromatography using dichloromethane and hexane as a developing solvent to obtain Compound 033-P3 (90 g, 91%).

### 3) Preparation of Compound 033-P2

After dissolving Compound 033-P3 (90 g, 345.77 mmol) and 1-bromo-4-chloro-2-fluorobenzene (79.66 g, 418.58 mmol) in N,N-dimethylacetamide (1000 ml) and then heating the mixture to 150°C, Cs₂CO₃ (225.32 g, 691.54 mmol) was introduced thereto, and the result was stirred for 30 minutes under reflux. After the reaction was completed, the result was extracted with dichloromethane and distilled water. The organic layer was dried with anhydrous MgSO₄, and after removing the solvent using a rotary evaporator, the result was purified by column chromatography using dichloromethane and hexane as a developing solvent to obtain Compound 033-P2 (130 g, 83%).

### 4) Preparation of Compound 033-P1

After dissolving Compound 033-P2 (130 g, 289.07 mmol) in N,N-dimethylacetamide (1000 ml), Pd(PPh₃)₄ (10.02 g, 8.67 mmol), Na₂CO₃ (61.28 g, 578.14 mmol) and PPh₃ (7.58 g, 28.91 mmol) were introduced thereto, and the result was stirred for 12 hours under reflux. After the reaction was completed, the result was extracted with dichloromethane and distilled water. The organic layer was dried with anhydrous MgSO₄, and after removing the solvent using a rotary evaporator, the result was purified by column chromatography using dichloromethane and hexane as a developing solvent to obtain Compound 033-P1 (76 g, 71%).

### 5) Preparation of Compound 033

After dissolving Compound 033-P1 (10 g, 27.11 mmol) and di([1,1'-biphenyl]-4-yl)amine (9.15 g, 28.47 mmol) in xylene (100 ml), Pd₂(dba)₃ (1.24 g, 1.36 mmol), P(t-Bu)₃ (1.26 ml, 2.71 mmol) and t-BuONa (6.51 g, 67.79 mmol) were introduced thereto, and the result was stirred for 3 hours under reflux. After the reaction was completed, methylene chloride (MC) was introduced to the reaction solution for dissolution, and the result was extracted with distilled water. The organic layer was dried with anhydrous MgSO₄, and after removing the solvent using a rotary evaporator, the result was purified by column chromatography using dichloromethane and hexane as a developing solvent to obtain Compound 033 (13 g, 73%).

Target compounds of the following Table 1 were synthesized in the same manner as in Preparation Example 1 except that Compound A of the following Table 1 was used instead of phenylboronic acid, Compound B of the following Table 1 was used instead of 1-bromo-4-chloro-2-fluorobenzene, and Compound C of the following Table 1 was used instead of di([1,1'-biphenyl]-4-yl)amine.

**[Table 1]**

| Compound No. | Compound A | Compound B | Compound C | Target Compound | Yield |
|---|---|---|---|---|---|
| 035 | 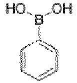 | 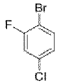 | 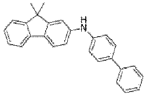 | 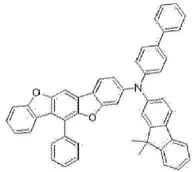 | 66% |
| 060 | 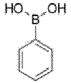 | 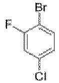 | 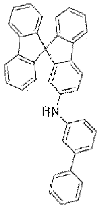 | 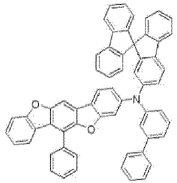 | 71% |
| 077 | 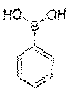 | 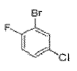 | 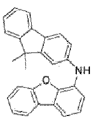 | 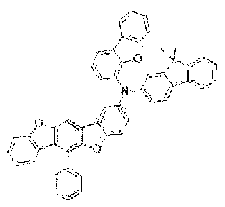 | 68% |
| 096 | 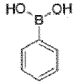 | 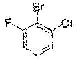 | 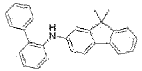 | 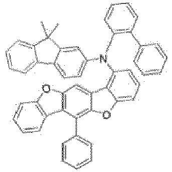 | 69% |
| 119 | 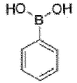 | 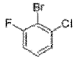 | 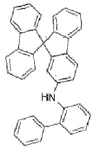 | 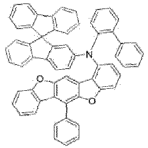 | 74% |
| 124 | 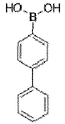 | 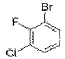 | 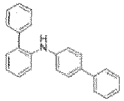 | 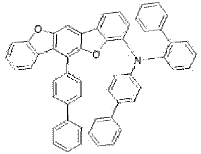 | 79% |
| 137 | 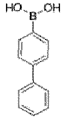 | 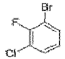 | 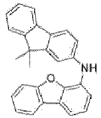 | 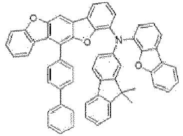 | 80% |
| 162 | 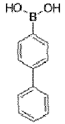 | 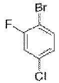 | 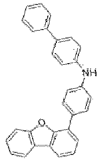 | 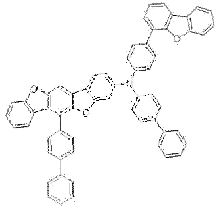 | 73% |
| 173 | 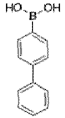 | 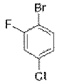 | 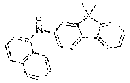 | 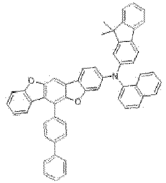 | 71% |
| 182 | 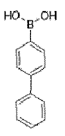 | 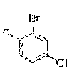 | 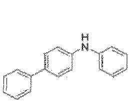 | 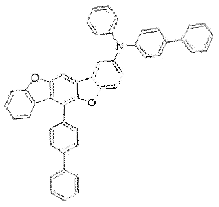 | 73% |
| 199 | 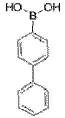 | 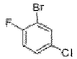 | 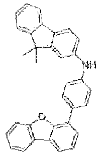 | 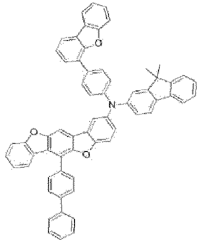 | 72% |
| 221 | 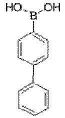 | 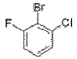 | 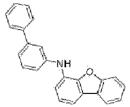 | 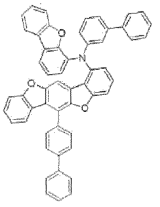 | 67% |
| 236 | 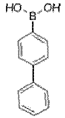 | 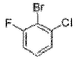 | 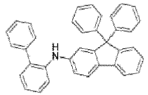 | 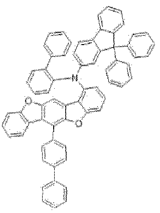 | 80% |
| 249 | 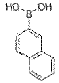 | 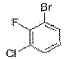 | 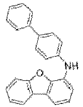 | 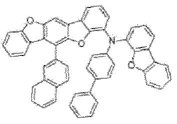 | 71% |
| 258 | 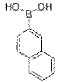 | 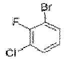 | 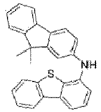 | 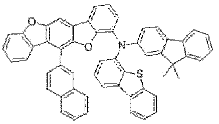 | 65% |
| 272 | 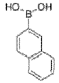 | 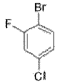 | 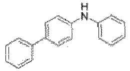 | 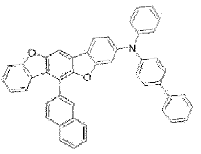 | 81% |
| 295 | 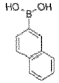 | 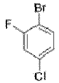 | 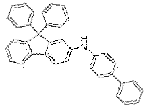 | 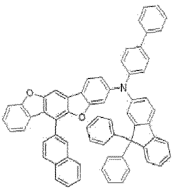 | 61% |
| 311 | 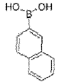 | 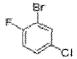 | 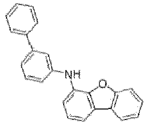 | 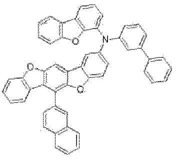 | 73% |
| 321 | 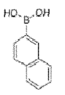 | 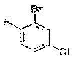 | 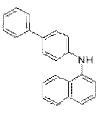 | 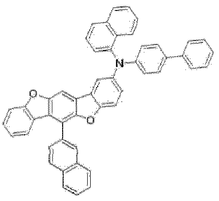 | 75% |
| 337 | 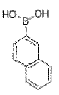 | 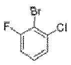 | 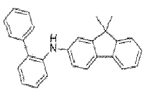 | 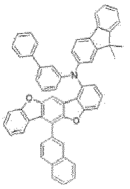 | 77% |
| 354 | 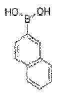 | 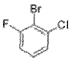 | 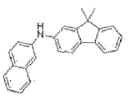 | 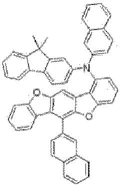 | 81% |
| 363 | 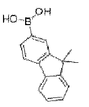 | 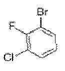 | 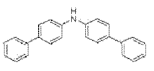 | 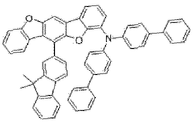 | 78% |
| 374 | 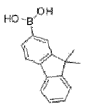 | 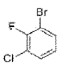 | 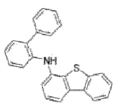 | 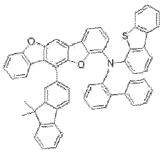 | 74% |
| 392 | 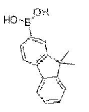 | 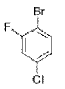 | 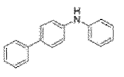 | 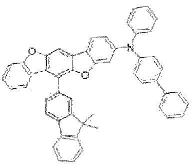 | 69% |
| 395 | 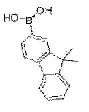 | 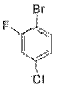 | 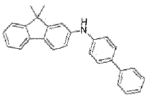 | 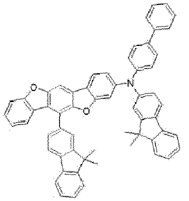 | 73% |
| 403 | 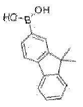 | 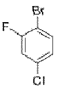 | 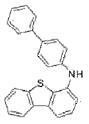 | 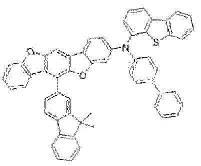 | 82% |
| 411 | 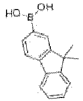 | 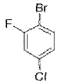 | 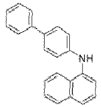 | 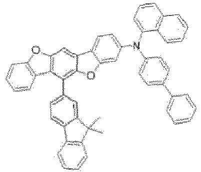 | 77% |
| 453 | 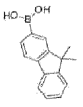 | 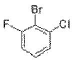 | 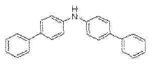 | 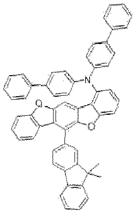 | 70% |
| 455 | 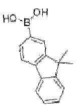 | 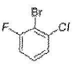 | 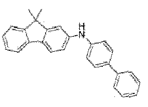 | 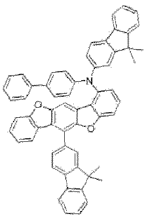 | 71% |
| 458 | 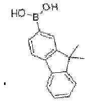 | 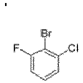 | 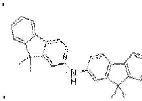 | 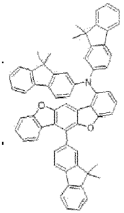 | 75% |
| 467 | 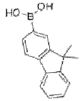 | 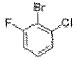 | 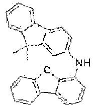 | 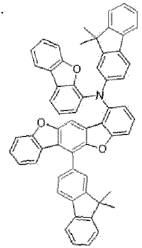 | 72% |
| 532 | 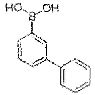 | 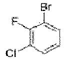 | 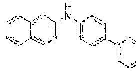 | 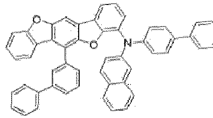 | 78% |
| 546 | 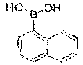 | 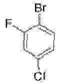 | 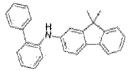 | 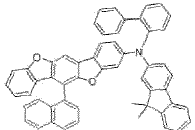 | 72% |
| 547 | 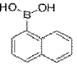 | 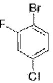 | 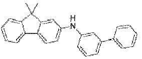 | 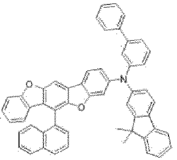 | 69% |
| 557 | 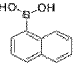 | 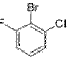 | 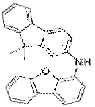 | 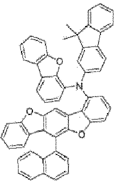 | 78% |
| 580 | 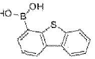 | 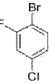 | 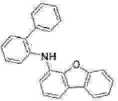 | 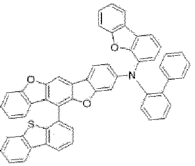 | 71% |
| 603 | 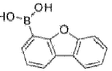 | 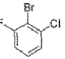 | 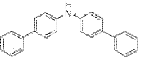 | 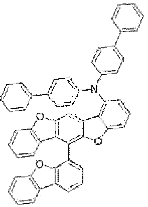 | 76% |
| 606 | 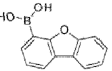 | 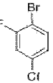 | 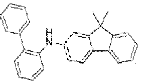 | 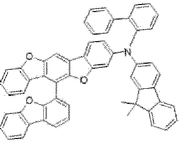 | 73% |
| 661 | 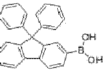 | 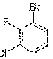 | 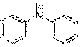 | 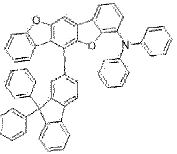 | 68% |
| 663 | 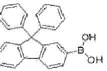 | 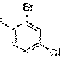 | 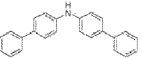 | 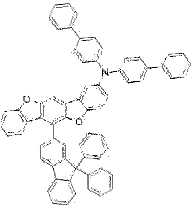 | 72% |
| 722 | 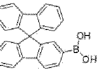 | 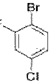 | 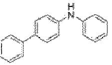 | 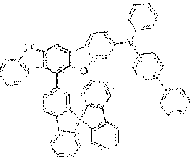 | 74% |
| 725 | 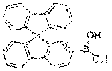 | 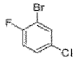 | 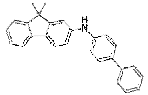 | 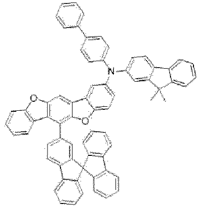 | 81% |

### <Preparation Example 2> Preparation of Compound 783

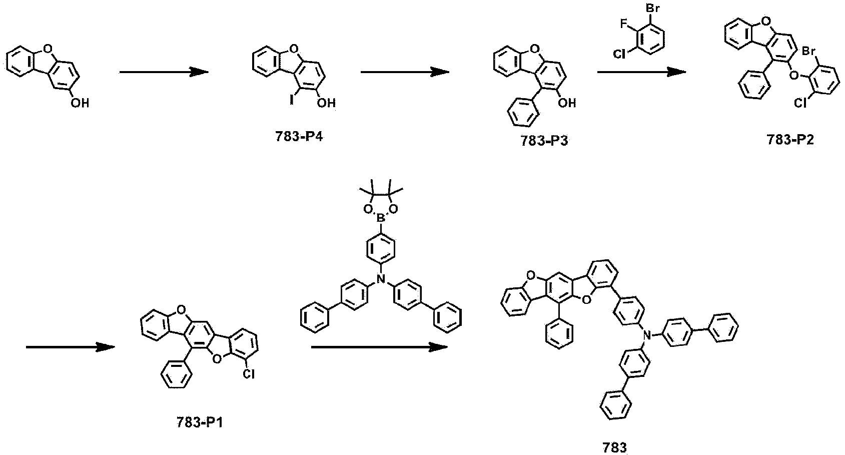

### 1) Preparation of Compound 783-P4

Dibenzo[b,d]furan-2-ol (150 g, 814.38 mmol) was introduced to acetic acid (600 ml), and the solution was stirred. Iodine monochloride (132.22 g, 814.38 mmol), HCl (195 ml) and acetic acid (345 ml) were mixed and then introduced to the solution prepared above, and the result was stirred for 12 hours at room temperature. After the reaction was completed, distilled water (3 L) was introduced thereto, and produced solids were filtered and then purified by column chromatography using dichloromethane and hexane as a developing solvent to obtain Compound 783-P4 (155 g, 61%).

### 2) Preparation of Compound 783-P3

After dissolving Compound 783-P4 (118 g, 380.53 mmol) and phenylboronic acid (51.04 g, 418.58 mmol) in THF (1000 ml) and distilled water (200 ml), Pd(PPh₃)₄ (13.19 g, 11.42 mmol) and K₂CO₃ (131.48 g, 951.33 mmol) were introduced thereto, and the result was stirred for 12 hours under reflux. After the reaction was completed, ethyl acetate was introduced to the reaction solution for dissolution, and the result was extracted with distilled water. The organic layer was dried with anhydrous MgSO₄, and after removing the solvent using a rotary evaporator, the result was purified by column chromatography using dichloromethane and hexane as a developing solvent to obtain Compound 783-P3 (90 g, 91%).

### 3) Preparation of Compound 783-P2

After dissolving Compound 783-P3 (90 g, 345.77 mmol) and 1-bromo-3-chloro-2-fluorobenzene (79.66 g, 418.58 mmol) in N,N-dimethylacetamide (1000 ml) and then heating the mixture to 150°C, Cs₂CO₃ (225.32 g, 691.54 mmol) was introduced thereto, and the result was stirred for 30 minutes under reflux. After the reaction was completed, the result was extracted with dichloromethane and distilled water. The organic layer was dried with anhydrous MgSO₄, and after removing the solvent using a rotary evaporator, the result was purified by column chromatography using dichloromethane and hexane as a developing solvent to obtain Compound 783-P2 (130 g, 83%).

### 4) Preparation of Compound 783-P1

After dissolving Compound 783-P2 (130 g, 289.07 mmol) in N,N-dimethylacetamide (1000 ml), Pd(PPh₃)₄ (10.02 g, 8.67 mmol), Na₂CO₃ (61.28 g, 578.14 mmol) and PPh₃ (7.58 g, 28.91 mmol) were introduced thereto, and the result was stirred for 12 hours under reflux. After the reaction was completed, the result was extracted with dichloromethane and distilled water. The organic layer was dried with anhydrous MgSO₄, and after removing the solvent using a rotary evaporator, the result was purified by column chromatography using dichloromethane and hexane as a developing solvent to obtain Compound 783-P1 (76 g, 71%).

### 5) Preparation of Compound 783

After dissolving Compound 783-P1 (10 g, 27.11 mmol) and N-([1,1'-biphenyl]-4-yl)-N-(4-(4,4,5,5-tetramethyl-1,3,2-dioxaborolan-2-yl)phenyl)-[1,1'-biphenyl]-4-amine (14.90 g, 28.47 mmol) in 1,4-dioxane (100 ml) and distilled water (20 ml), Pd(dba)₂ (0.78 g, 1.36 mmol), K₂CO₃ (9.37 g, 67.79 mmol) and xPhos (1.29 g, 2.71 mmol) were introduced thereto, and the result was stirred for 5 hours under reflux. After the reaction was completed, MC was introduced to the reaction solution for dissolution, and the result was extracted with distilled water. The organic layer was dried with anhydrous MgSO₄, and after removing the solvent using a rotary evaporator, the result was purified by column chromatography using dichloromethane and hexane as a developing solvent to obtain Compound 783 (14 g, 69%).

Target compounds of the following Table 2 were synthesized in the same manner as in Preparation Example 2 except that Compound D of the following Table 2 was used instead of phenylboronic acid, Compound E of the following Table 2 was used instead of 1-bromo-3-chloro-2-fluorobenzene, and Compound F of the following Table 2 was used instead of N-([1,1'-biphenyl]-4-yl)-N-(4-(4,4,5,5-tetramethyl-1,3,2-dioxaborolan-2-yl)phenyl)-[1,1'-biphenyl]-4-amine.

| Compoun d No. | Compound D | Compound E | Compound F | Target Compound | Yield |
|---|---|---|---|---|---|
| 785 | 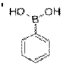 | 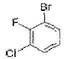 | 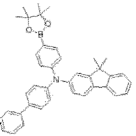 | 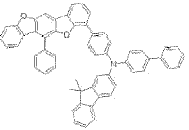 | 71% |
| 792 | 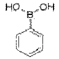 | 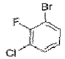 | 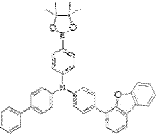 | 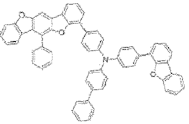 | 68% |
| 813 | 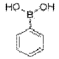 | 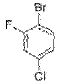 | 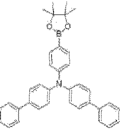 | 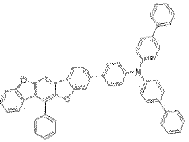 | 76% |
| 815 | 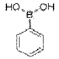 | 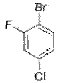 | 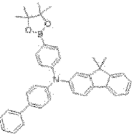 | 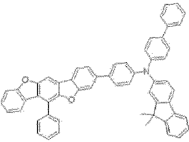 | 78% |
| 827 | 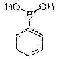 | 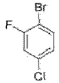 | 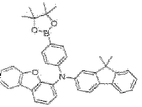 | 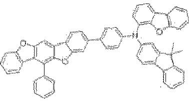 | 76% |
| 843 | 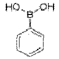 | 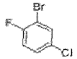 | 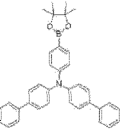 | 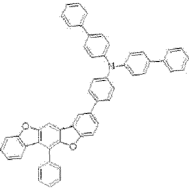 | 81% |
| 845 | 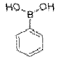 | 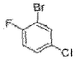 | 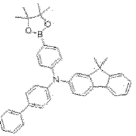 | 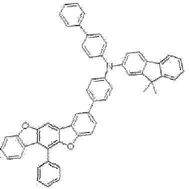 | 82% |
| 873 | 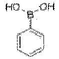 | 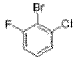 | 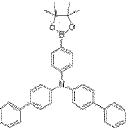 | 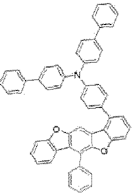 | 78% |
| 875 | 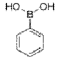 | 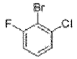 | 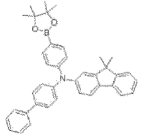 | 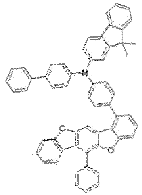 | 77% |
| 901 | 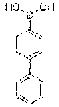 | 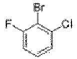 | 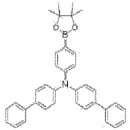 | 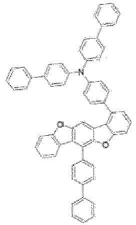 | 79% |
| 904 | 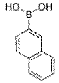 | 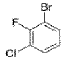 | 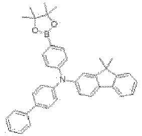 | 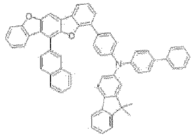 | 73% |

### <Preparation Example 3> Preparation of Compound 962

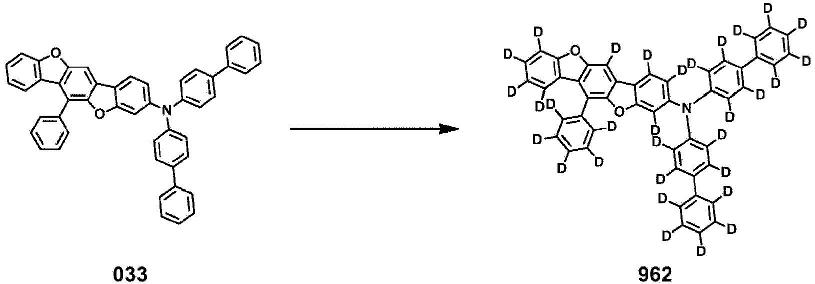

Compound 033 was introduced to C₆D₆ (50 ml), and the flask was purged with nitrogen for 30 minutes. An ethylaluminum dichloride solution (1.0 M in hexane) (1.5 ml) was added dropwise thereto via a syringe, and the reaction mixture was heated for 1 hour under reflux. The reaction mixture was cooled to room temperature, and then extracted by adding heavy water (50 ml) thereto. The organic layer was dried with anhydrous MgSO₄, and concentrated using a rotary evaporator. To obtained solids, the above-described reaction condition was further applied twice. After the last treatment, the result was recrystallized with toluene to obtain Compound 962 (3.4 g, 65%) . It was identified through a mass spectrum that an average of 17 deuterium atoms were mixed.

Compounds other than the compounds of the preparation examples were prepared in the same manner as in the preparation examples described above, and the synthesis identification results are shown in Table 3 and Table 4. Table 3 shows measurement values of ¹H NMR (CDCl₃, 300 MHz), and Table 4 shows measurement values of FD-mass spectrometry (FD-MS: field desorption mass spectrometry).

**[Table 3]**

| **Compoun d** | ¹H NMR (CDCl₃, 300 Mz) |
|---|---|
| **033** | δ=7.89 (1H, d), 7.66-7.64 (2H, m), 7.54-7.32 (23H, m), 6.69 (4H, d), 6.33 (1H, d) |
| **035** | δ=7.89 (1H, d), 7.66-7.62 (3H, m), 7.52-7.28 (19H, m), 6.75 (1H, s), 6.69 (2H, d), 6.58 (1H, d), 6.33 (1H, d), 1.72 (6H, s) |
| **060** | δ=7.89-7.87 (3H, m), 7.75 (1H, d), 7.66-7.64 (2H, m), 7.52-7.16 (25H, m), 6.89-6.88 (2H, m), 6.59-6.55 (2H, m), 6.39-6.33 (2H, m) |
| **077** | δ=7.89-7.87 (3H, m), 7.66-7.62 (4H, m), 7.55-7.51 (5H, m), 7.41-7.25 (10H, m), 7.07 (1H, t), 6.75 (1H, s), 6.58 (1H, d), 6.39 (2H, d), 1.72 (6H, s) |
| **096** | δ=7.89-7.87 (2H, m), 7.66-7.28 (17H, m), 7.16-7.02 (5H, m), 6.87 (1H, t), 6.75 (1H, s), 6.69 (1H, d), 6.58 (1H, d), 6.33 (1H, d), 1.72 (6H, s) |
| **119** | δ=7.89-7.87 (3H, m), 7.75 (1H, d), 7.66 (1H, d), 7.54-7.08 (27H, m), 6.87 (1H, t), 6.69 (1H, d), 6.55 (1H, s), 6.39-6.33 (2H, m) |
| **124** | δ=7.89 (1H, d), 7.66 (1H, d), 7.54-7.25 (24H, m), 7.16-7.07 (4H, m), 6.87 (1H, t), 6.69 (3H, d), 6.39 (1H, d) |
| **137** | δ=7.89-7.87 (3H, m), 7.66-7.62 (3H, m), 7.55-7.51 (5H, m), 7.41-7.25 (14H, m), 7.07 (2H, t), 6.75 (1H, s), 6.58 (1H, d), 6.39 (2H, d), 1.72 (6H, s) |
| **162** | δ=7.89-7.81 (4H, m), 7.66-7.64 (3H, m), 7.54-7.25 (25H, m), 6.69 (4H, d), 6.33 (1H, d) |
| **173** | δ=8.07-8.02 (2H, m), 7.89-7.87 (2H, m), 7.64-7.25 (23H, m), 6.98 (2H, d), 6.75 (1H, d), 6.58 (1H, d), 6.33 (1H, d) |
| **182** | δ=7.89 (1H, d), 7.66-7.65 (2H, m), 7.54-7.20 (22H, m), 6.81 (1H, t), 6.69-6.63 (4H, m), 6.39 (1H, d) |
| **199** | δ=7.89-7.81 (5H, m), 7.66-7.25 (25H, m), 6.69-6.65 (3H, m), 6.58 (1H, d), 6.39 (1H, d), 1.72 (6H, s) |
| **221** | δ=7.89 (2H, d), 7.66 (2H, d), 7.52-7.25 (21H, m), 7.13-7.02 (3H, m), 6.89-6.88 (2H, m), 6.59 (1H, d), 6.39-6.33 (2H, m) |
| **236** | δ=7.89-7.87 (2H, m), 7.66-7.02 (36H, m), 6.87 (1H, t), 6.75 (1H, s), 6.69 (1H, d), 6.58 (1H, d), 6.33 (1H, d) |
| **249** | δ=8.00-7.89 (5H, m), 7.73-7.25 (20H, m), 7.07 (2H, t), 6.69 (2H, d), 6.39 (2H, d) |
| **258** | δ=8.45 (1H, d), 8.00-7.81 (7H, m), 7.73-7.50 (9H, m), 7.38-7.25 (7H, m), 7.07 (1H, t), 6.86 (1H, d), 6.75 (1H, s), 6.58 (1H, d), 6.39 (1H, d) |
| **272** | δ=8.00-7.89 (4H, m), 7.73-7.32 (17H, m), 7.20 (2H, t), 6.81 (1H, t), 6.69-6.63 (4H, m), 6.33 (1H, d) |
| **295** | δ=8.00-7.87 (5H, m), 7.73-7.26 (27H, m), 7.11 (4H, d), 6.75 (1H, s), 6.69 (2H, d), 6.58 (1H, d), 6.33 (1H, d) |
| **311** | δ=8.00-7.89 (5H, m), 7.73-7.25 (20H, m), 7.07 (1H, t), 6.89 (1H, s), 6.88 (1H, d), 6.59 (1H, d), 6.39 (2H, d) |
| **321** | δ=8.07-7.89 (6H, m), 7.73-7.32 (21H, m), 6.98 (1H, d), 6.69 (2H, d), 6.39 (1H, d) |
| **337** | δ=8.00 (2H, d), 7.92-7.87 (3H m), 7.73-7.32 (18H, m), 7.13 (1H, t), 7.02 (1H, d), 6.89 (1H, s), 6.88 (1H, d), 6.75 (1H, s), 6.59-6.58 (2H, m), 6.33 (1H, d), 1.72 (6H, s) |
| **354** | δ=8.00-7.49 (18H, m), 7.38-7.28 (6H, m), 7.13 (1H, t), 7.02 (1H, d), 6.75 (1H, s), 6.58 (1H, d), 6.33 (1H, d), 1.72 (6H, s) |
| **363** | δ=7.93-7.87 (3H, m), 7.77 (1H, s), 7.66-7.63 (2H, m), 7.55-7.25 (21H, m), 7.07 (1H, t), 6.69 (4H, d), 6.39 (1H, d), 1.72 (6H, s) |
| **374** | δ=8.45 (1H, d), 7.98-7.87 (6H, m), 7.66-7.63 (2H, m), 7.55-7.25 (14H, m), 7.16-7.06 (3H, m), 6.87-6.86 (2H, m), 6.69 (1H, d), 6.39 (1H, d), 1.72 (6H, s) |
| **392** | δ=7.93 (2H, d), 7.87 (1H, d), 7.77 (1H, s), 7.66-7.63 (3H, m), 7.55-7.20 (16H, m), 6.81 (1H, t), 6.69 (2H, d), 6.63 (2H, d), 6.33 (1H, d), 1.72 (6H, s) |
| **395** | δ=7.93 (2H, d), 7.87 (2H, d), 7.77 (1H, s), 7.66-7.62 (4H, m), 7.55-7.51 (8H, m), 7.43-7.28 (9H, m), 6.75 (1H, s), 6.69 (2H, d), 6.58 (1H, d), 6.33 (1H, d) 1.72 (12H, s) |
| **403** | δ=8.45 (1H, d), 7.98-7.77 (6H, m), 7.66-7.63 (3H, m), 7.55-7.27 (17H, m), 6.86 (1H, d), 6.69 (2H, d), 6.33 (1H, d), 1.72 (6H, s) |
| **411** | δ=8.07 (1H, d), 8.02 (1H, d), 7.93-7.87 (3H, m), 7.77 (1H, s), 7.66-7.32 (21H, m), 6.98 (1H, d), 6.69 (2H, d), 6.33 (1H, d), 1.72 (6H, s) |
| **453** | δ=7.93 (1H, d), 7.89 (1H, d), 7.87 (1H, d), 7.77 (1H, s), 7.66-7.63 (2H, m), 7.55-7.28 (20H, m), 7.13 (1H, t), 7.02 (1H, d), 6.69 (4H, d), 6.33 (1H, d), 1.72 (6H, s) |
| **455** | δ=7.98 (1H, d), 7.89 (1H, d), 7.87 (1H, d), 7.77 (1H, s), 7.66-7.62 (3H, m), 7.55-7.51 (8H, m), 7.41-7.28 (8H, m), 7.13 (1H, t), 7.02 (1H, d), 6.75 (1H, s), 6.69 (2H, d), 6.58 (1H, d), 6.33 (1H, d), 1.72 (12H, s) |
| **458** | δ=7.93-7.87 (5H, m), 7.77 (1H, s), 7.66-7.62 (4H, m), 7.55 (2H, d), 7.38-7.28 (9H, m), 7.13 (1H, t), 7.02 (1H, d), 6.75 (2H, s), 6.58 (2H, d), 6.33 (1H, d), 1.72 (18H, s) |
| **467** | δ=7.93-7.87 (5H, m), 7.77 (1H, s), 7.66-7.62 (4H, m), 7.55 (2H, d), 7.38-7.25 (10H, m), 7.13-7.02 (3H, m), 6.75 (1H, s), 6.58 (1H, d), 6.39-6.33 (2H, m), 1.72 (12H, s) |
| **532** | δ=7.89-7.84 (3H, m), 7.77-7.66 (4H, m), 7.57-7.25 (22H, m), 7.07 (1H, t), 6.69 (2H, d), 6.39 (1H, d) |
| **546** | δ=8.55 (1H, d), 8.42 (1H, d), 8.08-8.04 (2H, m), 7.87 (1H, d), 7.66-7.51 (10H, m), 7.43-7.28 (7H, m), 716-7.08 (3H, m), 6.87 (1H, t), 6.75 (1H, s), 6.69 (1H, d), 6.58 (1H, d), 6.33 (1H, d), 1.72 (6H, s) |
| **547** | δ=8.55 (1H, d), 8.42 (1H, d), 8.08-8.04 (2H, m), 7.89-7.87 (2H, m), 7.66-7.28 (19H, m), 6.89-6.88 (2H, m), 6.75 (1H, s), 6.59-6.58 (2H, m), 6.33 (1H, d), 1.72 (6H, s) |
| **557** | δ=8.55 (1H, d), 8.42 (1H, d), 8.08-8.04 (2H, m) 7.89-7.87 (3H, m), 7.66-7.55 (7H, m), 7.38-7.28 (8H, m), 7.13-7.02 (3H, m), 6.75 (1H, s), 6.58 (1H, d), 6.39 (1H, d), 6.33 (1H, d), 1.72 (6H, s) |
| **580** | δ=8.45-8.41 (2H, m), 8.20 (1H, d), 7.98 (1H, d), 7.89 (2H, d), 7.66-7.25 (17H, m), 7.16-7.07 (4H, m), 6.87 (1H, t), 6.69 (1H, d), 6.39 (1H, d), 6.33 (1H, d) |
| **603** | δ=7.89-7.81 (4H, m), 7.66 (2H, d), 7.54-7.32 (20H, m), 7.13 (1H, t), 7.02 (1H, d), 6.69 (4H, d), 6.33 (1H, d) |
| **606** | δ=7.89-7.81 (5H, m), 7.66-7.51 (8H, m), 7.43-7.28 (10H, m), 7.16 (1H, t), 7.08 (2H, d), 6.87 (1H, t), 6.75 (1H, s), 6.69 (1H, d), 6.58 (1H, d), 6.33 (1H, d), 1.72 (6H, s) |
| **661** | δ=7.93-7.87 (3H, m), 7.77 (1H, s), 7.66-7.63 (2H, m), 7.55 (1H, d), 7.38-7.07 (21H, m), 6.81 (2H, t), 6.63 (4H, d), 6.39 (1H, d) |
| **663** | δ=7.93-7.87 (3H, m), 7.77 (1H, s), 7.66-7.63 (3H, m), 7.54-7.26 (27H, m), 7.11 (4H, d), 6.69 (4H, d), 6.39 (1H, d) |
| **722** | δ=7.93-7.87 (3H, m), 7.77-7.75 (3H, m), 7.66-7.63 (3H, m), 7.54-7.20 (22H, m), 6.81 (1H, t), 6.69 (2H, d), 6.63 (2H, d), 6.33 (1H, d) |
| **725** | δ=7.93-7.87 (4H, m), 7.77-7.75 (3H, m), 7.66-7.16 (27H, m), 6.75 (1H, s), 6.69 (2H, d), 6.58 (1H, d), 6.39 (1H, d), 1.72 (6H, s) |
| **783** | δ=7.89-7.81 (3H, m), 7.66 (1H, d), 7.54-7.32 (25H, m), 6.69 (6H, d) |
| **785** | δ=7.89-7.81 (4H, m), 7.66-7.62 (2H, m), 7.55-7.28 (21H, m), 6.75 (1H, s), 6.69 (4H, d), 6.58 (1H, d), 1.72 (6H, s) |
| **792** | δ=7.89-7.81 (6H, m), 7.66 (2H, d), 7.54-7.32 (23H, m), 6.69 (6H, d) |
| **813** | δ=7.95 (1H, d), 7.89 (1H, d), 7.75 (1H, d), 7.66 (1H, d), 7.64 (1H, s), 7.54-7.32 (24H, m), 6.69 (6H, d) |
| **815** | δ=7.95 (1H, d), 7.89 (1H, d), 7.87 (1H, d), 7.75 (1H, d), 7.66-7.62 (3H, m), 7.55-7.28 (20H, m), 6.75 (1H, s), 6.69 (4H, d), 6.58 (1H, d), 1.72 (6H, s) |
| **827** | δ=7.95-7.87 (4H, m), 7.75 (1H, d), 7.66-7.25 (20H, m), 7.07 (1H, t), 6.75 (1H, s), 6.69 (2H, d), 6.58 (1H, d), 6.39 (1H, d), 1.72 (6H, s) |
| **843** | δ=7.89 (1H, d), 7.81 (1H, d), 7.72 (1H, d), 7.71 (1H, s), 7.66 (1H, d), 7.54-7.32 (24H, m), 6.69 (6H, d) |
| **845** | δ=7.89 (1H, d), 7.87 (1H, d), 7.81 (1H, d), 7.72-7.62 (4H, m), 7.55-7.28 (20H, m), 6.75 (1H, s), 6.69 (4H, d), 6.58 (1H, d), 1.72 (6H, s) |
| **873** | δ=7.89 (1H, d), 7.75 (1H, d), 7.66-7.62 (2H, m), 7.54-7.32 (25H, m), 6.69 (6H, d) |
| **875** | δ=7.89 (1H, d), 7.87 (1H, d), 7.75 (1H, d), 7.62-7.28 (24H, m), 6.75 (1H, s), 6.69 (4H, d), 6.58 (1H, d), 1.72 (6H, s) |
| **901** | δ=7.89 (1H, d), 7.75 (1H, d), 7.66 (1H, d), 7.62 (1H, d), 7.54-7.32 (25H, m), 7.25 (4H, s), 6.69 (6H, d) |
| **904** | δ=8.00-7.81 (7H, m), 7.73-7.28 (22H, m), 6.75 (1H, s), 6.69 (4H, d), 6.58 (1H, d), 1.72 (6H, s) |

**[Table 4]**

| **Compoun d** | **FD-MS** | **Compo und** | **FD-MS** |
|---|---|---|---|
| **033** | m/z=653.77 (C₄₈H₃₁NO₂=653.24) | **035** | m/z=693.83 (C₅₁H₃₅NO₂=693.27) |
| **060** | m/z=815.95 (C₆₁H₃₇NO₂=815.28) | **077** | m/z=707.81 (C₅₁H₃₃NO₃=707.25) |
| **096** | m/z=693.83 (C₅₁H₃₅NO₂=693.27) | **119** | m/z=815.95 (C₆₁H₃₇NO₂=815.28) |
| **124** | m/z=729.86 (C₅₄H₃₅NO₂=729.27) | **137** | m/z=783.91 (C₅₇H₃₇NO₃=783.28) |
| **162** | m/z=819.94 (C₆₀H₃₇NO₃=819.28) | **173** | m/z=743.89 (C₅₅H₃₇NO₂=743.28) |
| **182** | m/z=653.77 (C₄₈H₃₁NO₂=653.24) | **199** | m/z=860.00 (C₆₃H₄₁NO₃=859.31) |
| **221** | m/z=743.84 (C₅₄H₃₃NO₃=743.25) | **236** | m/z=894.06 (C₆₇H₄₃NO₂=893.33) |
| **249** | m/z=717.81 (C₅₂H₃₁NO₃=717.23) | **258** | m/z=773.94 (C₅₅H₃₅NO₂S=773.24) |
| **272** | m/z=627.73 (C₄₆H₂₉NO₂=627.22) | **295** | m/z=868.03 (C₆₅H₄₁NO₂=867.31) |
| **311** | m/z=717.81 (C₅₂H₃₁NO₃=717.23) | **321** | m/z=677.79 (C₅₀H₃₁NO₂=677.24) |
| **337** | m/z=743.89 (C₅₅H₃₇NO₂=743.28) | **354** | m/z=717.85 (C₅₃H₃₅NO₂=717.27) |
| **363** | m/z=769.96 (C₅₇H₃₉NO₂=769.30) | **374** | m/z=799.97 (C₅₇H₃₇NO₂S=799.25) |
| **392** | m/z=693.83 (C₅₁H₃₅NO₂=693.27) | **395** | m/z=809.99 (C₆₀H₄₃NO₂=809.33) |
| **403** | m/z=799.97 (C₅₇H₃₇NO₂S=799.25) | **411** | m/z=743.89 (C₅₅H₃₇NO₂=743.28) |
| **453** | m/z=769.93 (C₅₇H₃₉NO₂=769.30) | **455** | m/z=809.99 (C₆₀H₄₃NO₂=809.33) |
| **458** | m/z=850.05 (C₆₃H₄₇NO₂=849.36) | **467** | m/z=823.97 (C₆₀H₄₁NO₃=823.31) |
| **532** | m/z=703.82 (C₅₂H₃₃NO₂=703.25) | **546** | m/z=743.89 (C₅₅H₃₇NO₂=743.28) |
| **547** | m/z=743.89 (C₅₅H₃₇NO₂=743.28) | **557** | m/z=757.87 (C₅₅H₃₅NO₃=757.26) |
| **580** | m/z=773.89 (C₅₄H₃₁NO₃S=773.20) | **603** | m/z=743.84 (C₅₄H₃₃NO₃=743.25) |
| **606** | m/z=783.91 (C₅₇H₃₇NO₃=783.28) | **661** | m/z=741.87 (C₅₅H₃₅NO₂=741.27) |
| **663** | m/z=894.06 (C₆₇H₄₃NO₂=893.33) | **722** | m/z=815.95 (C₆₁H₃₇NO₂=815.28) |
| **725** | m/z=932.11 (C₇₀H₄₅NO₂=931.35) | **783** | m/z=729.86 (C₅₄H₃₅NO₂=729.27) |
| **785** | m/z=769.93 (C₅₇H₃₉NO₂=769.30) | **792** | m/z=819.94 (C₆₀H₃₇NO₃=819.28) |
| **813** | m/z=729.86 (C₅₄H₃₅NO₂=729.27) | **815** | m/z=769.93 (C₅₇H₃₉NO₂=769.30) |
| **827** | m/z=783.91 (C₅₇H₃₇NO₃=783.28) | **843** | m/z=729.86 (C₅₄H₃₅NO₂=729.27) |
| **845** | m/z=769.93 (C₅₇H₃₉NO₂=769.30) | **873** | m/z=729.86 (C₅₄H₃₅NO₂=729.27) |
| **875** | m/z=769.93 (C₅₇H₃₉NO₂=769.30) | **901** | m/z=805.96 (C₆₀H₃₉NO₂=805.30) |
| **904** | m/z=819.98 (C₆₁H₄₁NO₂=819.31) | | |

### <Experimental Example 1>

### -Manufacture of Organic Light Emitting Device

### (1) Manufacture of Organic Light Emitting Device

### (Comparative Example 1)

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

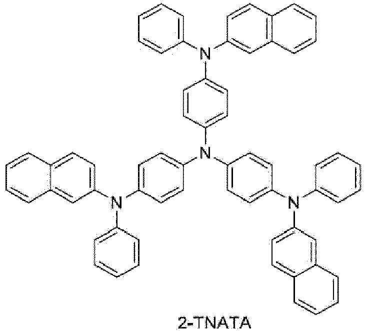

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

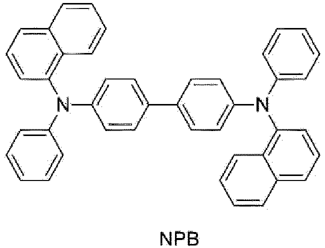

A light emitting layer was thermal vacuum deposited thereon as follows. As the light emitting layer, a compound of 9-[4-(4,6-diphenyl-1,3,5-triazin-2-yl)phenyl]-9'-phenyl-3,3'-bi-9H-carbazole was deposited to 400 Å as a host, and, as a green phosphorescent dopant, Ir(ppy)₃ was doped by 7% and deposited. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

### (Comparative Examples 2 to 4 and Examples 1 to 23)

Organic electroluminescent devices were manufactured in the same manner as in Comparative Example 1 except that compounds shown in the following Table 5 were used instead of the compound NPB used when forming the hole transfer layer.

Herein, the hole transfer compounds of the comparative examples other than NPB are as follows.

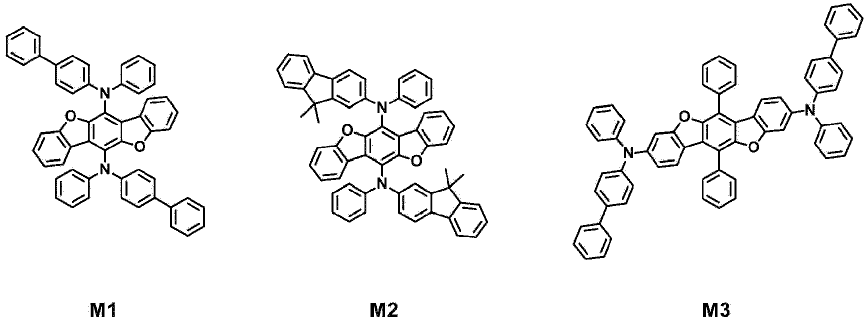

### (2) Driving Voltage and Light Emission Efficiency of Organic Light Emitting Device

For each of the organic electroluminescent devices manufactured as above, electroluminescent (EL) properties were measured using M7000 manufactured by McScience Inc., and with the measurement results, T₉₅ was measured when standard luminance was 6,000 cd/m² through a lifetime measurement system (M6000) manufactured by McScience Inc..

Properties of the organic electroluminescent devices of the present disclosure are as shown in the following Table 5.

**[Table 5]**

| | Compound | Driving Voltage (V) | Light Emission Efficiency (cd/A) | Lifetime (T₉₅) |
|---|---|---|---|---|
| Example 1 | 033 | 4.11 | 119.59 | 167 |
| Example 2 | 035 | 4.21 | 117.88 | 159 |
| Example 3 | 060 | 3.98 | 121.32 | 173 |
| Example 4 | 162 | 4.09 | 120.51 | 154 |
| Example 5 | 182 | 3.77 | 124.32 | 214 |
| Example 6 | 295 | 4.19 | 120.33 | 177 |
| Example 7 | 321 | 4.30 | 118.99 | 168 |
| Example 8 | 337 | 4.13 | 116.88 | 192 |
| Example 9 | 354 | 3.98 | 120.61 | 168 |
| Example 10 | 363 | 3.79 | 122.23 | 195 |
| Example 11 | 392 | 4.27 | 118.96 | 172 |
| Example 12 | 395 | 4.04 | 128.76 | 207 |
| Example 13 | 453 | 3.99 | 121.02 | 168 |
| Example 14 | 532 | 3.91 | 120.11 | 165 |
| Example 15 | 663 | 3.92 | 128.17 | 210 |
| Example 16 | 722 | 4.08 | 112.85 | 180 |
| Example 17 | 725 | 4.19 | 128.67 | 152 |
| Example 18 | 792 | 4.09 | 123.76 | 162 |
| Example 19 | 813 | 3.97 | 124.11 | 215 |
| Example 20 | 827 | 4.13 | 116.08 | 198 |
| Example 21 | 901 | 4.15 | 117.79 | 184 |
| Example 22 | 904 | 4.04 | 119.57 | 182 |
| Example 23 | 962 | 4.08 | 119.42 | 311 |
| Comparative Example 1 | NPB | 4.53 | 112.24 | 158 |
| Comparative Example 2 | M1 | 4.38 | 114.11 | 150 |
| Comparative Example 3 | M2 | 4.45 | 116.58 | 172 |
| Comparative Example 4 | M3 | 4.33 | 120.02 | 142 |

It was seen that the devices of Examples 1 to 23 according to one embodiment of the present disclosure had low driving voltage and superior efficiency and lifetime compared to the devices of Comparative Examples 1 to 4.

### <Experimental Example 2>

### -Manufacture of Organic Light Emitting Device

### (Comparative Example 5)

A transparent ITO electrode thin film obtained from glass for an OLED (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenylamine (2-TNATA) was introduced to a cell in the vacuum deposition apparatus.

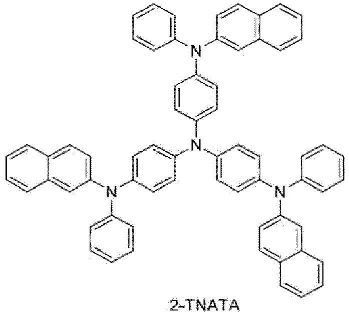

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 150 Å on the hole injection layer, and then an electron blocking layer was formed to a thickness of 50 Å by depositing Compound M1 on the hole transfer layer.

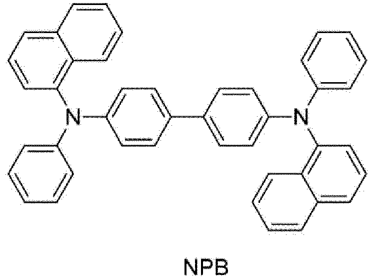

After forming the hole injection layer, the hole transfer layer and the electron blocking layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

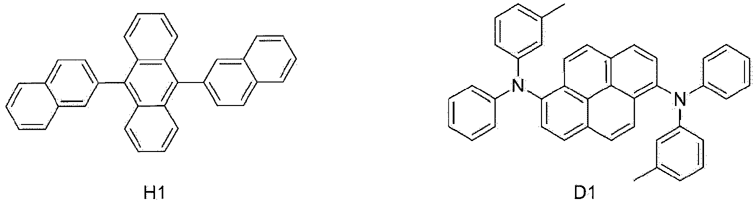

Subsequently, a compound of the following Structural Formula E1 was deposited to a thickness of 300 Å as an electron transfer layer.

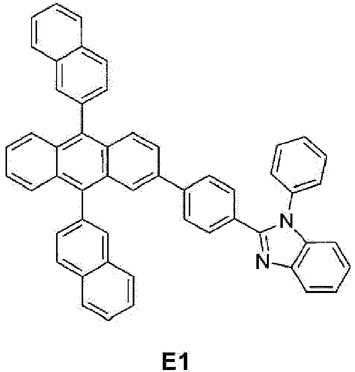

As an electron injection layer, lithium fluoride (LiF) was deposited to a thickness of 10 Å, and an Al cathode was employed to a thickness of 1,000 Å, and as a result, an OLED was manufactured.

Meanwhile, all the organic compounds required to manufacture the OLED were vacuum sublimation purified under 10⁻⁸ torr to 10⁻⁶ torr by each material to be used in the OLED manufacture.

### (Comparative Examples 6 to 8 and Examples 24 to 63)

Organic electroluminescent devices were manufactured in the same manner as in Comparative Example 5 except that compounds shown in the following Table 6 were used instead of Compound M1. Results of measuring driving voltage, light emission efficiency and lifetime of the blue organic light emitting devices manufactured according to the present disclosure are as shown in the following Table 6.

Herein, the electron blocking layer compounds of the comparative examples are as follows.

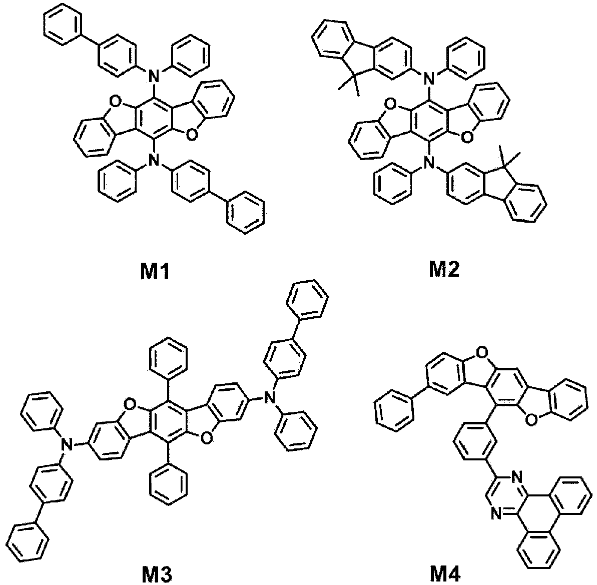

**[Table 6]**

| | Compound | Driving Voltage (V) | Light Emission Efficiency (cd/A) | Lifetime (T₉₅) |
|---|---|---|---|---|
| Example 24 | 035 | 5.11 | 7.22 | 57 |
| Example 25 | 077 | 5.38 | 6.69 | 47 |
| Example 26 | 096 | 5.64 | 6.89 | 49 |
| Example 27 | 119 | 5.68 | 6.83 | 52 |
| Example 28 | 124 | 5.36 | 6.75 | 53 |
| Example 29 | 137 | 5.35 | 6.81 | 47 |
| Example 30 | 162 | 5.24 | 7.27 | 61 |
| Example 31 | 173 | 5.33 | 6.98 | 49 |
| Example 32 | 199 | 5.72 | 6.78 | 51 |
| Example 33 | 221 | 5.47 | 6.92 | 45 |
| Example 34 | 249 | 5.55 | 6.88 | 51 |
| Example 35 | 258 | 5.68 | 6.97 | 48 |
| Example 36 | 272 | 5.63 | 6.99 | 51 |
| Example 37 | 311 | 5.61 | 6.94 | 53 |
| Example 38 | 354 | 5.59 | 7.01 | 50 |
| Example 39 | 363 | 5.23 | 7.29 | 59 |
| Example 40 | 374 | 5.85 | 6.97 | 48 |
| Example 41 | 392 | 5.31 | 6.80 | 51 |
| Example 42 | 395 | 5.03 | 7.05 | 63 |
| Example 43 | 403 | 5.48 | 6.90 | 52 |
| Example 44 | 411 | 5.44 | 6.82 | 51 |
| Example 45 | 453 | 5.29 | 7.03 | 63 |
| Example 46 | 455 | 5.13 | 7.12 | 60 |
| Example 47 | 458 | 5.67 | 6.87 | 52 |
| Example 48 | 467 | 5.59 | 6.90 | 49 |
| Example 49 | 532 | 5.55 | 6.91 | 50 |
| Example 50 | 546 | 5.41 | 6.80 | 50 |
| Example 51 | 547 | 5.43 | 6.95 | 49 |
| Example 52 | 580 | 5.52 | 6.93 | 46 |
| Example 53 | 603 | 5.57 | 6.98 | 47 |
| Example 54 | 606 | 5.41 | 6.91 | 46 |
| Example 55 | 661 | 5.53 | 6.77 | 45 |
| Example 56 | 663 | 5.57 | 6.81 | 51 |
| Example 57 | 722 | 5.58 | 6.92 | 47 |
| Example 58 | 725 | 5.65 | 6.96 | 52 |
| Example 59 | 783 | 5.19 | 6.90 | 49 |
| Example 60 | 785 | 5.42 | 6.74 | 43 |
| Example 61 | 815 | 5.21 | 7.19 | 59 |
| Example 62 | 873 | 5.39 | 7.15 | 64 |
| Example 63 | 875 | 5.66 | 6.99 | 52 |
| Comparative Example 5 | M1 | 5.74 | 6.30 | 43 |
| Comparative Example 6 | M2 | 5.68 | 6.66 | 42 |
| Comparative Example 7 | M3 | 5.49 | 6.41 | 44 |
| Comparative Example 8 | M4 | 6.89 | 4.88 | 32 |

It was seen that the devices of Examples 24 to 63 according to one embodiment of the present disclosure had low driving voltage and superior efficiency and lifetime compared to the devices of Comparative Examples 5 to 8.

Particularly, M3 used in Comparative Example 7 has a symmetrical structure and thereby has crystalline properties, and a material having such properties has a reduced charge transfer ability by having an uneven deposited film when deposited. Such a problem causes an increase in the driving voltage and a decrease in the efficiency.

In addition, it was identified that M4 used in Comparative Example 8 had an increased driving voltage and decreased efficiency since holes were not readily delivered by having a deeper HOMO energy level.

### <Experimental Example 3>

### 1) Manufacture of Organic Light Emitting Device

### (Examples 64 to 88 and Comparative Examples 9 to 14)

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. The light emitting layer was deposited to a thickness of 500 Å by using, as a host, a compound described in the following Table 7 as a single host or depositing two host compounds in one source of supply using an n-host (n-type host) having a favorable electron transfer ability as a first host and using a p-host (p-type host) having a favorable hole transfer ability as a second host, and doping (piq)₂(Ir) (acac) to the host by 3% with respect to the host material weight as a red phosphorescent dopant or doping Ir(ppy)₃ to the host by 7% with respect to the host material weight as a green phosphorescent dopant.

After that, BCP was deposited to a thickness of 60 Å as a hole blocking layer, and Alq₃ was deposited to a thickness of 200 Å thereon as an electron transfer layer.

Herein, the compound used as the n-host when using two hosts is as follows.

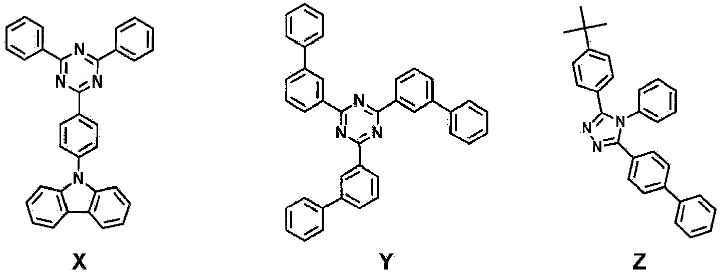

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

Specifically, the compounds used as the host in Examples 64 to 88 and Comparative Examples 9 to 14 are as shown in the following Table 7.

Herein, Compounds M1 to M3 used as the host of Comparative Examples 9 to 14 of the following Table 7 are as follows.

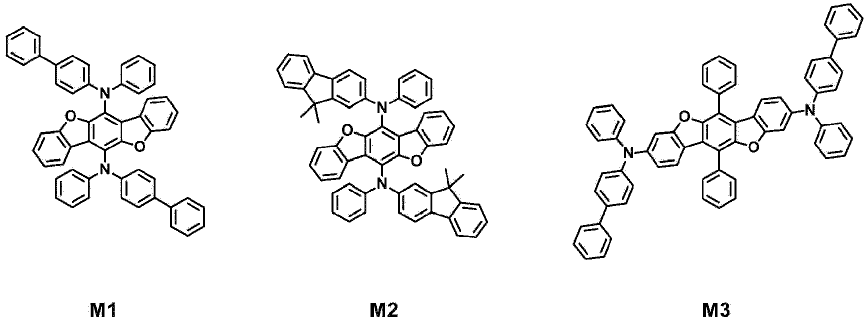

Meanwhile, all the organic compounds required to manufacture the OLED were vacuum sublimation purified under 10⁻⁸ torr to 10⁻⁶ torr for each material to be used in the organic light emitting device manufacture.

### 2) Driving Voltage and Light Emission Efficiency of Organic Light Emitting Device

For each of the organic light emitting devices manufactured as above, electroluminescent (EL) properties were measured using M7000 manufactured by McScience Inc., and with the measurement results, T₉₅ was measured when standard luminance was 6,000 cd/m² through a lifetime measurement system (M6000) manufactured by McScience Inc.. Results of measuring driving voltage, light emission efficiency, light emitting color and lifetime of the organic light emitting devices manufactured according to the present disclosure are as shown in the following Table 7.

**[Table 7]**

| | First Host | Second Host | Driving Voltage (V) | Efficien cy (cd/A) | Light Emitting Color | Lifetime (T₉₅) |
|---|---|---|---|---|---|---|
| Example 64 | 077 | | 3.73 | 18.1 | Red | 103 |
| Example 65 | | | 3.81 | 15.2 | Green | 105 |
| Example 66 | 392 | | 3.89 | 17.5 | Red | 98 |
| Example 67 | | | 4.02 | 14.1 | Green | 102 |
| Example 68 | 580 | | 3.76 | 19.3 | Red | 112 |
| Example 69 | | | 3.97 | 16.2 | Green | 117 |
| Example 70 | 603 | | 3.71 | 18.2 | Red | 94 |
| Example 71 | | | 3.79 | 17.3 | Green | 101 |
| Example 72 | 661 | | 3.84 | 19.1 | Red | 107 |
| Example 73 | | | 3.88 | 18.3 | Green | 113 |
| Example 74 | X | 077 | 3.70 | 24.6 | Red | 156 |
| Example 75 | | 392 | 3.63 | 23.5 | Red | 176 |
| Example 76 | | 580 | 3.55 | 23.1 | Red | 149 |
| Example 77 | | 603 | 3.57 | 23.3 | Red | 163 |
| Example 78 | | 661 | 3.71 | 21.8 | Red | 136 |
| Example 79 | Y | 077 | 3.77 | 22.9 | Green | 139 |
| Example 80 | | 392 | 3.70 | 23.4 | Green | 151 |
| Example 81 | | 580 | 3.63 | 23.5 | Green | 149 |
| Example 82 | | 603 | 3.69 | 21.1 | Green | 153 |
| Example 83 | | 661 | 3.69 | 22.4 | Green | 162 |
| Example 84 | Z | 077 | 3.65 | 24.3 | Red | 139 |
| Example 85 | | 392 | 3.61 | 25.2 | Red | 130 |
| Example 86 | | 580 | 3.58 | 23.8 | Red | 125 |
| Example 87 | | 603 | 3.57 | 24.9 | Red | 139 |
| Example 88 | | 661 | 3.57 | 23.0 | Red | 132 |
| Comparative Example 9 | M1 | | 4.43 | 7.5 | Red | 52 |
| Comparative Example 10 | X | M1 | 4.38 | 18.1 | Red | 110 |
| Comparative Example 11 | M2 | | 4.30 | 8.8 | Green | 65 |
| Comparative Example 12 | Y | M2 | 4.10 | 17.4 | Green | 135 |
| Comparative Example 13 | M3 | | 4.10 | 9.6 | Red | 23 |
| Comparative Example 14 | Z | M3 | 3.94 | 21.2 | Red | 109 |

From Table 7, it was identified that the organic light emitting devices of Examples 64 to 73 forming the light emitting layer using the compound according to the present application as a single host material had superior light emission efficiency and lifetime compared to the organic light emitting devices of Comparative Examples 9, 11 and 13 that did not use the compound according to the present application as a single host material when forming the light emitting layer using a single host material.

In addition, from Table 7, it was identified that the organic light emitting devices of Examples 74 to 88 forming the light emitting layer using both a first host material corresponding to the n-host and the compound according to the present application as a second host material corresponding to the p-host had superior light emission efficiency and lifetime compared to the organic light emitting devices of Comparative Examples 10, 12 and 14 forming the light emitting layer using both a first host material corresponding to the n-host and a compound that is not the compound according to the present application as a second host material corresponding to the p-host.

In addition, the organic light emitting devices of Examples 64 to 73 forming the light emitting layer using the compound according to the present application as a single host material had similar or sometimes superior light emission efficiency and lifetime compared to the organic light emitting devices of Comparative Examples 10, 12 and 14 forming the light emitting layer using both a first host material corresponding to the n-host and a compound that is not the compound according to the present application as a second host material corresponding to the p-host.

Considering that an organic light emitting device using an n-host (n-type host) having a favorable electron transfer ability as a first host and using a p-host (p-type host) having a favorable hole transfer ability as a second host generally has superior light emission efficiency and lifetime compared to an organic light emitting device using a single host material, this means that an organic light emitting device may have significantly improved light emission efficiency and lifetime when using the compound according to the present application as a host material.

This is considered to be due to the fact that, when using the compound according to the present application as a host material, holes and electrons are efficiently injected to a light emitting layer from each charge transfer layer. As described above, this is also considered to be due to the influence of orientation and space size formed by interactions between materials during deposition.

This is due to the fact that efficiently injecting holes and electrons to a light emitting layer is also affected by orientation and space size formed by interactions between materials during deposition, and as described above, this is considered to be an effect obtained by differences in the orientation properties and the space size between the compound of the present application and M1 to M3.

The present disclosure is not limited to the above-described examples and may be prepared in various different forms, and those skilled in the art may understand that the present disclosure may be embodied in other specific forms without changing technical ideas and essential features. Therefore, the examples described above are for illustrative purposes in all aspects and need to be construed as nonlimiting.

## Claims

1. A heterocyclic compound represented by the following Chemical Formula 1: 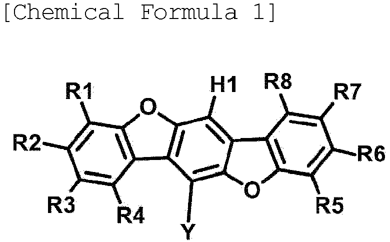 wherein, in Chemical Formula 1,
R1 to R8 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted phosphine oxide group; and a substituted or unsubstituted amine group, or two or more groups adjacent to each other bond to each other to form a substituted or unsubstituted C6 to C60 aliphatic or aromatic hydrocarbon ring; or a substituted or unsubstituted C2 to C60 heteroring;
H1 is hydrogen; or deuterium; and
Y is represented by any one of the following Chemical Formula 1-1 to Chemical Formula 1-4, 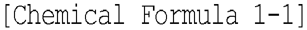 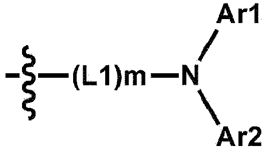 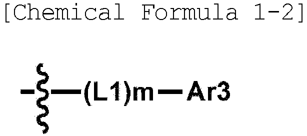 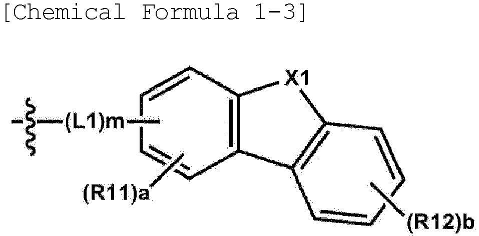 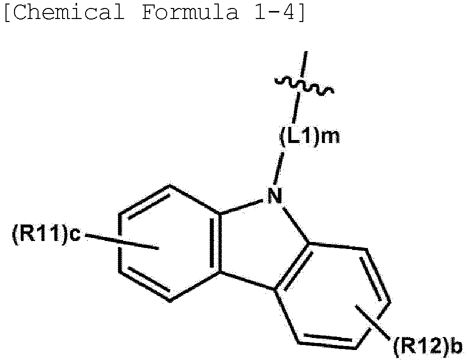 in Chemical Formulae 1-1 to 1-4,
X1 is O or S;
L1 is a direct bond; a substituted or unsubstituted C6 to C60 arylene group; or a substituted or unsubstituted C2 to C60 heteroarylene group;
R11 and R12 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted phosphine oxide group; and a substituted or unsubstituted amine group, or two or more groups adjacent to each other bond to each other to form a substituted or unsubstituted C6 to C60 aliphatic or aromatic hydrocarbon ring; or a substituted or unsubstituted C2 to C60 heteroring;
Ar1 and Ar2 are each independently a substituted or unsubstituted C1 to C60 alkyl group; a substituted or unsubstituted C6 to C60 aryl group; or a substituted or unsubstituted C2 to C60 heteroaryl group;
Ar3 is a substituted or unsubstituted C6 to C60 aryl group;
m is an integer of 0 to 5; and
a is an integer of 0 to 3, and b and c are each an integer of 0 to 4.

2. The heterocyclic compound of Claim 1, wherein the heterocyclic compound of Chemical Formula 1 is represented by the following Chemical Formula 2 or Chemical Formula 3: 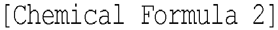 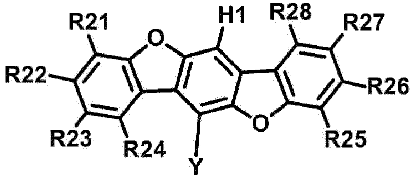 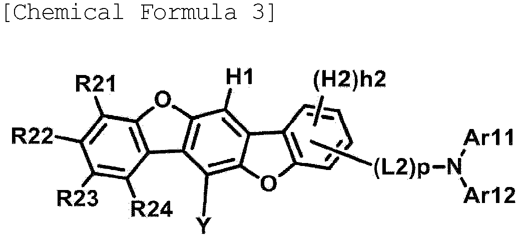 in Chemical Formula 2 and Chemical Formula 3,
Y and H1 have the same definitions as in Chemical Formula 1;
R21 to R28 are the same as or different from each other, and each independently hydrogen; deuterium; a substituted or unsubstituted C1 to C60 alkyl group; or a substituted or unsubstituted C6 to C60 aryl group;
L2 is a direct bond; a substituted or unsubstituted C6 to C60 arylene group; or a substituted or unsubstituted C2 to C60 heteroarylene group;
p is an integer of 0 to 5, and when 2 or greater, L2s are the same as or different from each other;
Ar11 and Ar12 are each independently a substituted or unsubstituted C1 to C60 alkyl group; a substituted or unsubstituted C6 to C60 aryl group; or a substituted or unsubstituted C2 to C60 heteroaryl group;
H2 is hydrogen; or deuterium; and
h2 is an integer of 0 to 3, and when 2 or greater, H2s are the same as or different from each other.

3. The heterocyclic compound of Claim 2, wherein Chemical Formula 3 is represented by any one of the following Chemical Formulae 3-1 to 3-4: 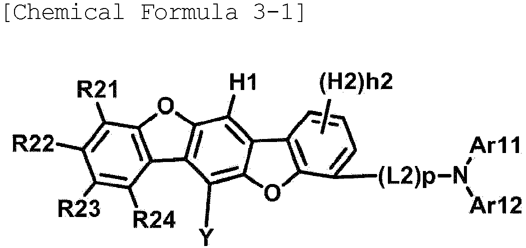 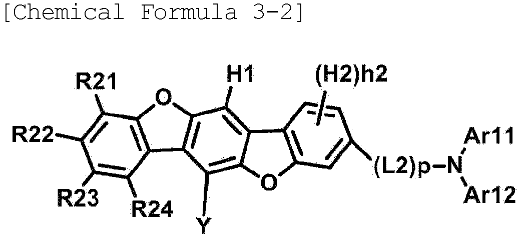 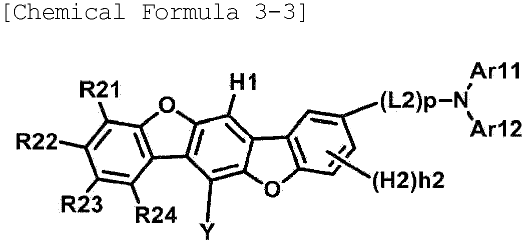 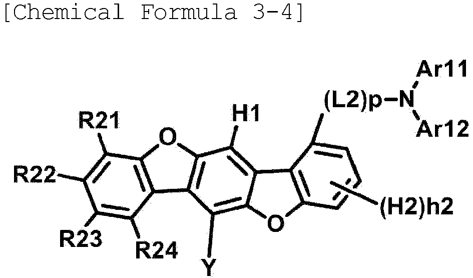 in Chemical Formulae 3-1 to 3-4,
each substituent has the same definition as in Chemical Formula 3.

4. The heterocyclic compound Claim 2, wherein 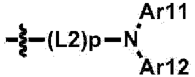 of Chemical Formula 3 is represented by the following Chemical Formula 4 or Chemical Formula 5: 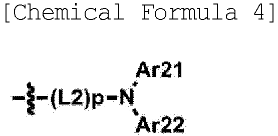 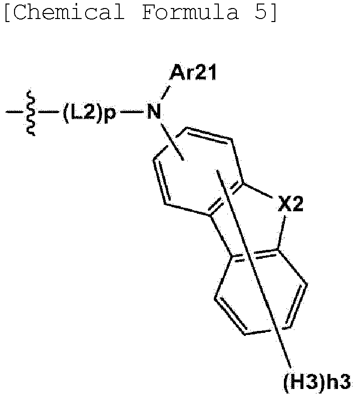 in Chemical Formula 4 and Chemical Formula 5,
L2 and p have the same definitions as in Chemical Formula 3;
X2 is O; or S;
Ar21 and Ar22 are each independently a substituted or unsubstituted C1 to C60 alkyl group; or a substituted or unsubstituted C6 to C60 aryl group;
H3 is hydrogen; or deuterium; and
h3 is an integer of 0 to 7, and when 2 or greater, H3s are the same as or different from each other.

5. The heterocyclic compound of Claim 1, wherein R1 to R8 are each independently hydrogen; or deuterium.

6. The heterocyclic compound of Claim 1, wherein Chemical Formula 1 is represented by any one of the following compounds:

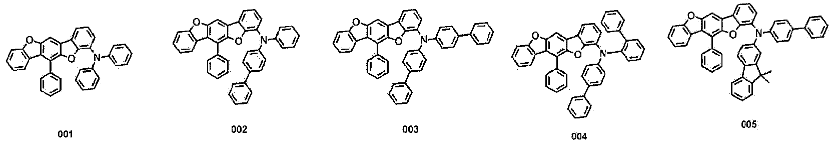

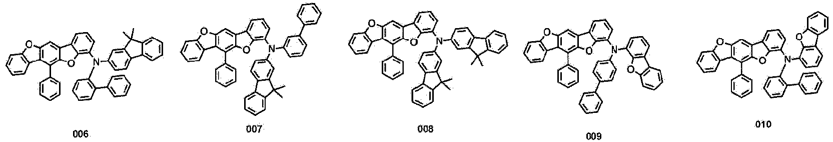

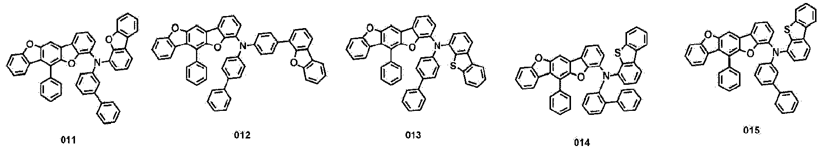

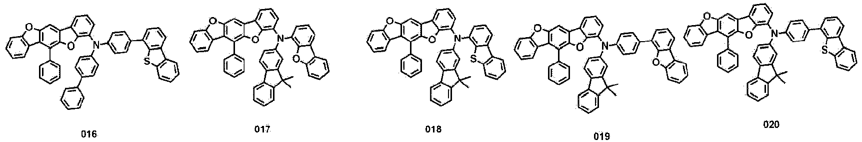

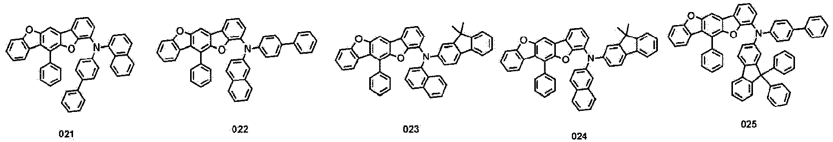

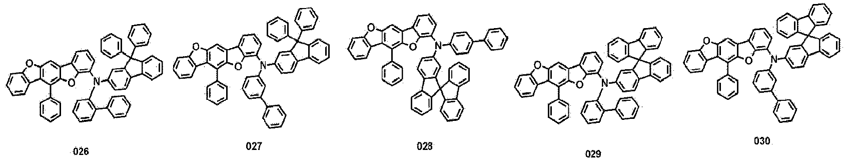

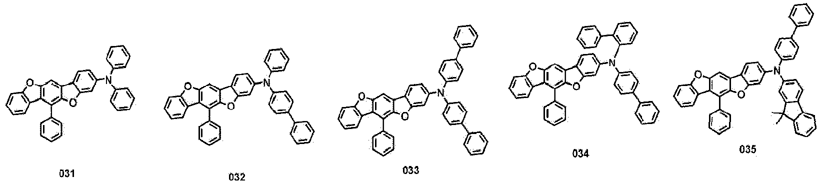

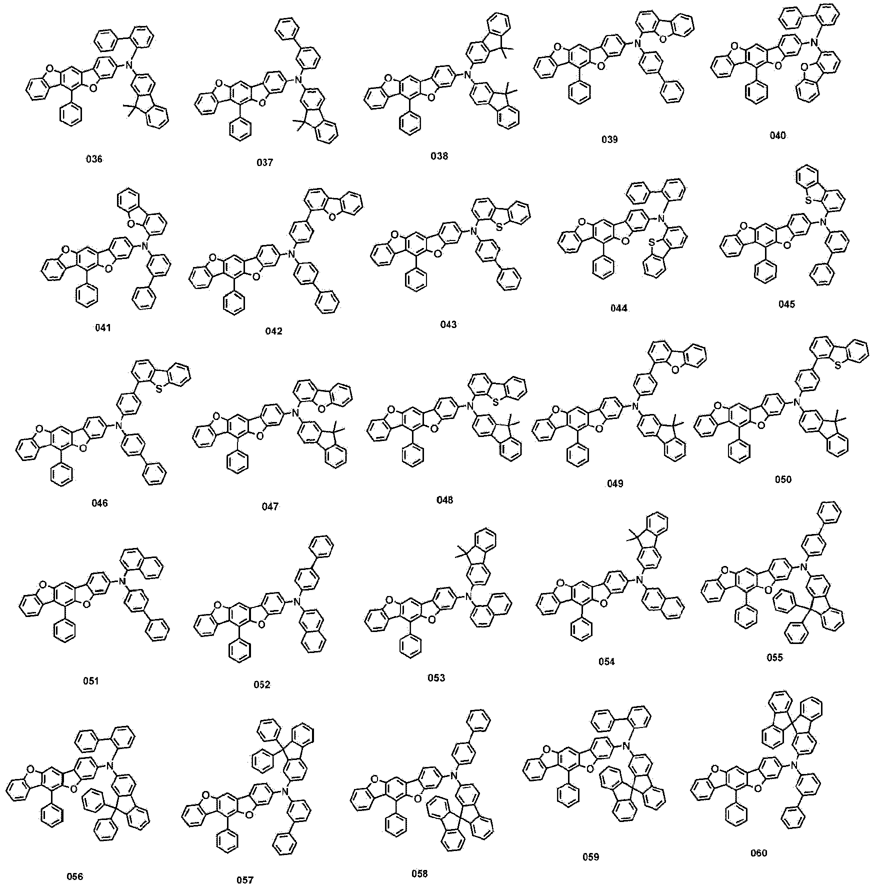

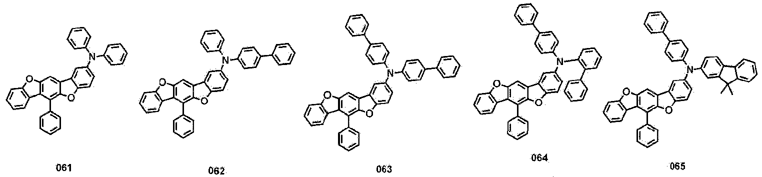

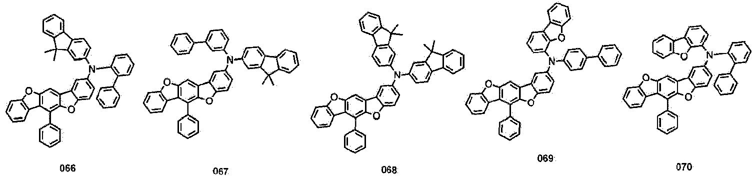

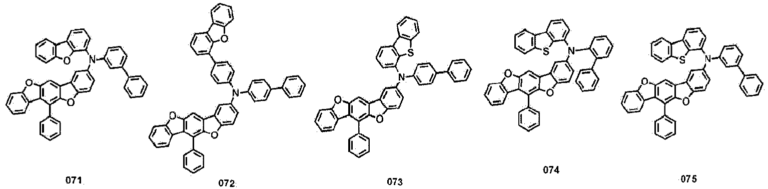

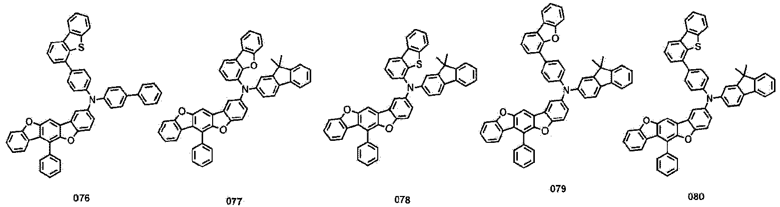

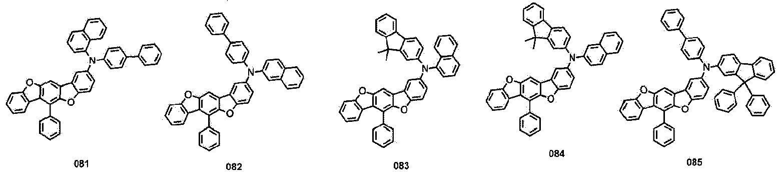

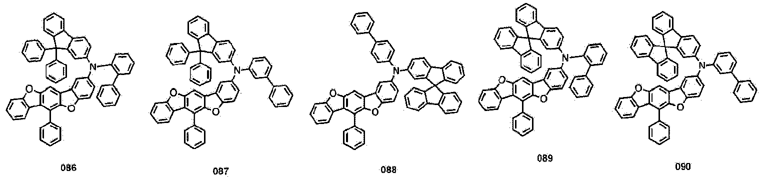

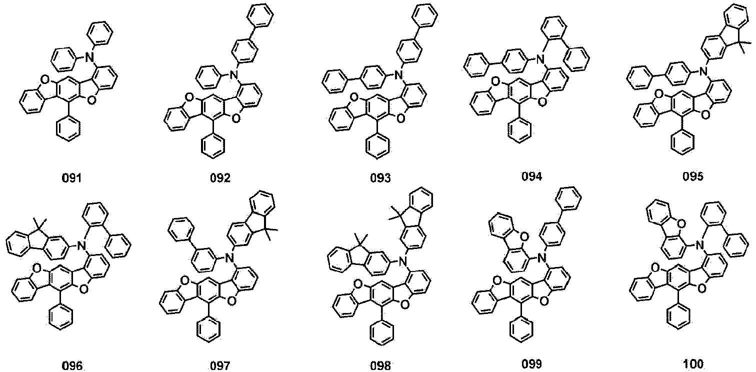

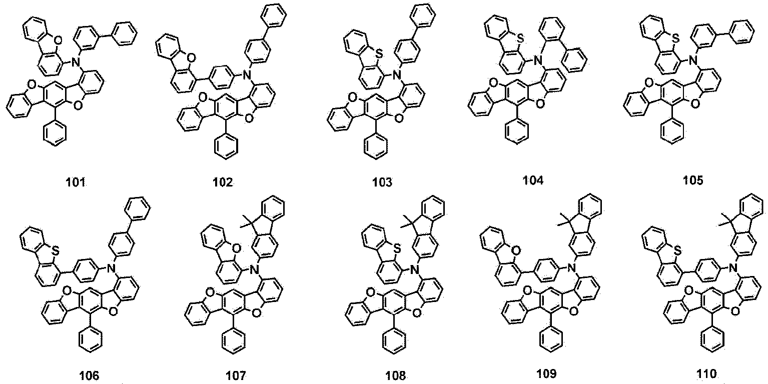

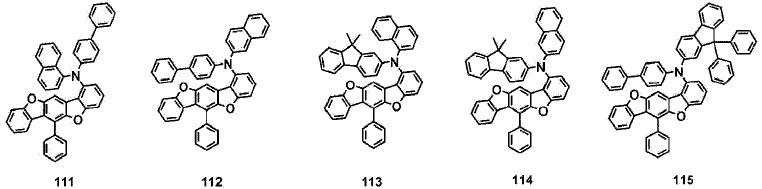

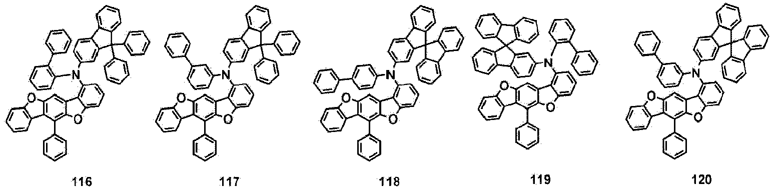

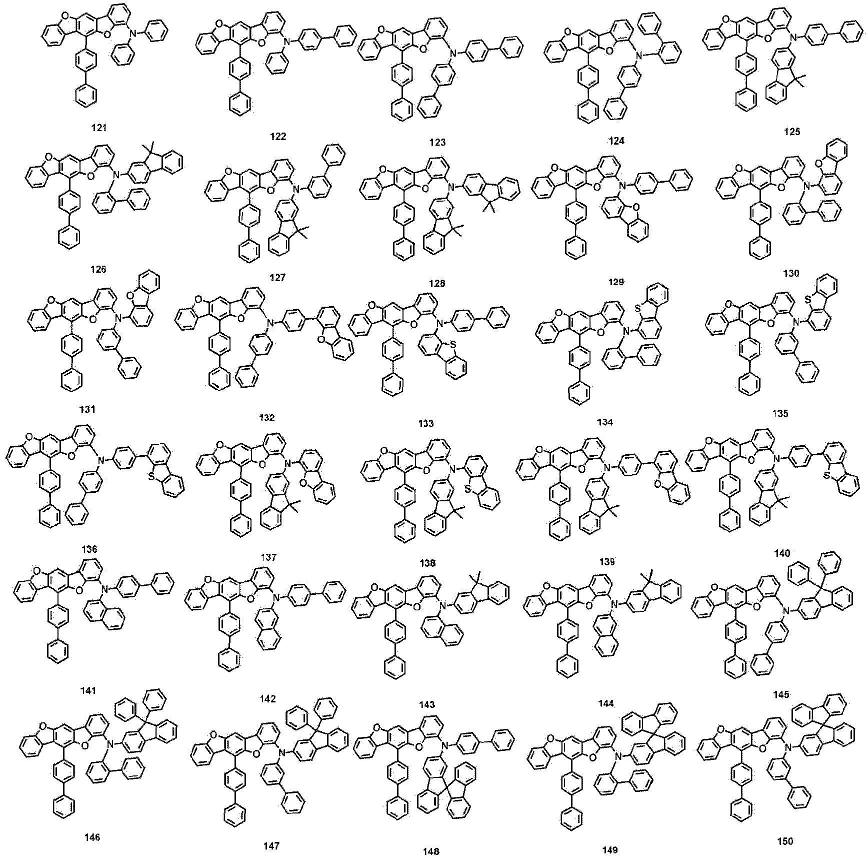

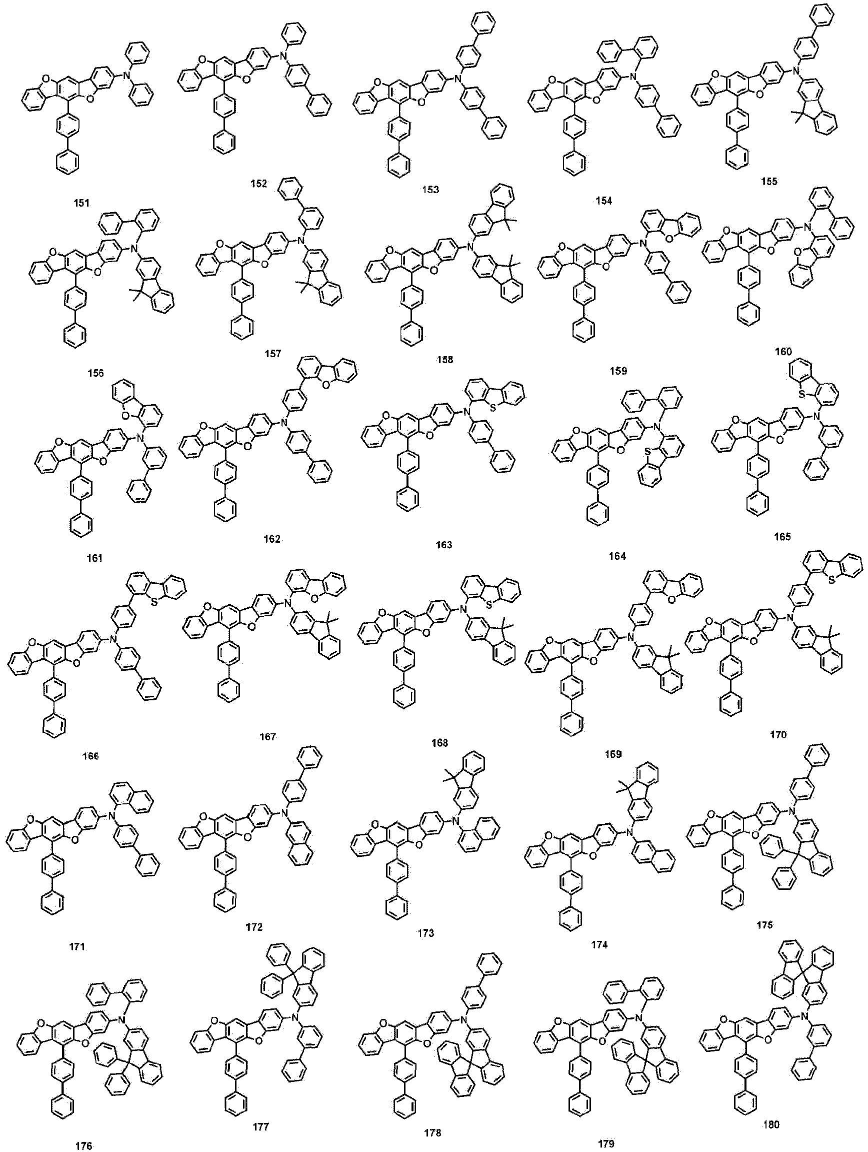

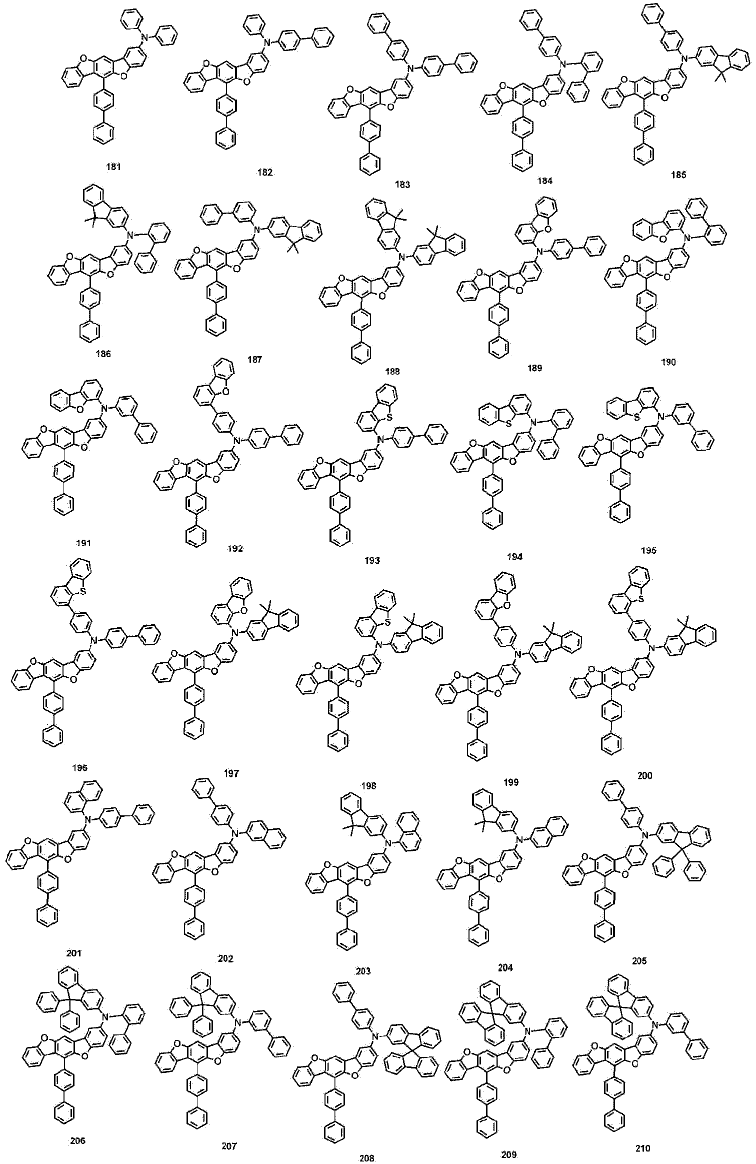

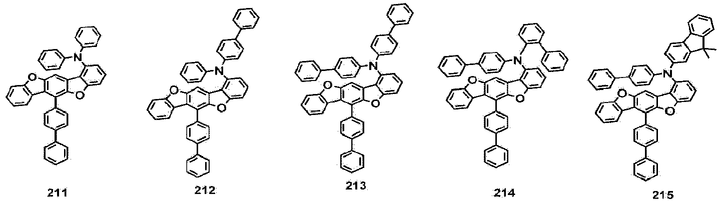

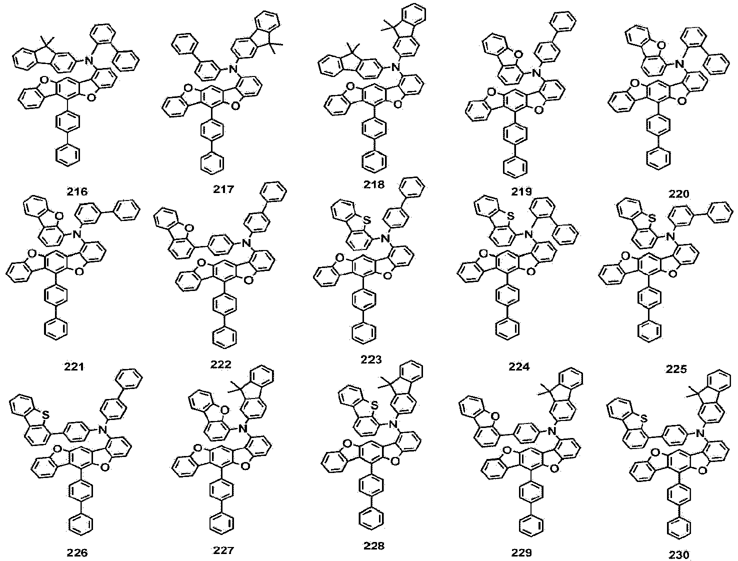

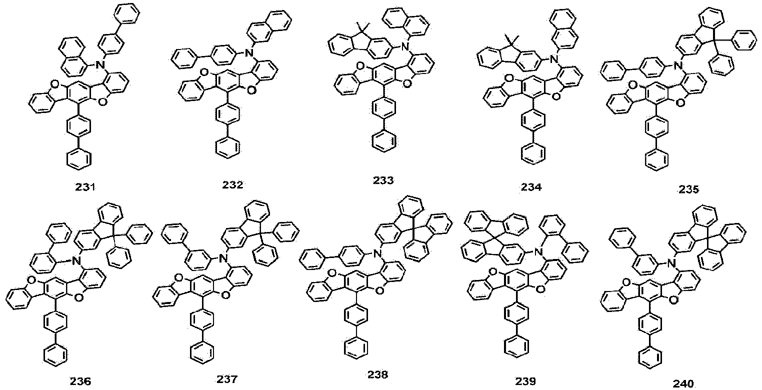

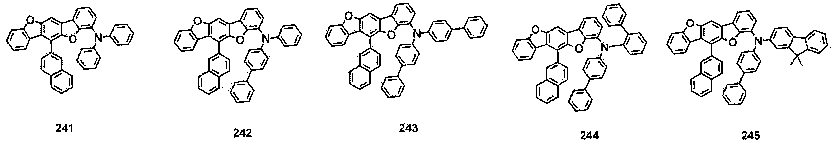

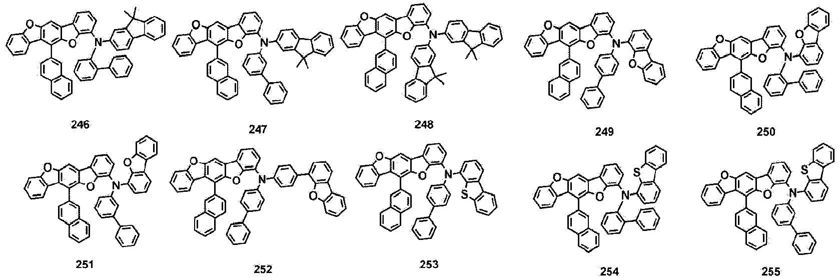

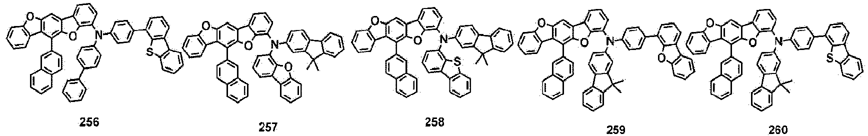

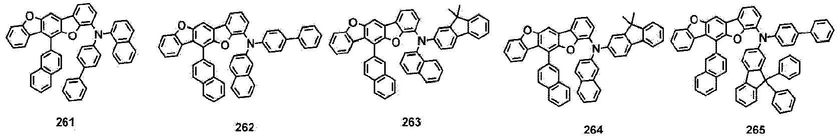

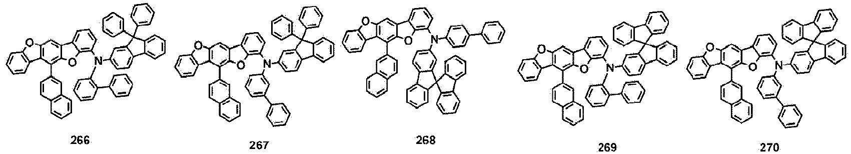

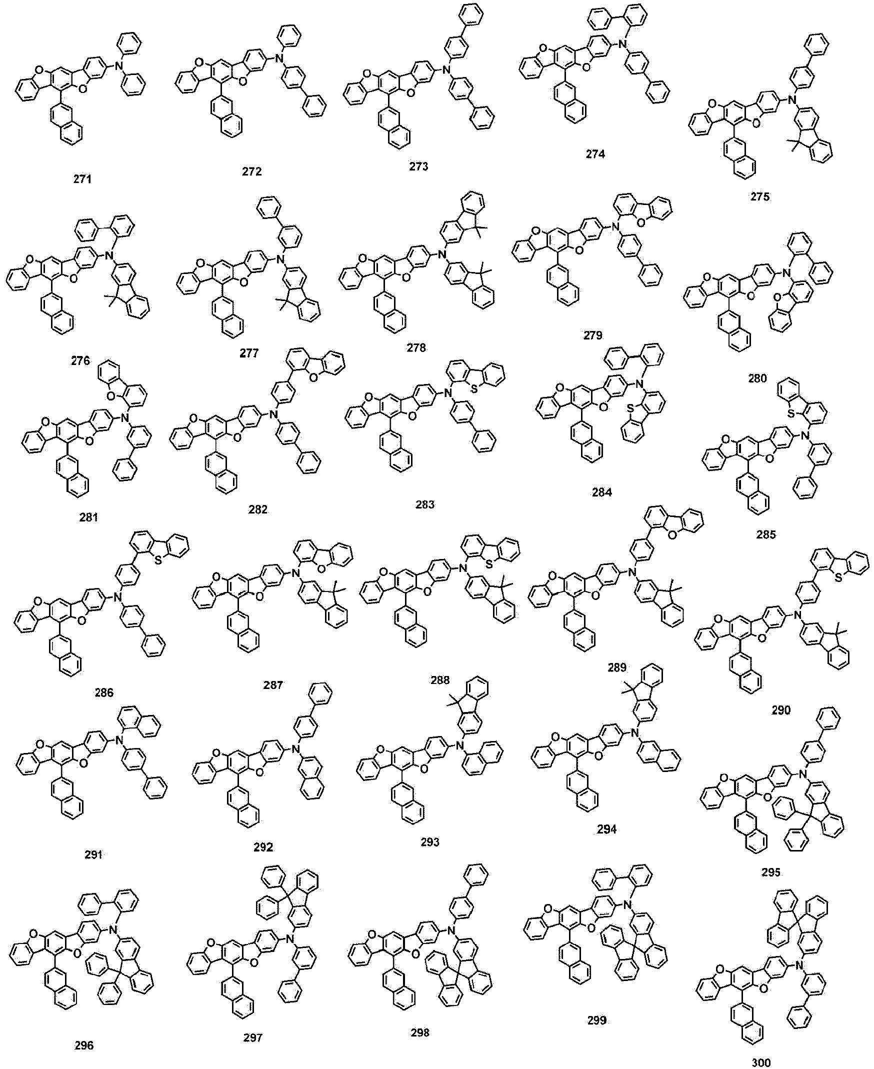

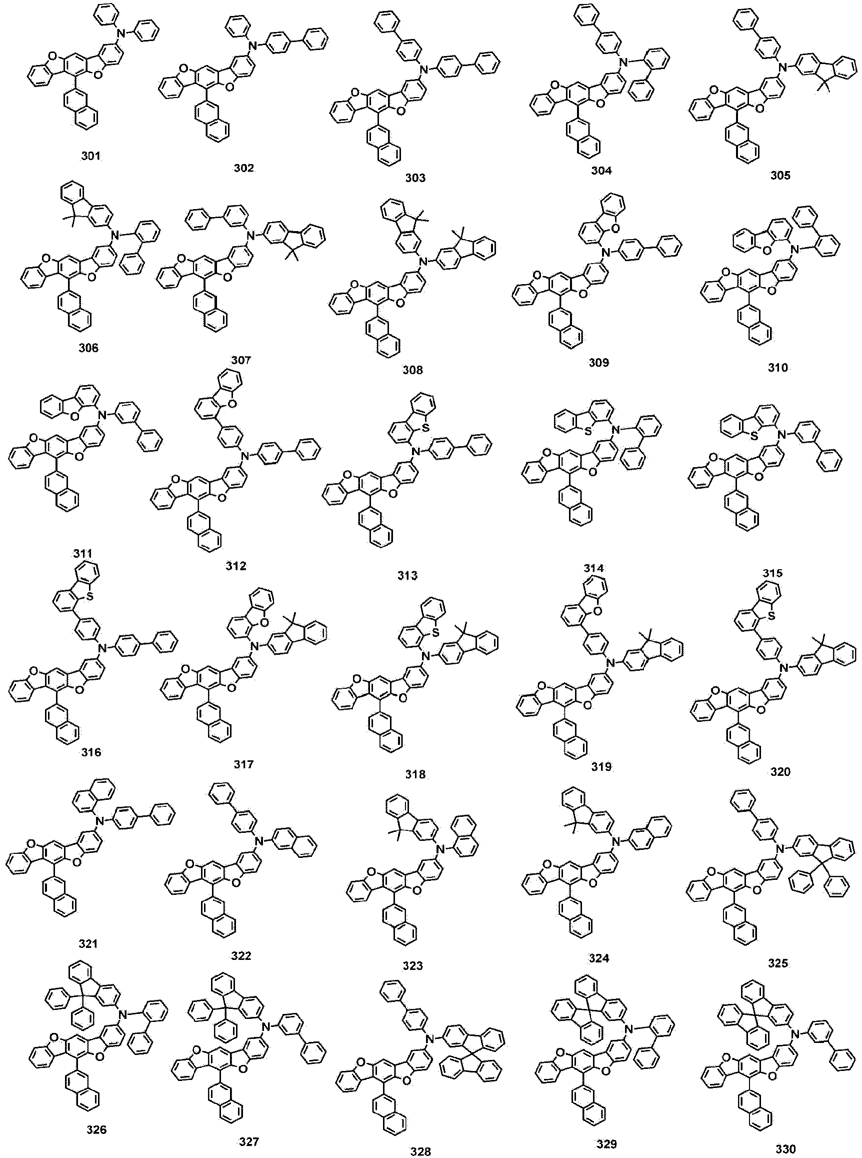

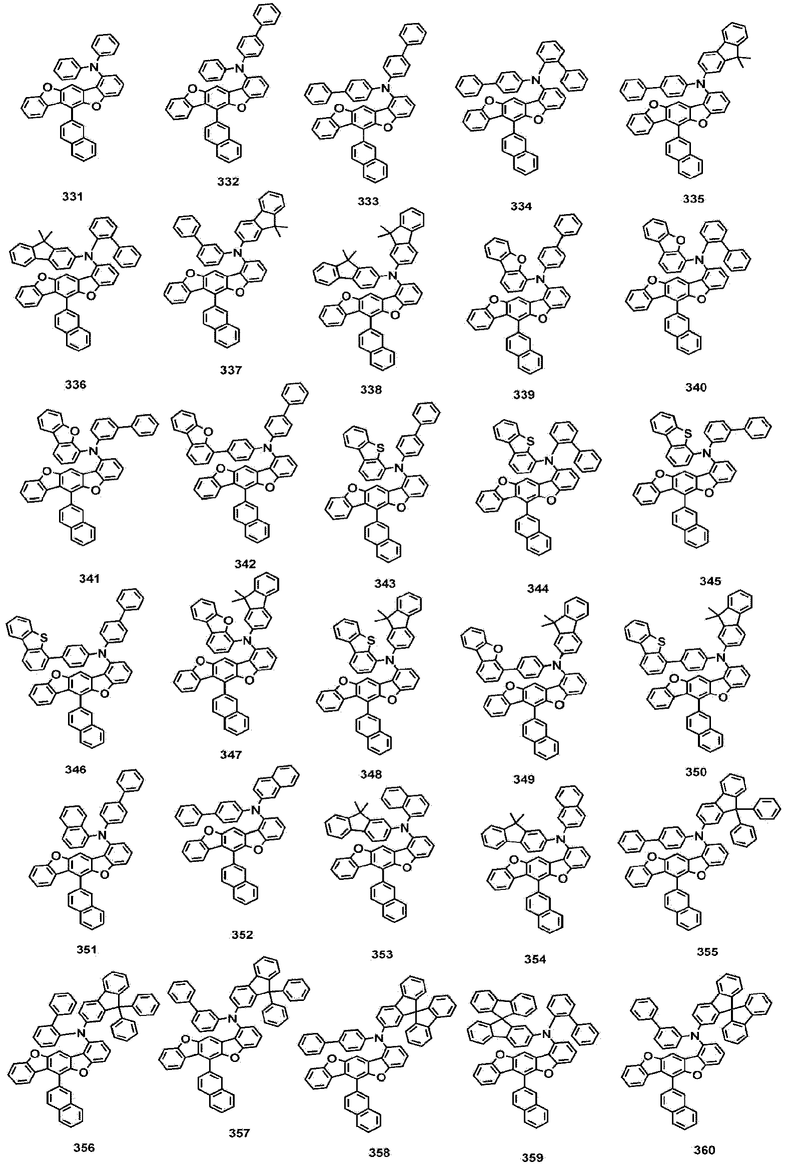

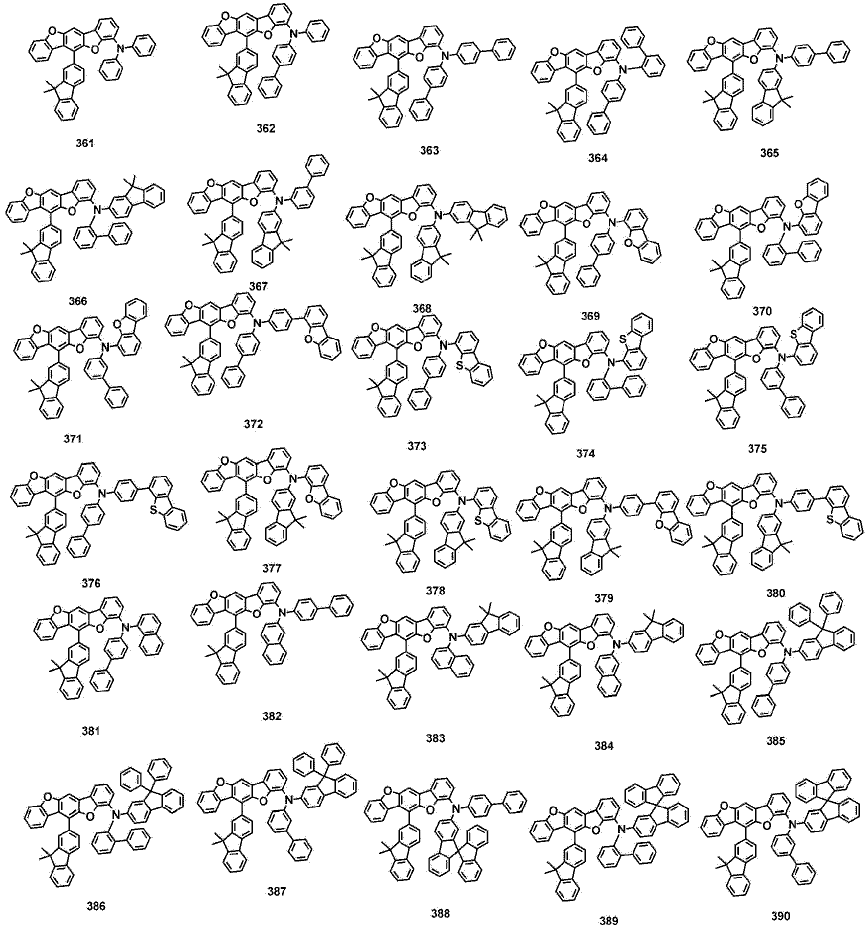

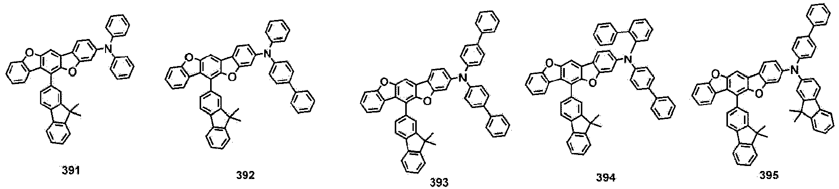

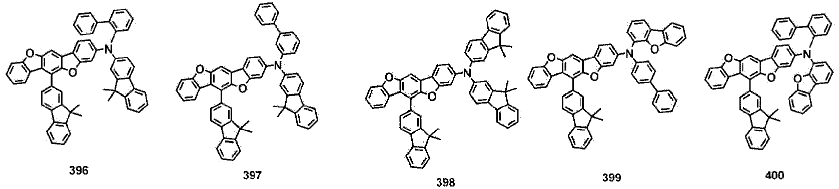

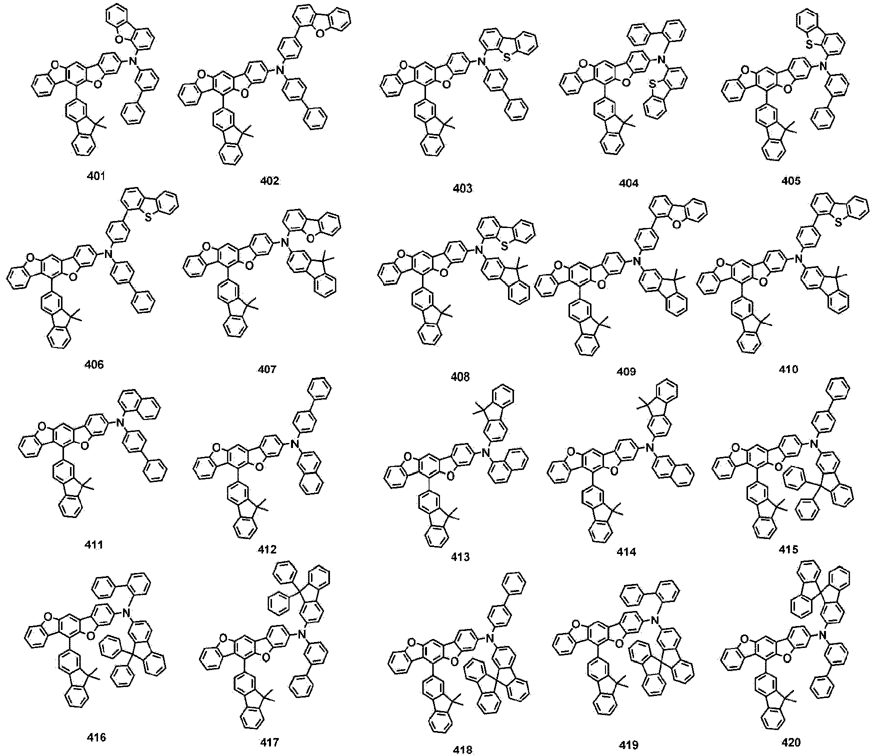

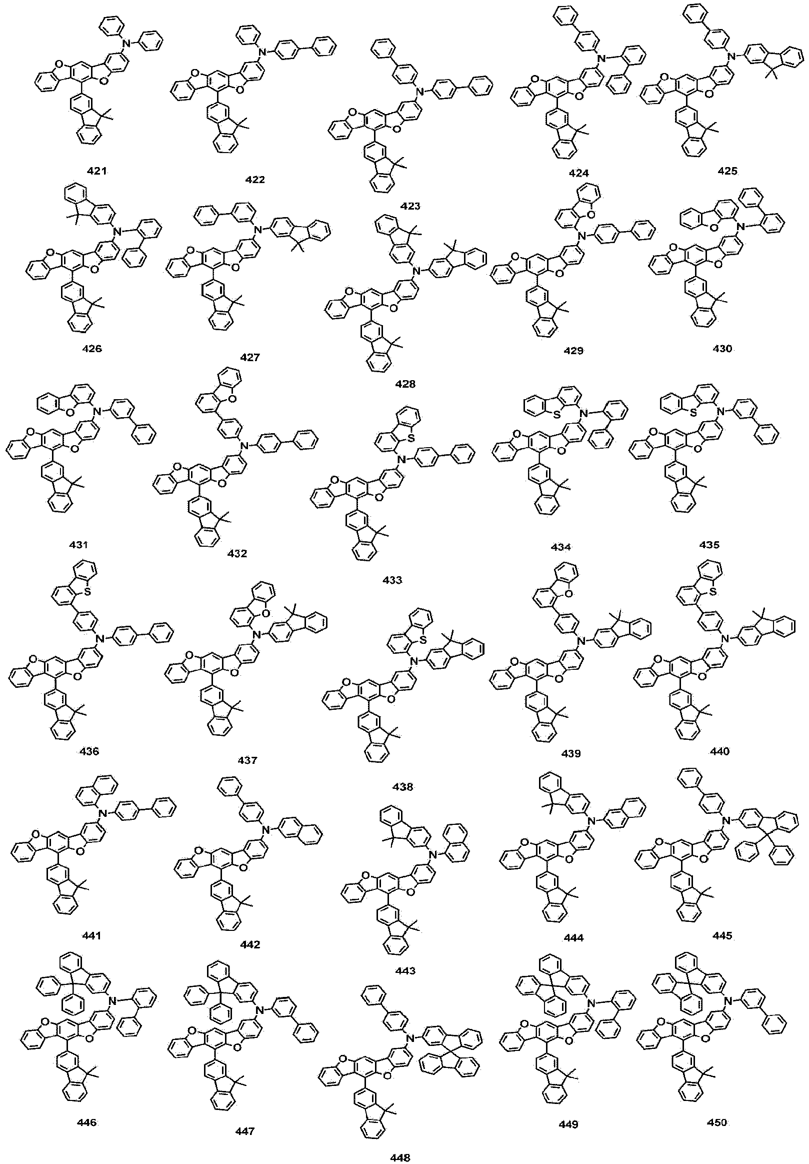

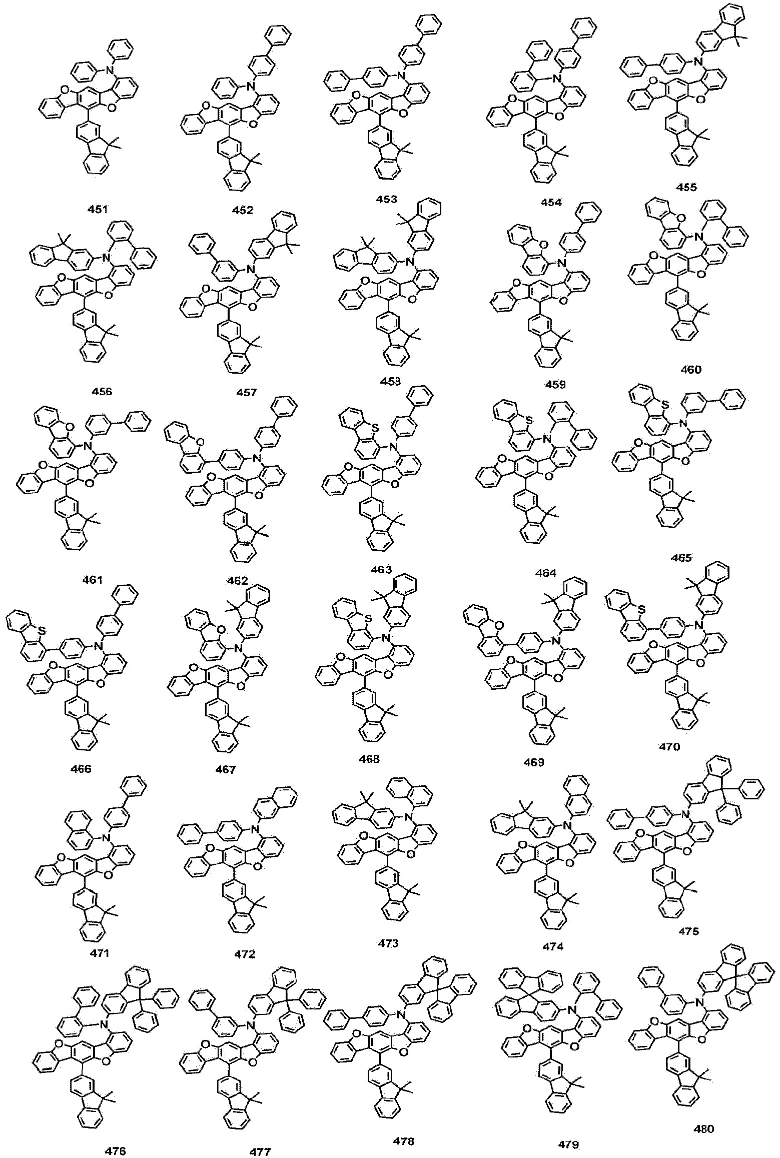

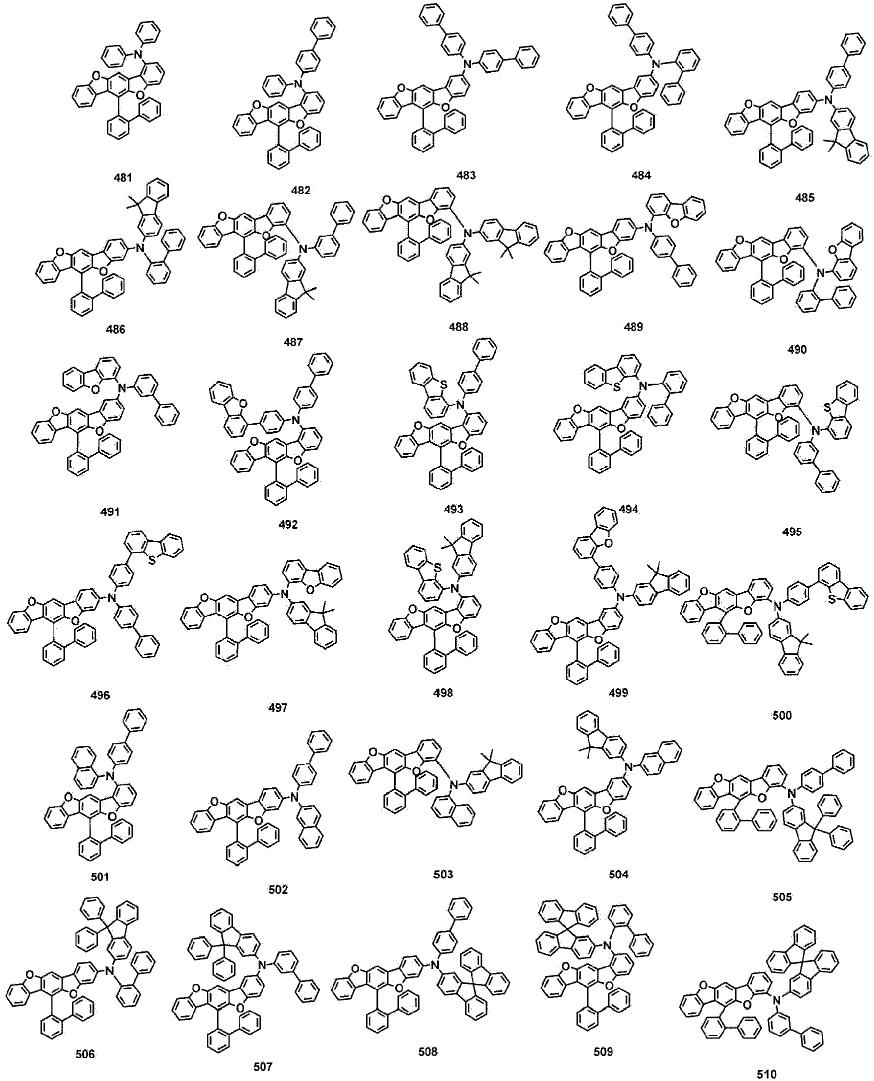

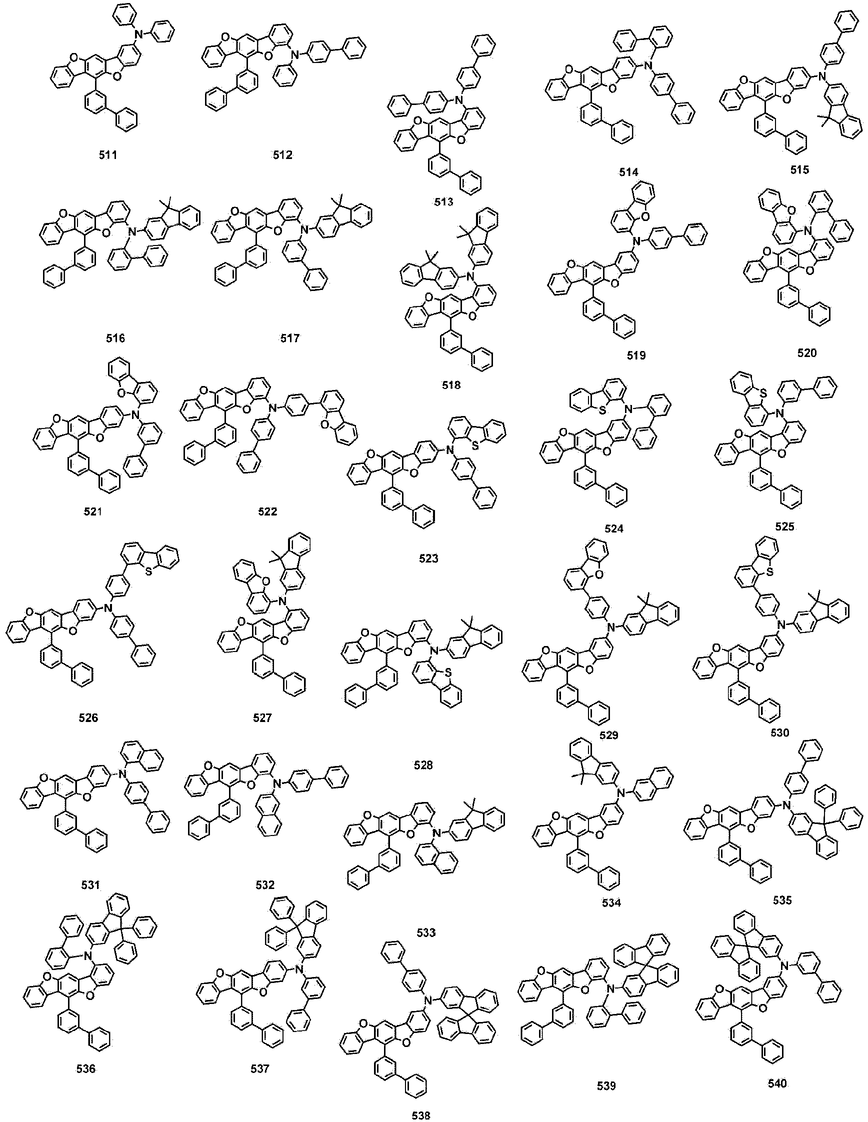

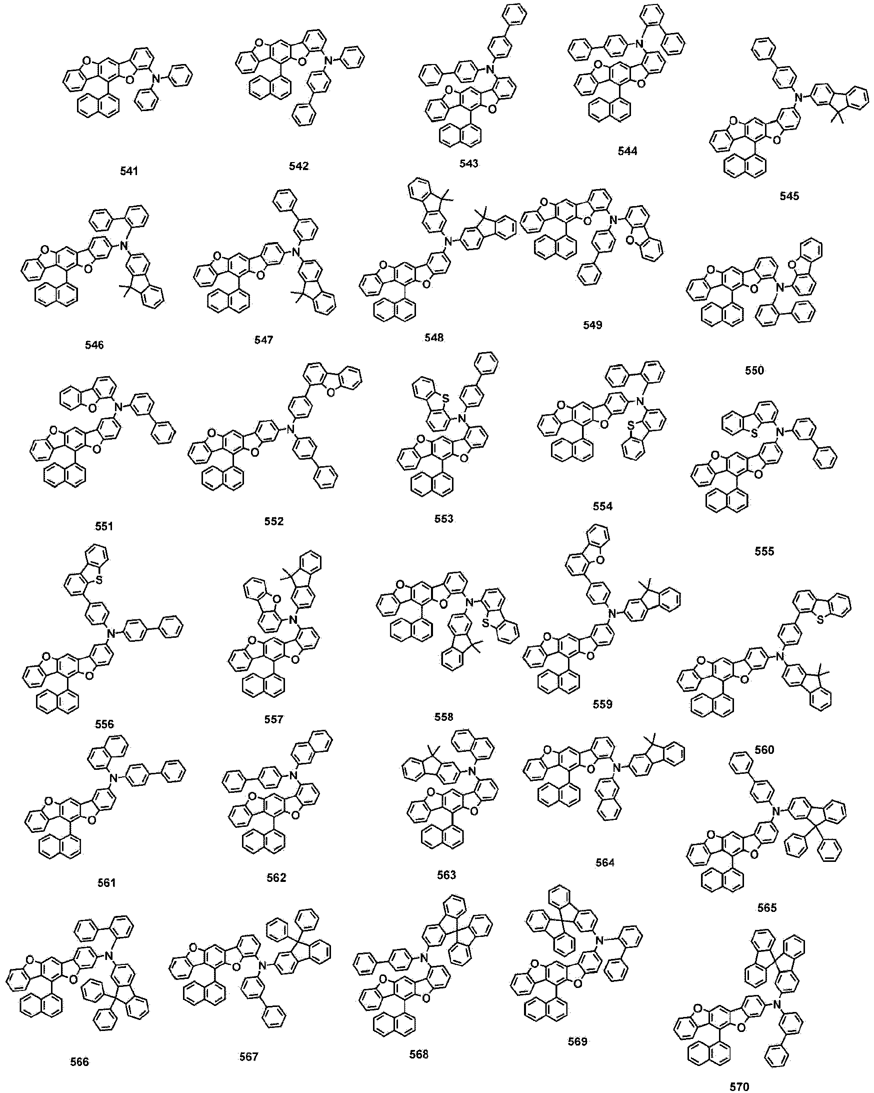

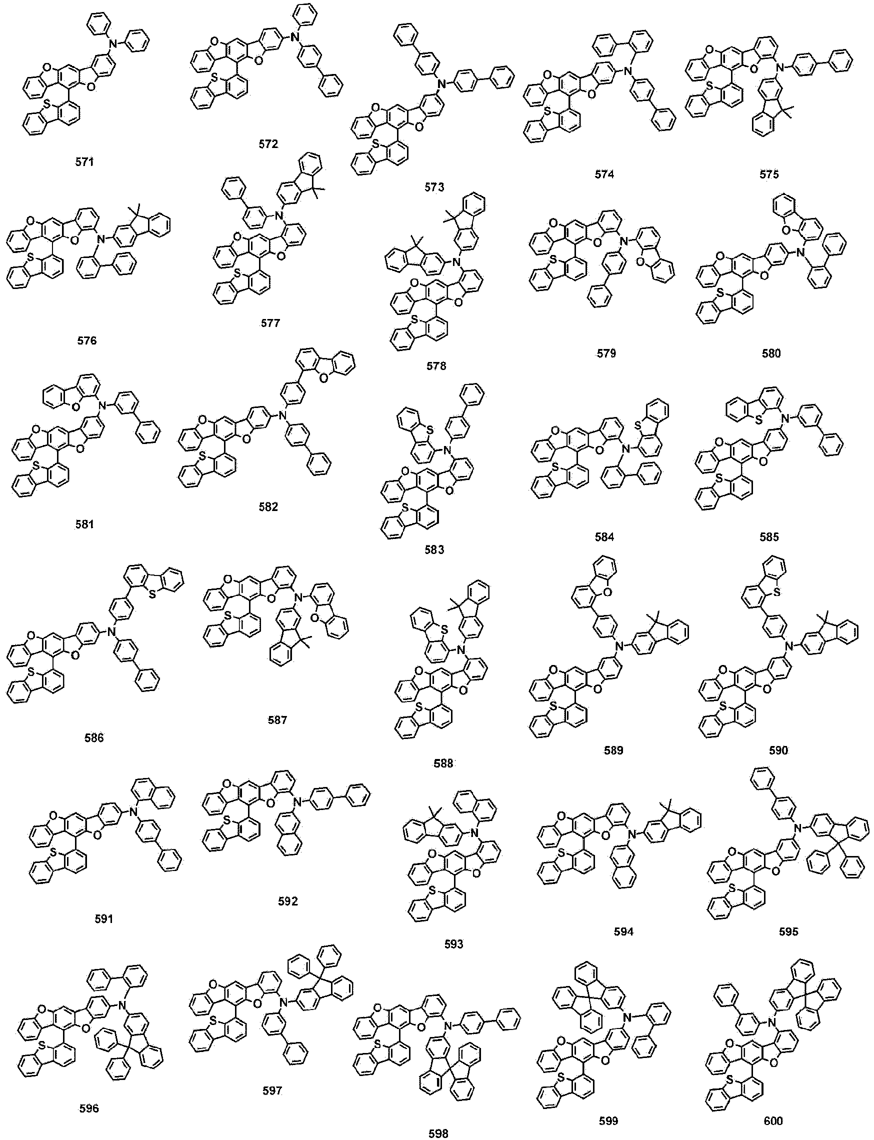

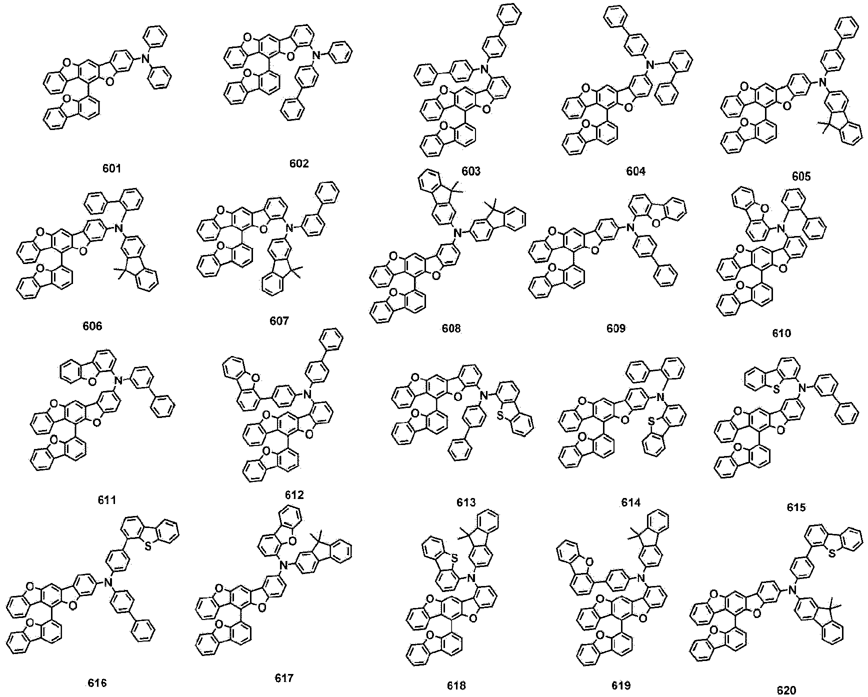

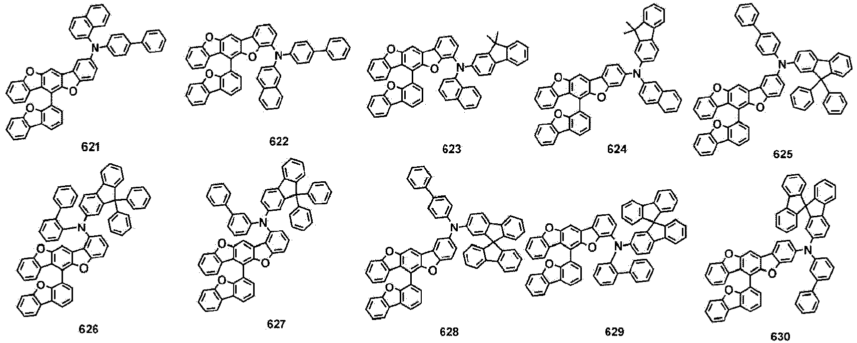

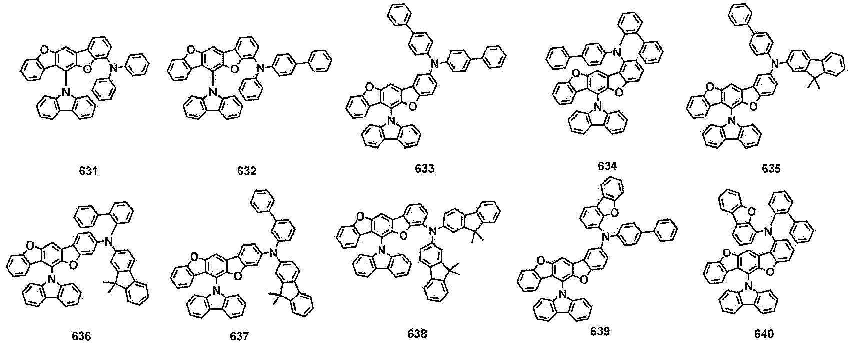

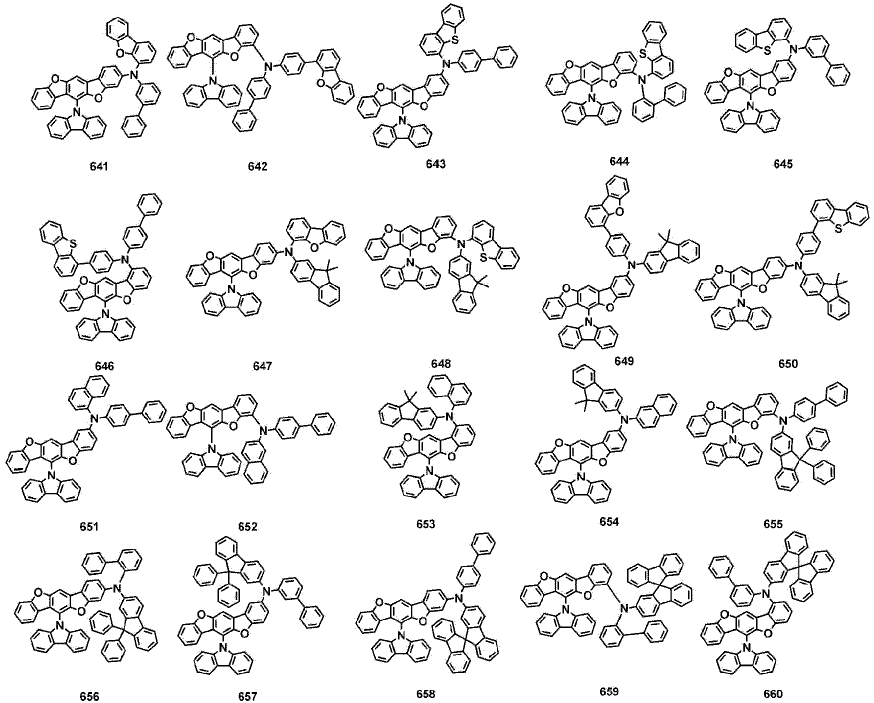

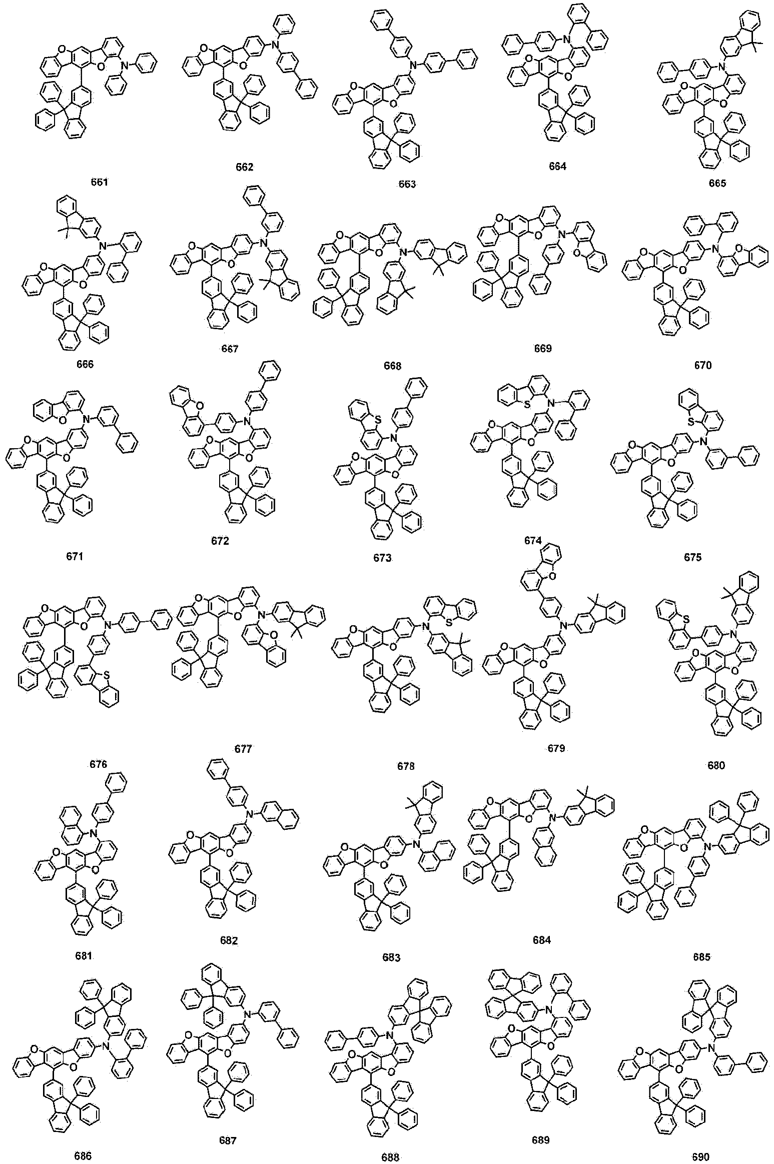

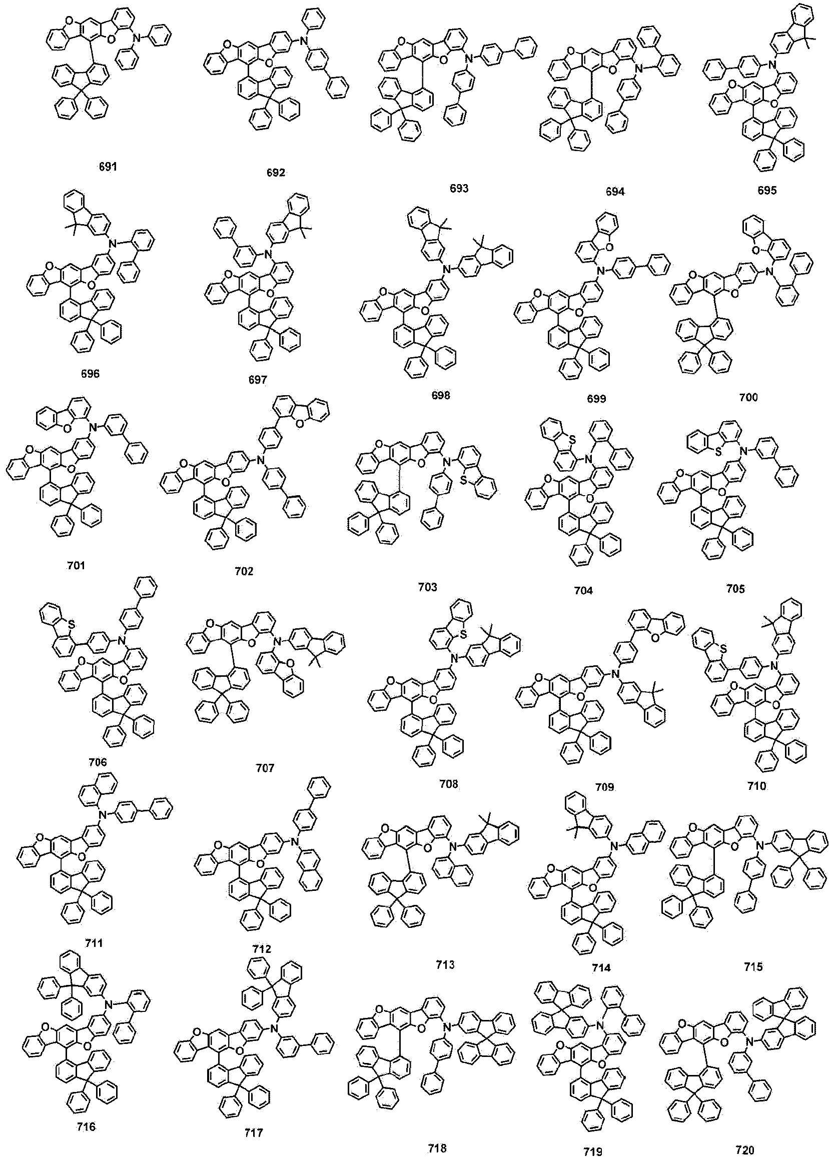

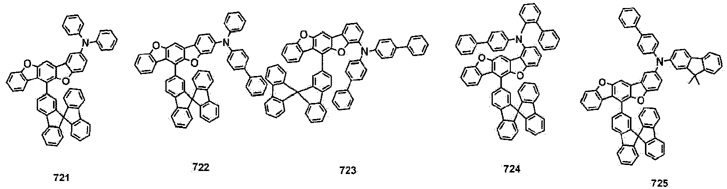

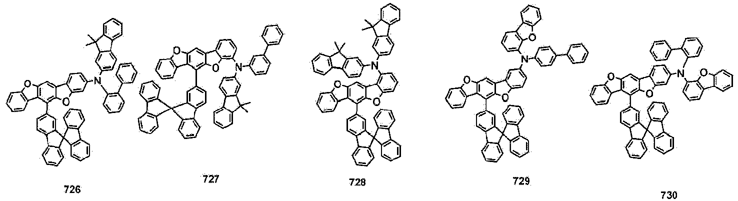

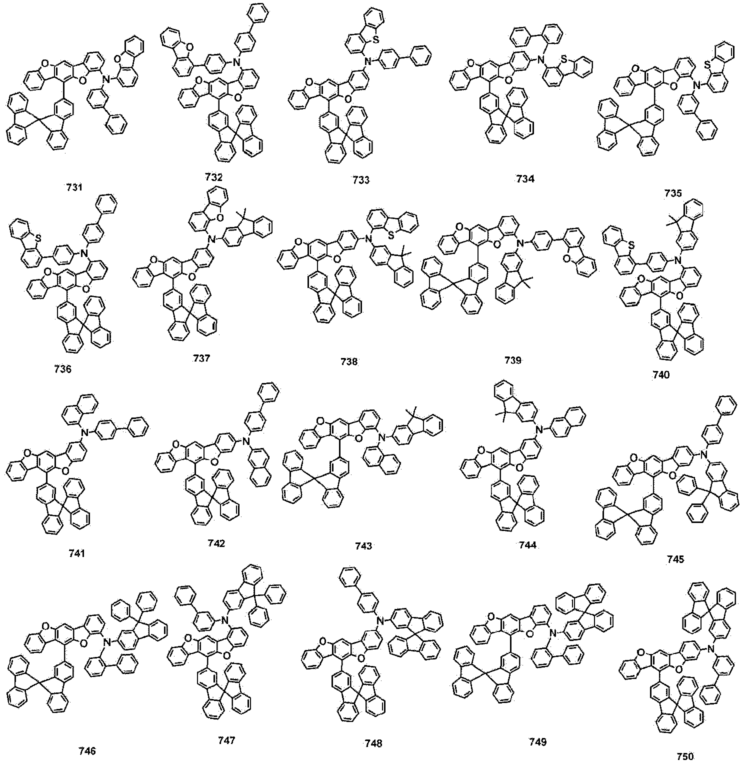

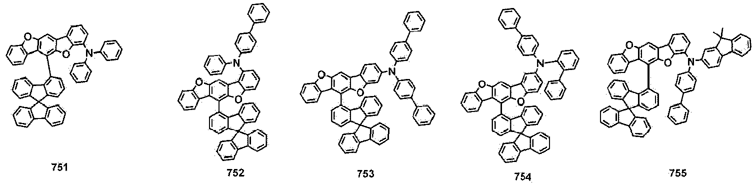

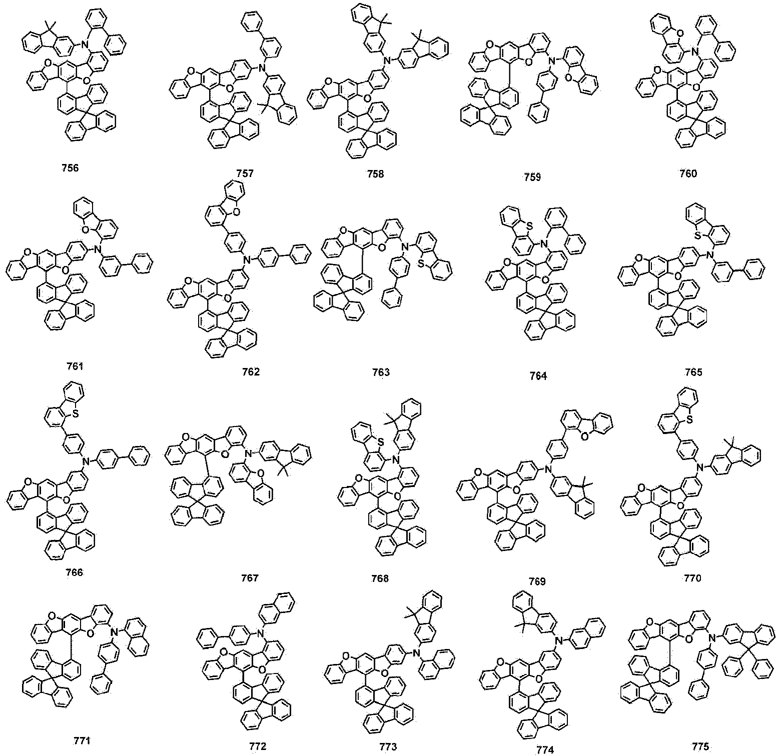

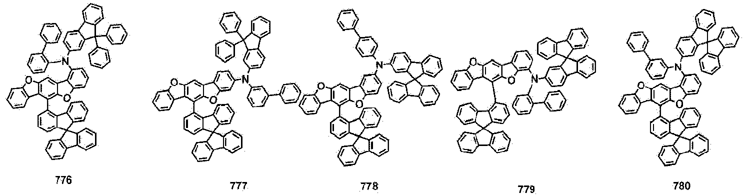

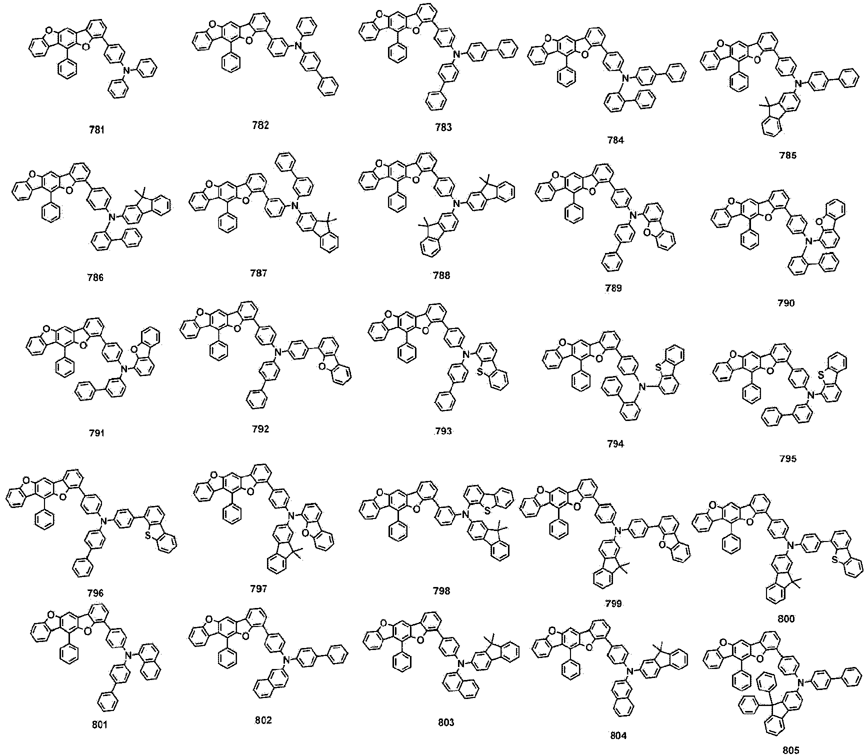

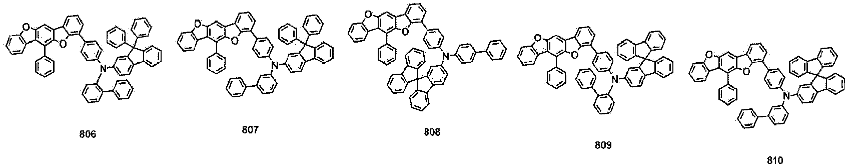

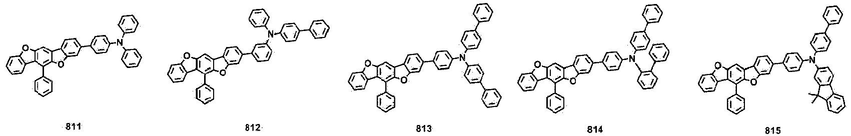

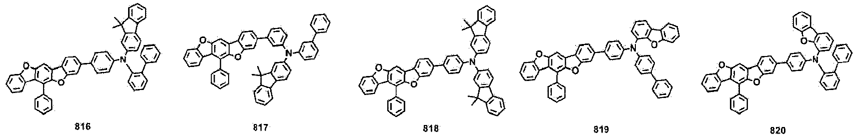

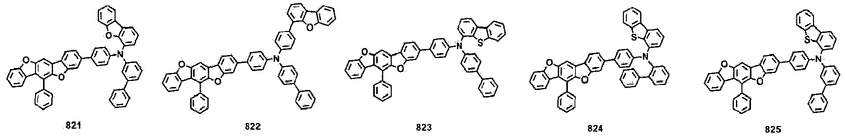

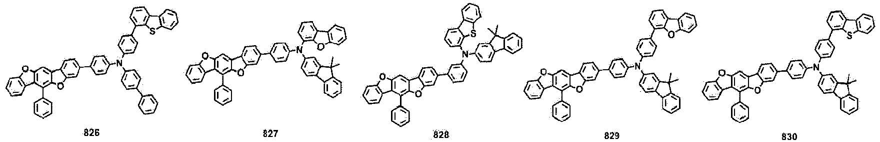

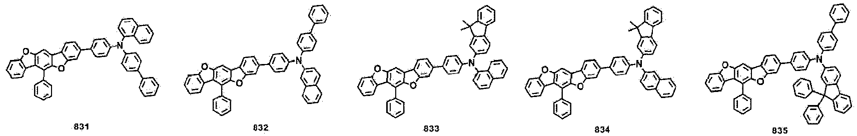

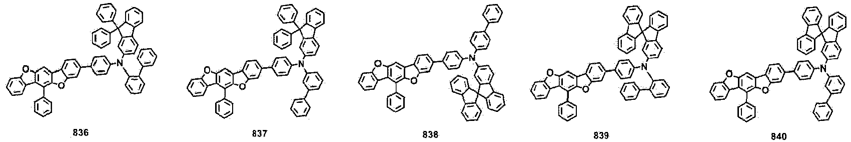

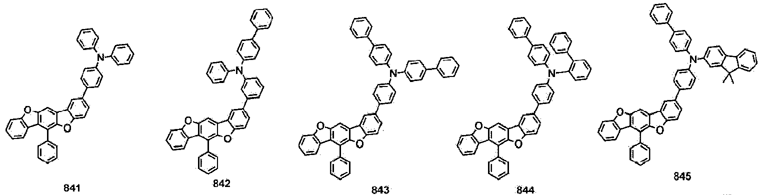

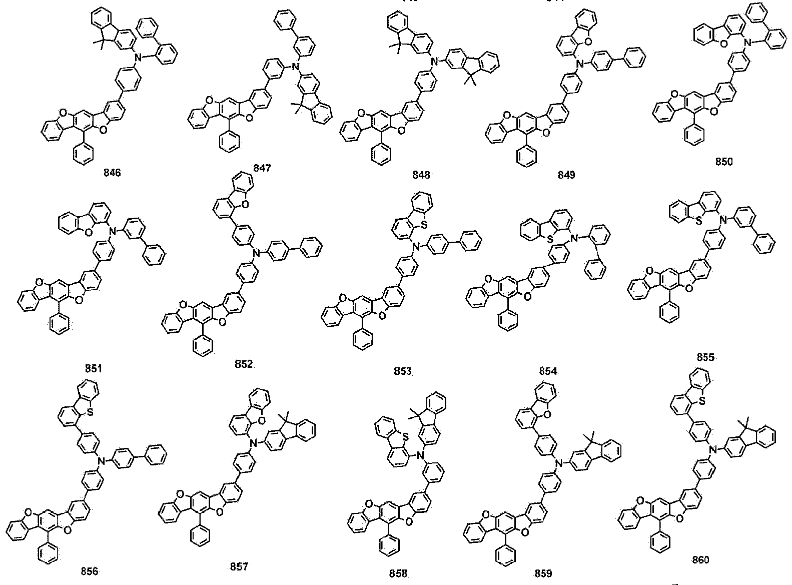

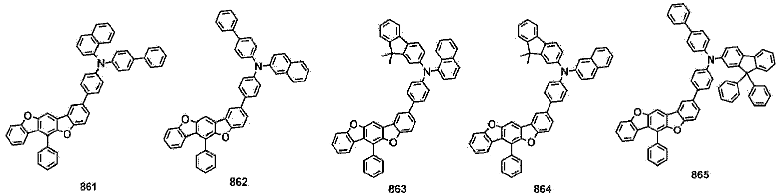

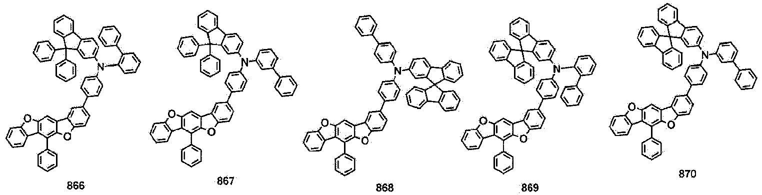

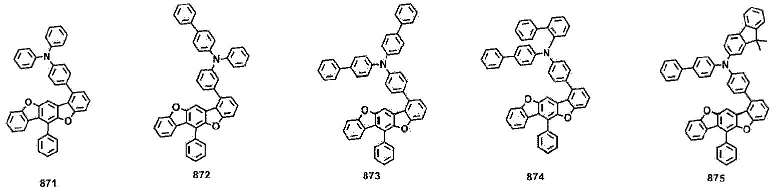

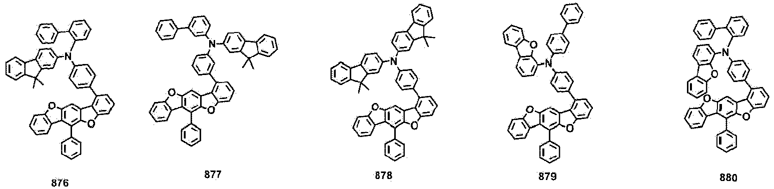

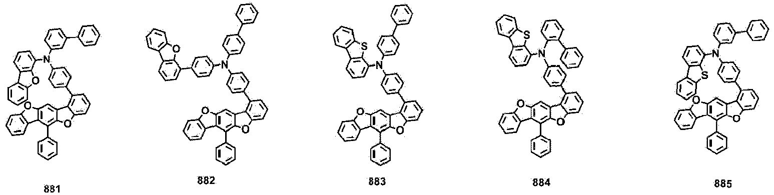

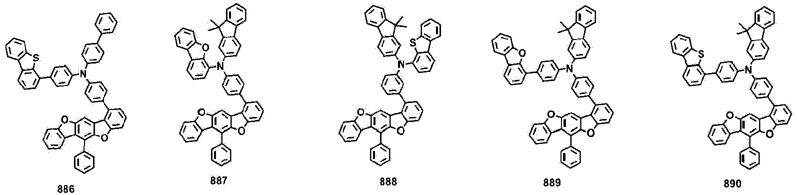

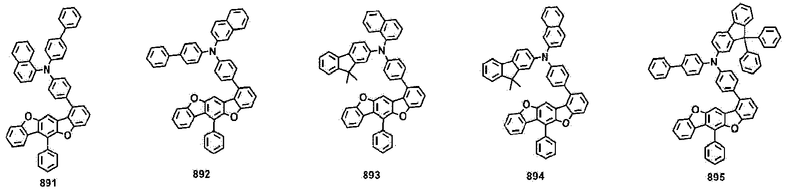

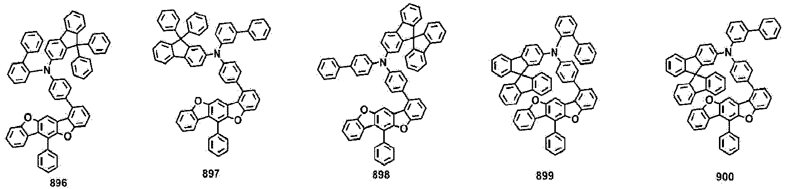

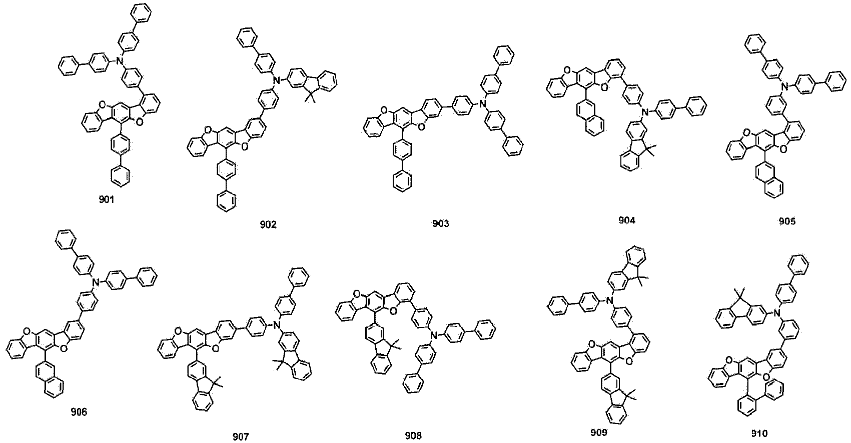

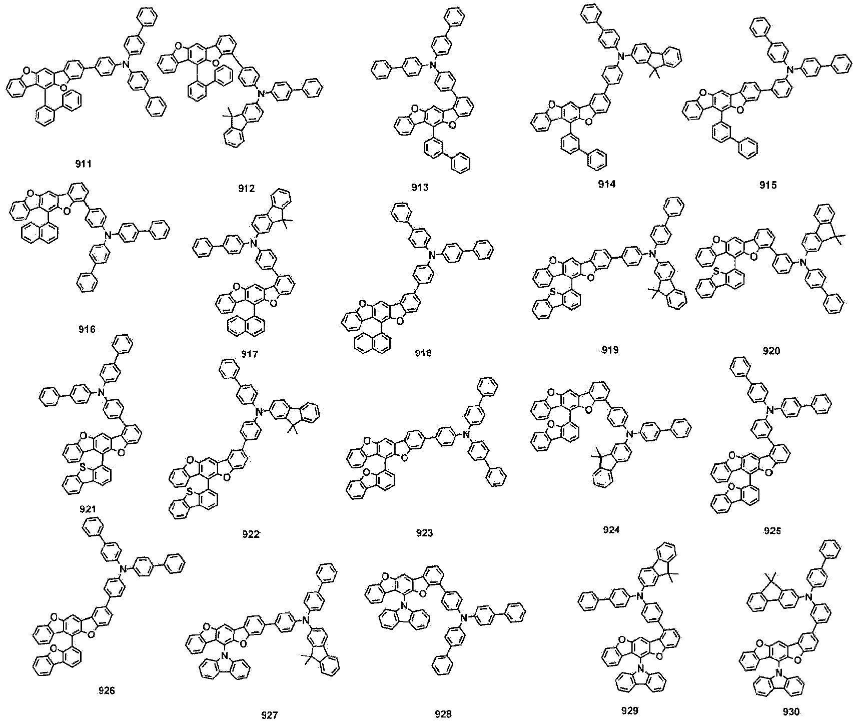

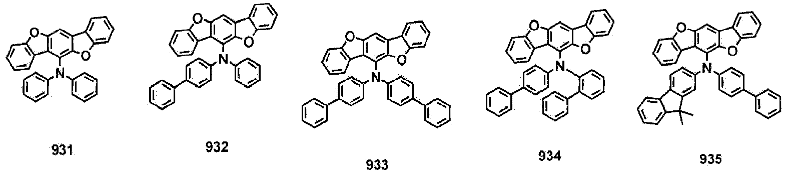

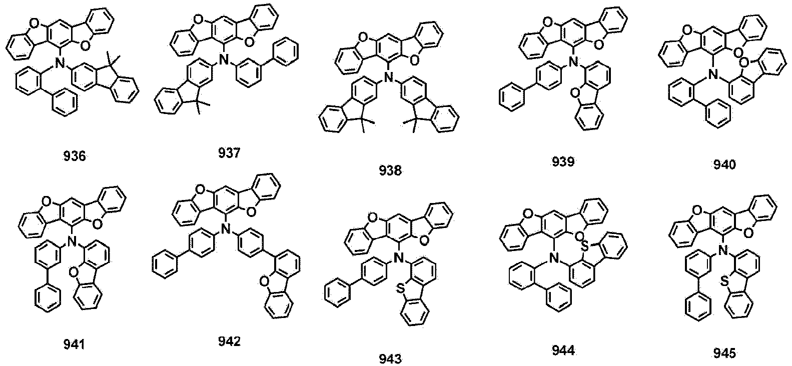

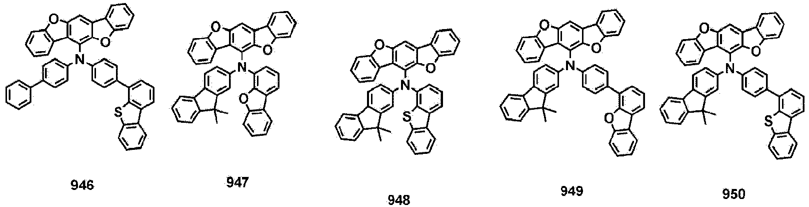

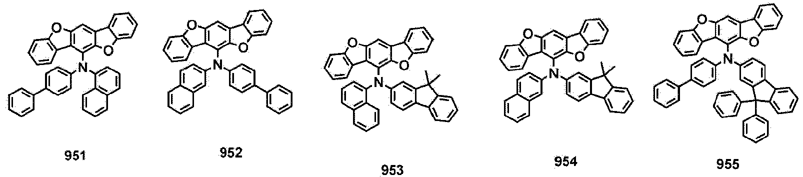

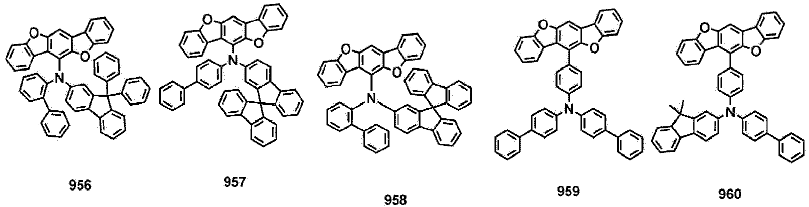

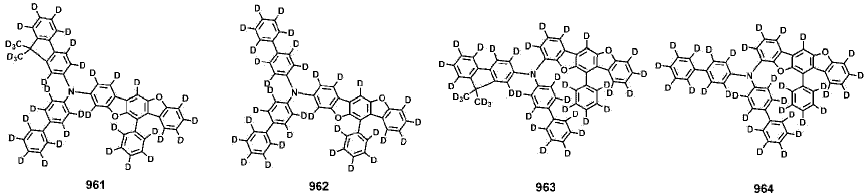

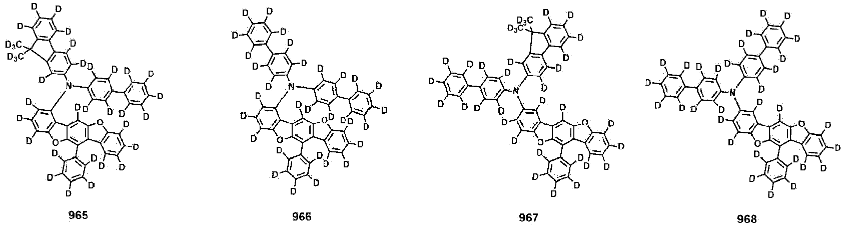

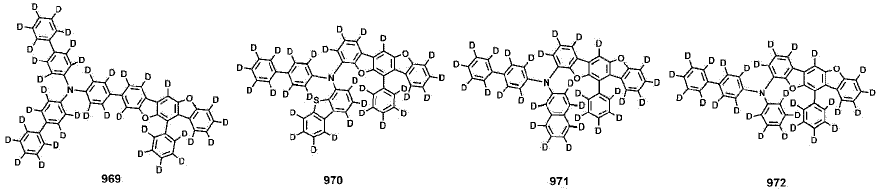

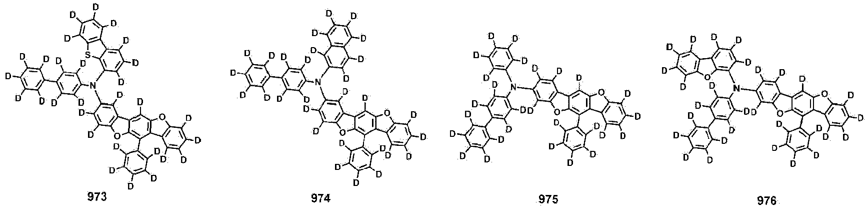

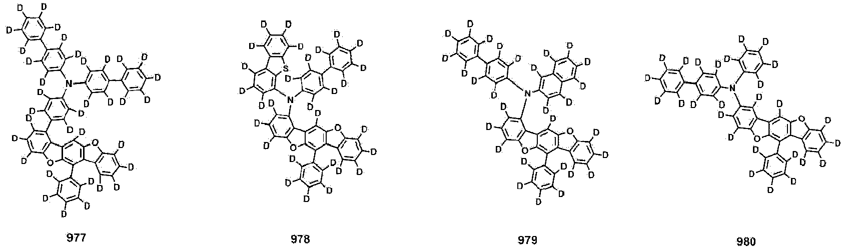

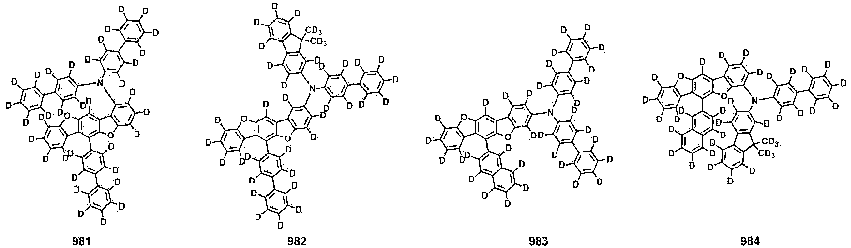

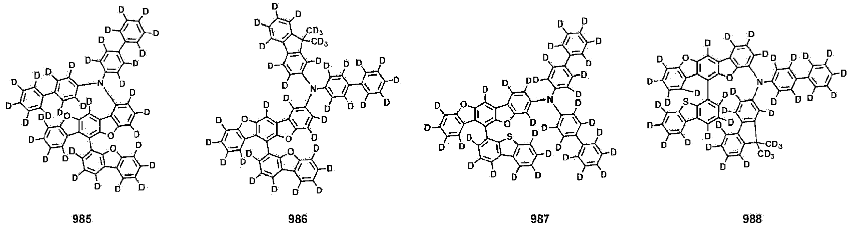

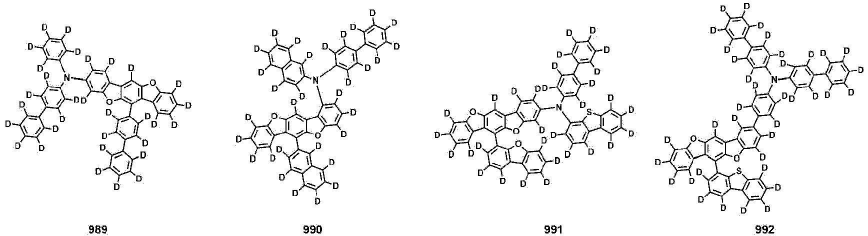

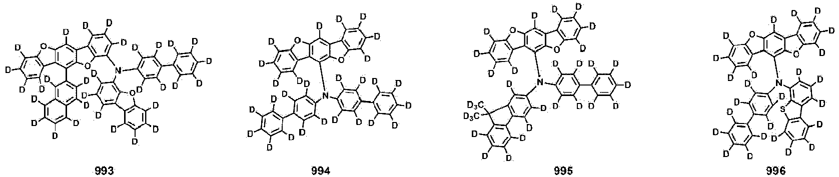

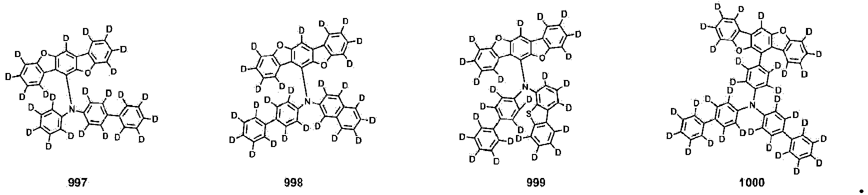

7. An organic light emitting device comprising:
a first electrode;
a second electrode provided opposite to the first electrode; and
one or more organic material layers provided between the first electrode and the second electrode,
wherein one or more layers of the organic material layers include the heterocyclic compound of any one of Claims 1 to 6.

8. The organic light emitting device of Claim 7, wherein the organic material layer includes a hole transfer layer, and the hole transfer layer includes the heterocyclic compound.

9. The organic light emitting device of Claim 7, wherein the organic material layer includes an electron injection layer or an electron transfer layer, and the electron injection layer or the electron transfer layer includes the heterocyclic compound.

10. The organic light emitting device of Claim 7, wherein the organic material layer includes an electron blocking layer or a hole blocking layer, and the electron blocking layer or the hole blocking layer includes the heterocyclic compound.

11. The organic light emitting device of Claim 7, further comprising one, two or more layers selected from the group consisting of a light emitting layer, a hole injection layer, a hole transfer layer, an electron injection layer, an electron transfer layer, an electron blocking layer and a hole blocking layer.
